# EUROPEAN PATENT APPLICATION

(11) **EP 2 338 492 A1**
(43) Date of publication of application: **29.06.2011**
(21) Application number: 09382304.5
(22) Date of filing: 24.12.2009
(51) Int. Cl.: A61K 31/498, A61K 31/517, A61K 31/551, A61K 31/185, A61K 45/06, A61P 25/16, A61P 25/18, A61P 25/28

(54) **Methods and compositions for the treatment of alzheimer**

(71) Applicant: Universidad del Pais Vasco, 48940 Leioa Vizcaya (ES)
(72) Inventor: Alberdi Alfonso, Elena, E-48940, Leioa - Vizcaya (ES); Matute Almau, Carlos, E-48940, Leioa - Vizcaya (ES)
(74) Representative: ABG Patentes, S.L.

(57) **Abstract**

The invention relates to AMPA receptor inhibitors and NMDA receptor inhibitors for use in the treatment of a disease associated with deposition of amyloid proteins, more precisely, Alzheimer.

SEQUENCE LISTING

<110>  Universidad del Pais Vasco

<120>  METHODS AND COMPOSITIONS FOR THE TREATMENT OF ALZHEIMER

<130>  P5477EP00

<160>  30

<170>  PatentIn version 3.5

<210>  1
<211>  5747
<212>  DNA
<213>  Homo sapiens

<400>  1
atagagcttg ctgcctgtgt gagtgtgagg gggagagcga gagagagcaa gggagggaga      60

gagaggcagg ctgcgagggg agaggagagg gagtggggga gccagcgctc cagctagcat     120

gaggacgggc ttcttttccc gtgctcagtt aatctggctg tcagttggtg ttaacgctgc     180

agtttaagtg ttcggattcc aagggaaaca gacaaacctc acgaaaggaa ggaagcaagc     240

aagcaaggaa ggaactgcag gaggaaaaga acaggcagaa cagcgagaag aataaaggga     300

aaggggggga aacaccaaat ctatgattgg acctgggctt ctttttcgcc aatgcaaaaa     360

ggaatatgca gcacattttt gccttcttct gcaccggttt cctaggcgcg gtagtaggtg     420

ccaatttccc caacaatatc cagatcgggg gattatttcc aaaccagcag tcacaggaac     480

atgctgcttt tagatttgct ttgtcgcaac tcacagagcc cccgaagctg ctcccccaga     540

ttgatattgt gaacatcagc gacagctttg agatgaccta tagattctgt tcccagttct     600

ccaaaggagt ctatgccatc tttgggtttt atgaacgtag gactgtcaac atgctgacct     660

ccttttgtgg ggccctccac gtctgcttca ttacgccgag ctttcccgtt gatacatcca     720

atcagtttgt ccttcagctg cgccctgaac tgcaggatgc cctcatcagc atcattgacc     780

attacaagtg gcagaaattt gtctacattt atgatgccga ccggggctta tccgtcctgc     840

agaaagtcct ggatacagct gctgagaaga actggcaggt gacagcagtc aacattttga     900

caaccacaga ggagggatac cggatgctct ttcaggacct ggagaagaaa aaggagcggc     960

tggtggtggt ggactgtgaa tcagaacgcc tcaatgctat cttgggccag attataaagc    1020

tagagaagaa tggcatcggc taccactaca ttcttgcaaa tctgggcttc atggacattg    1080

acttaaacaa attcaaggag agtggcgcca atgtgacagg tttccagctg gtgaactaca    1140

cagacactat tccggccaag atcatgcagc agtggaagaa tagtgatgct cgagaccaca    1200

cacgggtgga ctggaagaga cccaagtaca cctctgcgct cacctacgat ggggtgaagg    1260

tgatggctga ggctttccag agcctgcgga ggcagagaat tgatatatct cgccggggga 1320

atgctgggga ttgtctggct aacccagctg ttccctgggg ccaagggatc gacatccaga 1380

gagctctgca gcaggtgcga tttgaaggtt taacaggaaa cgtgcagttt aatgagaaag 1440

gacgccggac caactacacg ctccacgtga ttgaaatgaa acatgacggc atccgaaaga 1500

ttggttactg gaatgaagat gataagtttg tccctgcagc caccgatgcc caagctgggg 1560

gcgataattc aagtgttcag aacagaacat acatcgtcac aacaatccta gaagatcctt 1620

atgtgatgct caagaagaac gccaatcagt ttgagggcaa tgaccgttac gagggctact 1680

gtgtagagct ggcggcagag attgccaagc acgtgggcta ctcctaccgt ctggagattg 1740

tcagtgatgg aaaatacgga gcccgagacc ctgacacgaa ggcctggaat ggcatggtgg 1800

gagagctggt ctatggaaga gcagatgtgg ctgtggctcc cttaactatc actttggtcc 1860

gggaagaagt tatagatttc tccaaaccat ttatgagttt ggggatctcc atcatgatta 1920

aaaaaccaca gaaatccaag ccgggtgtct tctccttcct tgatcctttg gcttatgaga 1980

tttggatgtg cattgttttt gcctacattg gagtgagtgt tgtcctcttc ctggtcagcc 2040

gcttcagtcc ctatgaatgg cacagtgaag agtttgagga aggacgggac cagacaacca 2100

gtgaccagtc caatgagttt gggatattca acagtttgtg gttctccctg ggagccttca 2160

tgcagcaagg atgtgacatt tctcccaggt ccctgtctgg tcgcatcgtt ggtggcgtct 2220

ggtggttctt caccttaatc atcatctcct catatacagc caatctggcc gccttcctga 2280

ccgtggagag gatggtgtct cccattgaga gtgcagagga cctagcgaag cagacagaaa 2340

ttgcctacgg gacgctggaa gcaggatcta ctaaggagtt cttcaggagg tctaaaattg 2400

ctgtgtttga gaagatgtgg acatacatga agtcagcaga gccatcagtt tttgtgcgga 2460

ccacagagga ggggatgatt cgagtgagga aatccaaagg caaatatgcc tacctcctgg 2520

agtccaccat gaatgagtac attgagcagc ggaaaccctg tgacaccatg aaggtgggag 2580

gtaacttgga ttccaaaggc tatggcattg caacacccaa ggggtctgcc ctgagaaatc 2640

cagtaaacct ggcagtgtta aaactgaacg agcaggggct tttggacaaa ttgaaaaaca 2700

aatggtggta cgacaagggc gagtgcggca gcgggggagg tgattccaag gacaagacaa 2760

gcgctctgag cctcagcaat gtggcaggcg tgttctacat cctgatcgga ggacttggac 2820

tagccatgct ggttgcctta atcgagttct gctacaaatc ccgtagtgaa tccaagcgga 2880

tgaagggttt ttgtttgatc ccacagcaat ccatcaacga agccatacgg acatcgaccc 2940

tcccccgcaa cagcggggca ggagccagca gcggcggcag tggagagaat ggtcgggtgg 3000

tcagccatga cttccccaag tccatgcaat cgattccttg catgagccac agttcaggga 3060

tgcccttggg agccacggga ttgtaactgg agcagatgga gaccccttgg ggagcaggct 3120

cgggctcccc agccccatcc caaacccttc agtgccaaaa acaacaacaa aatgaaacgc 3180

aaccaccacc aaccactgcg accacaagaa ggatgattca acaggttttc ctgaagaatt 3240

gaaaaaccat tttgctgtcc cttttccttt tttgatgttc tttcaccctt ttctgtttgc 3300

taagtgagga tgaaaaaata acactgtact gcaataaggg gagagtaacc ctgtctaatg 3360

aaacctgtgt ctctgagagt agagtcactg gaacactaat gaggaaactg cactgtttta 3420

ttttaattca gttgttagtg tgtcttagtg tgtgcaattt tttttcttac taatatccat 3480

ggtttgcagg ttctgttagg ccctttcctt ctccttactt cttatcccca actccctacc 3540

cacccctctt cagttttcag attggagatt caagatttgt tccactttac aagcaagagg 3600

aaaaaaaagc aaccttcaaa ctaattctcc atgggggctc tccatgttac cctccactcc 3660

ttggcccaaa cctctgatgg agatagacat tgttggagaa gtgggctgcc ttccccaagt 3720

ggggcactgc ttaagcactt attcagtgga gaacacaggt gaaaagcaac tcaggatgag 3780

ggtggtggag agggcagggg cagatgtgca gtcagagaag gactcctgaa gttactgctg 3840

ctcagaaaaa cagttccttt aatgtggaag agccatttca taggtcatag gtggtatggt 3900

atatttcttc agagtcaacc ttggccctga gaagtatgtc ctcctggtgt gctcaggctc 3960

aacggcagtc tggtggctga aggcacttgg cctcctaaac caagcagaat tttgggaaga 4020

gataacagcc agggagatat tgcccatgat tctcactttt tctttgcctg gcatctaagc 4080

aggaacccat tgtggagtag actctcttct tctatggagc ctctgacatg gggagcaatg 4140

ctaagcaagc taagtgtaaa agaaaagtga cagaataatt ttggaagagg aagcctcatc 4200

aaaagctcac acaaaataga gcttcccatg gtgtgcccta tcctaggttt aagaaaacac 4260

gtatgaagtt tatgctgatg caaagaactt gggtttttat gttaatataa agtgttgttt 4320

tagcatgtgg ccagatgatg ctctgtcatc tttagaaagt gagataacca aggaaataat 4380

tgaaggagta tagggagatg gattaagttg ataatgacat ttagggcaac ttaagacctt 4440

tgatcccagg ttctaactca aagaggctga ccttccccca gctaagatag catgaggacg 4500

ttgtattcca atatacgtat gattggggct acaaagctga actaaagcaa gattggtgaa 4560

gtggcagggt ttatagagag aagcccaggc tgagttcagc ttttgttgga agtgagaatc 4620

cctgacatat agctttcttg gagatcccaa ctctcattct tggtgcaact ggcttccagc 4680

tctccagcag tcactctcct aggtgcatga ttcagtgcgt gccatgtgtc attagctttt 4740

actgataacc atattctggc ttgttccctt accccctact tctatccaat tttctctgct 4800

aggggttatc attagcaatt gacatgctaa aggttttgga gcccacctag gggtaggtgc 4860

agctttattg gcttttctgt ggattctctc agtggaccca caccatctct atgtctctcc 4920

actctcctgc cttcagccat agcaaagaat ccttccaaaa tcaaactctt cacttttttg 4980

actcaagtgt tgttgttcag tctctcgcgt gtcaatgtgg tcatggttca tgaaaccgga 5040

ccctcaagat ggatgattgc ttttaactac tgccagctga tgtctctcag cccctgccct 5100

catacaagat ttttctcagc cttcagccta ccactgcaga atccgatgtg acccaccatt 5160

agggagtctg catcttggaa gagttggaaa taacccttta acatcaacat gcttcaaaga 5220

ctttttgcct ttggcctagt aagatgcctc tccagctact gagcccacaa gtaacatgag 5280

cggataaaaa gagacttgtt tgtgctagaa atgagggtct atgctatgag ggggtccaag 5340

actctggcga aatgtgcttt ttcatcaatg gagaaatgaa aggaaaacac aagcaagaaa 5400

aaagttaact tgtattatgt atttttacta cacttttctt aaaaatagag cattgggaaa 5460

actctgaaag agactgacat ttttctcaac aggaatccat acttaacagt tctggctttc 5520

attaaatttt gctctttggt acctgggcct tttatttaac atctatattt gttttaactc 5580

tcttggcaga tgtgtgaaag gattcttgct tgatcaaaca ctaagtattt ttttggttct 5640

tgtttttctt tcaaatagcc aggttttttt cttttggtat ttgcataaaa tgaaaatatc 5700

accgaatatt aaatcactgt ggatccatta aaaaaaaaaa aaaaaaa 5747

<210> 2
<211> 5747
<212> DNA
<213> Homo sapiens

<400> 2
atagagcttg ctgcctgtgt gagtgtgagg gggagagcga gagagagcaa gggagggaga 60

gagaggcagg ctgcgagggg agaggagagg gagtggggga gccagcgctc cagctagcat 120

gaggacgggc ttcttttccc gtgctcagtt aatctggctg tcagttggtg ttaacgctgc 180

agtttaagtg ttcggattcc aagggaaaca gacaaacctc acgaaaggaa ggaagcaagc 240

aagcaaggaa ggaactgcag gaggaaaaga acaggcagaa cagcgagaag aataaaggga 300

aaggggggga aacaccaaat ctatgattgg acctgggctt ctttttcgcc aatgcaaaaa 360

ggaatatgca gcacattttt gccttcttct gcaccggttt cctaggcgcg gtagtaggtg 420

ccaatttccc caacaatatc cagatcgggg gattatttcc aaaccagcag tcacaggaac 480

atgctgcttt tagatttgct ttgtcgcaac tcacagagcc cccgaagctg ctcccccaga 540

ttgatattgt gaacatcagc gacagctttg agatgaccta tagattctgt tcccagttct 600

ccaaaggagt ctatgccatc tttgggtttt atgaacgtag gactgtcaac atgctgacct 660

ccttttgtgg ggccctccac gtctgcttca ttacgccgag ctttcccgtt gatacatcca 720

atcagtttgt ccttcagctg cgccctgaac tgcaggatgc cctcatcagc atcattgacc 780

attacaagtg gcagaaattt gtctacattt atgatgccga ccggggctta tccgtcctgc 840

agaaagtcct ggatacagct gctgagaaga actggcaggt gacagcagtc aacattttga 900

caaccacaga ggagggatac cggatgctct ttcaggacct ggagaagaaa aaggagcggc 960

tggtggtggt ggactgtgaa tcagaacgcc tcaatgctat cttgggccag attataaagc 1020

tagagaagaa tggcatcggc taccactaca ttcttgcaaa tctgggcttc atggacattg 1080

acttaaacaa attcaaggag agtggcgcca atgtgacagg tttccagctg gtgaactaca 1140

cagacactat tccggccaag atcatgcagc agtggaagaa tagtgatgct cgagaccaca 1200

cacgggtgga ctggaagaga cccaagtaca cctctgcgct cacctacgat ggggtgaagg 1260

tgatggctga ggctttccag agcctgcgga ggcagagaat tgatatatct cgccggggga 1320

atgctgggga ttgtctggct aacccagctg ttccctgggg ccaagggatc gacatccaga 1380

gagctctgca gcaggtgcga tttgaaggtt taacaggaaa cgtgcagttt aatgagaaag 1440

gacgccggac caactacacg ctccacgtga ttgaaatgaa acatgacggc atccgaaaga 1500

ttggttactg gaatgaagat gataagtttg tccctgcagc caccgatgcc caagctgggg 1560

gcgataattc aagtgttcag aacagaacat acatcgtcac aacaatccta gaagatcctt 1620

atgtgatgct caagaagaac gccaatcagt ttgagggcaa tgaccgttac gagggctact 1680

gtgtagagct ggcggcagag attgccaagc acgtgggcta ctcctaccgt ctggagattg 1740

tcagtgatgg aaaatacgga gcccgagacc ctgacacgaa ggcctggaat ggcatggtgg 1800

gagagctggt ctatggaaga gcagatgtgg ctgtggctcc cttaactatc actttggtcc 1860

gggaagaagt tatagatttc tccaaaccat ttatgagttt ggggatctcc atcatgatta 1920

aaaaaccaca gaaatccaag ccgggtgtct tctccttcct tgatcctttg gcttatgaga 1980

tttggatgtg cattgttttt gcctacattg gagtgagtgt tgtcctcttc ctggtcagcc 2040

gcttcagtcc ctatgaatgg cacagtgaag agtttgagga aggacgggac cagacaacca 2100

gtgaccagtc caatgagttt gggatattca acagtttgtg gttctccctg ggagccttca 2160

tgcagcaagg atgtgacatt tctcccaggt ccctgtctgg tcgcatcgtt ggtggcgtct 2220

ggtggttctt caccttaatc atcatctcct catatacagc caatctggcc gccttcctga 2280

ccgtggagag gatggtgtct cccattgaga gtgcagagga cctagcgaag cagacagaaa 2340

ttgcctacgg gacgctggaa gcaggatcta ctaaggagtt cttcaggagg tctaaaattg 2400

ctgtgtttga gaagatgtgg acatacatga agtcagcaga gccatcagtt tttgtgcgga 2460

ccacagagga ggggatgatt cgagtgagga aatccaaagg caaatatgcc tacctcctgg 2520

agtccaccat gaatgagtac attgagcagc ggaaaccctg tgacaccatg aaggtgggag 2580

gtaacttgga ttccaaaggc tatggcattg caacacccaa ggggtctgcc ctgagaggtc 2640

ccgtaaacct agcggttttg aaactcagtg agcaaggcgt cttagacaag ctgaaaagca 2700

aatggtggta cgataaaggg gaatgtggaa gcaaggactc cggaagtaag gacaagacaa 2760

gcgctctgag cctcagcaat gtggcaggcg tgttctacat cctgatcgga ggacttggac 2820

tagccatgct ggttgcctta atcgagttct gctacaaatc ccgtagtgaa tccaagcgga 2880

tgaagggttt ttgtttgatc ccacagcaat ccatcaacga agccatacgg acatcgaccc 2940

tcccccgcaa cagcggggca ggagccagca gcggcggcag tggagagaat ggtcgggtgg 3000

tcagccatga cttccccaag tccatgcaat cgattccttg catgagccac agttcaggga 3060

tgcccttggg agccacggga ttgtaactgg agcagatgga gaccccttgg ggagcaggct 3120

cgggctcccc agccccatcc caaacccttc agtgccaaaa acaacaacaa aatgaaacgc 3180

aaccaccacc aaccactgcg accacaagaa ggatgattca acaggtttcc ctgaagaatt 3240

gaaaaaccat tttgctgtcc cttttccttt tttgatgttc tttcaccctt ttctgtttgc 3300

taagtgagga tgaaaaaata acactgtact gcaataaggg gagagtaacc ctgtctaatg 3360

aaacctgtgt ctctgagagt agagtcactg gaacactaat gaggaaactg cactgtttta 3420

ttttaattca gttgttagtg tgtcttagtg tgtgcaattt tttttcttac taatatccat 3480

ggtttgcagg ttctgttagg ccctttcctt ctccttactt cttatcccca actccctacc 3540

cacccctctt cagttttcag attggagatt caagatttgt tccactttac aagcaagagg 3600

aaaaaaaagc aaccttcaaa ctaattctcc atgggggctc tccatgttac cctccactcc 3660

ttggcccaaa cctctgatgg agatagacat tgttggagaa gtgggctgcc ttccccaagt 3720

ggggcactgc ttaagcactt attcagtgga gaacacaggt gaaaagcaac tcaggatgag 3780

ggtggtggag agggcagggg cagatgtgca gtcagagaag gactcctgaa gttactgctg 3840

ctcagaaaaa cagttccttt aatgtggaag agccatttca taggtcatag gtggtatggt 3900

atatttcttc agagtcaacc ttggccctga gaagtatgtc ctcctggtgt gctcaggctc 3960

aacggcagtc tggtggctga aggcacttgg cctcctaaac caagcagaat tttgggaaga 4020

gataacagcc agggagatat tgcccatgat tctcactttt tctttgcctg gcatctaagc 4080

aggaacccat tgtggagtag actctcttct tctatggagc ctctgacatg gggagcaatg 4140

ctaagcaagc taagtgtaaa agaaaagtga cagaataatt ttggaagagg aagcctcatc 4200

aaaagctcac acaaaataga gcttcccatg gtgtgcccta tcctaggttt aagaaaacac 4260

gtatgaagtt tatgctgatg caaagaactt gggtttttat gttaatataa agtgttgttt 4320

tagcatgtgg ccagatgatg ctctgtcatc tttagaaagt gagataacca aggaaataat 4380

tgaaggagta tagggagatg gattaagttg ataatgacat ttagggcaac ttaagacctt 4440

tgatcccagg ttctaactca aagaggctga ccttccccca gctaagatag catgaggacg 4500

ttgtattcca atatacgtat gattggggct acaaagctga actaaagcaa gattggtgaa 4560

gtggcagggt ttatagagag aagcccaggc tgagttcagc ttttgttgga agtgagaatc 4620

cctgacatat agctttcttg gagatcccaa ctctcattct tggtgcaact ggcttccagc 4680

tctccagcag tcactctcct aggtgcatga ttcagtgcgt gccatgtgtc attagctttt 4740

actgataacc atattctggc ttgttccctt accccctact tctatccaat tttctctgct 4800

aggggttatc attagcaatt gacatgctaa aggttttgga gcccacctag gggtaggtgc 4860

agctttattg gcttttctgt ggattctctc agtggaccca caccatctct atgtctctcc 4920

actctcctgc cttcagccat agcaaagaat ccttccaaaa tcaaactctt cacttttttg 4980

actcaagtgt tgttgttcag tctctcgcgt gtcaatgtgg tcatggttca tgaaaccgga 5040

ccctcaagat ggatgattgc ttttaactac tgccagctga tgtctctcag cccctgccct 5100

catacaagat ttttctcagc cttcagccta ccactgcaga atccgatgtg acccaccatt 5160

agggagtctg catcttggaa gagttggaaa taacccttta acatcaacat gcttcaaaga 5220

ctttttgcct ttggcctagt aagatgcctc tccagctact gagcccacaa gtaacatgag 5280

cggataaaaa gagacttgtt tgtgctagaa atgagggtct atgctatgag ggggtccaag 5340

actctggcga aatgtgcttt ttcatcaatg gagaaatgaa aggaaaacac aagcaagaaa 5400

aaagttaact tgtattatgt atttttacta cacttttctt aaaaatagag cattgggaaa 5460

actctgaaag agactgacat ttttctcaac aggaatccat acttaacagt tctggctttc 5520

attaaatttt gctctttggt acctgggcct tttatttaac atctatattt gttttaactc 5580

tcttggcaga tgtgtgaaag gattcttgct tgatcaaaca ctaagtattt ttttggttct 5640

tgtttttctt tcaaatagcc aggttttttt cttttggtat ttgcataaaa tgaaaatatc 5700

accgaatatt aaatcactgt ggatccatta aaaaaaaaaa aaaaaaa 5747

<210> 3
<211> 5755
<212> DNA

<213>  Homo sapiens

<400>  3
gagtcgcgca cgcgcgcccg ggactgcctg cccctctctg tgacttgcct gtgtgtgtgc      60

gtgtgtgtat gtgtgtgtgt gtgtgtgtgt gcgcgcgcgc gtgagtgaga gaggagagag     120

ggagaagaga gcgcgagaga gggtgagtgt gtgtgagtgc atgggagggt gctgaatatt     180

ccgagacact gggaccacag cggcagctcc gctgaaaact gcattcagcc agtcctccgg     240

acttctggag cggggacagg gcgcagggca tcagcagcca ccagcaggac ctgggaaata     300

gggattcttc tgcctccact tcaggtttta gcagcttggt gctaaattgc tgtctcaaaa     360

tgcagaggat ctaatttgca gaggaaaaca gccaaagaag gaagaggagg aaaaggaaaa     420

aaaaaggggt atattgtgga tgctctactt ttcttggaaa tgcaaaagat tatgcatatt     480

tctgtcctcc tttctcctgt tttatgggga ctgatttttg gtgtctcttc taacagcata     540

cagatagggg ggctatttcc taggggcgcc gatcaagaat acagtgcatt tcgagtaggg     600

atggttcagt tttccacttc ggagttcaga ctgacacccc acatcgacaa tttggaggtg     660

gcaaacagct tcgcagtcac taatgctttc tgctcccagt tttcgagagg agtctatgct     720

atttttggat tttatgacaa gaagtctgta aataccatca catcattttg cggaacactc     780

cacgtctcct tcatcactcc cagcttccca acagatggca cacatccatt tgtcattcag     840

atgagacccg acctcaaagg agctctcctt agcttgattg aatactatca atgggacaag     900

tttgcatacc tctatgacag tgacagaggc ttatcaacac tgcaagctgt gctggattct     960

gctgctgaaa agaaatggca agtgactgct atcaatgtgg gaaacattaa caatgacaag    1020

aaagatgaga tgtaccgatc actttttcaa gatctggagt taaaaaagga acggcgtgta    1080

attctggact gtgaaaggga taaagtaaac gacattgtag accaggttat taccattgga    1140

aaacatgtta aagggtacca ctacatcatt gcaaatctgg gatttactga tggagaccta    1200

ttaaaaatcc agtttggagg tgcaaatgtc tctggatttc agatagtgga ctatgatgat    1260

tcgttggtat ctaaatttat agaaagatgg tcaacactgg aagaaaaaga ataccctgga    1320

gctcacacaa caacaattaa gtatacttct gctctgacct atgatgccgt tcaagtgatg    1380

actgaagcct tccgcaacct aaggaagcaa agaattgaaa tctcccgaag ggggaatgca    1440

ggagactgtc tggcaaaccc agcagtgccc tggggacaag gtgtagaaat agaaagggcc    1500

ctcaaacagg ttcaggttga aggtctctca ggaaatataa agtttgacca gaatggaaaa    1560

agaataaact atacaattaa catcatggag ctcaaaacta atgggccccg gaagattggc    1620

tactggagtg aagtggacaa aatggttgtt acccttactg agctcccttc tggaaatgac    1680

acctctgggc ttgagaataa gactgttgtt gtcaccacaa ttttggaatc tccgtatgtt 1740

atgatgaaga aaaatcatga aatgcttgaa ggcaatgagc gctatgaggg ctactgtgtt 1800

gacctggctg cagaaatcgc caaacattgt gggttcaagt acaagttgac aattgttggt 1860

gatggcaagt atggggccag ggatgcagac acgaaaattt ggaatgggat ggttggagaa 1920

cttgtatatg ggaaagctga tattgcaatt gctccattaa ctattaccct tgtgagagaa 1980

gaggtgattg acttctcaaa gcccttcatg agcctcggga tatctatcat gatcaagaag 2040

cctcagaagt ccaaaccagg agtgttttcc tttcttgatc ctttagccta tgagatctgg 2100

atgtgcattg tttttgccta cattggggtc agtgtagttt tattcctggt cagcagattt 2160

agcccctacg agtggcacac tgaggagttt gaagatggaa gagaaacaca aagtagtgaa 2220

tcaactaatg aatttgggat ttttaatagt ctctggtttt ccttgggtgc ctttatgcgg 2280

caaggatgcg atatttcgcc aagatccctc tctgggcgca ttgttggagg tgtgtggtgg 2340

ttctttaccc tgatcataat ctcctcctac acggctaact tagctgcctt cctgactgta 2400

gagaggatgg tgtctcccat cgaaagtgct gaggatcttt ctaagcaaac agaaattgct 2460

tatggaacat tagactctgg ctccactaaa gagtttttca ggagatctaa aattgcagtg 2520

tttgataaaa tgtggaccta catgcggagt gcggagccct ctgtgtttgt gaggactacg 2580

gccgaagggg tggctagagt gcggaagtcc aaagggaaat atgcctactt gttggagtcc 2640

acgatgaacg agtacattga gcaaaggaag ccttgcgaca ccatgaaagt tggtggaaac 2700

ctggattcca aaggctatgg catcgcaaca cctaaaggat cctcattaag aaatgcggtt 2760

aacctcgcag tactaaaact gaatgaacaa ggcctgttgg acaaattgaa aaacaaatgg 2820

tggtacgaca aaggagagtg cggcagcggg ggaggtgatt ccaaggaaaa gaccagtgcc 2880

ctcagtctga gcaacgttgc tggagtattc tacatccttg tcgggggcct tggtttggca 2940

atgctggtgg ctttgattga gttctgttac aagtcaaggg ccgaggcgaa acgaatgaag 3000

gtggcaaaga atgcacagaa tattaaccca tcttcctcgc agaattcaca gaattttgca 3060

acttataagg aaggttacaa cgtatatggc atcgaaagtg ttaaaattta ggggatgacc 3120

ttgaatgatg ccatgaggaa caaggcaagg ctgtcaatta caggaagtac tggagaaaat 3180

ggacgtgtta tgactccaga atttcccaaa gcagtgcatg ctgtccctta cgtgagtcct 3240

ggcatgggaa tgaatgtcag tgtgactgat ctctcgtgat tgataagaac cttttgagtg 3300

ccttacacaa tggttttctt gtgtgtttat tgtcaaagtg gtgagaggca tccagtatct 3360

tgaagacttt tctttcagcc aagaattctt aaatatgtgg agttcatctt gaattgtaag 3420

gaatgattaa ttaaaacaca acatcttttt ctactcgagt tacagacaaa gcgtggtgga 3480

catgcacagc taacatggaa gtactataat ttacctgaag tctttgtaca gacaacaaac 3540

ctgtttctgc agccactatt gttagtctct tgattcataa tgacttaagc acacttgaca 3600

tcaactgcat caagatgtga catgttttat aaaaaaagga aaaaaaacat ttaaaactaa 3660

aaaatatttt taggtatttt cacaaacaaa ctggctttta aataaatttg cttccatatt 3720

ggttgaataa gacaaaaaca attaaactga gtgggaagtg aataaaaaaa ggctttaggt 3780

atcgattcca tatttttcaa agccaaatat gtaaatgcta aggaaagtaa acaaagagga 3840

gattccaatc ttgtaattta atattgttat taaaacttta atgtatccta ttctttaaca 3900

tttggtgtta atataaaatt acttggcaat gcttgacatt tgaaataaac atttttctat 3960

tgttttattg caagtggtcc aattaatttt gcttagctac agtttggtca taaatcaagt 4020

gagtttaaag acactaccaa gttgttaggt gcccagagaa aatttctccc ttttaaaaag 4080

gccaggtgat ttttcaaatg taatcttgcc cccaaagtaa tatctgaata tctttttgac 4140

atgtctaaat atatatatat ataaagaaat atttgttaac acaaaagcat ttgatctatg 4200

tagataaatg ctaatagatt taaaaagcta atattaacaa ataccagaat acgtgaagtt 4260

ccatttttaa agtgtttgag cttacagaag agaaacattc attttaaatg aagtaaaaaa 4320

tgccttgaaa gtaattcttt agatagttgc ccattgatta aattccaaaa actaaatatg 4380

tttttagctt taaaattata aaagctgtca taaactttat atattatgaa ttttaaaata 4440

tgtttgagtc tcctgcaata tagtttcatc ccattgacat caattaaaaa taaccctaat 4500

atattatttt tatatttatt cctcaggtgg aatggctatt ttaatatgcc cagtgtggat 4560

aaaatgtcac atttctgtaa cttttgacta aagagcctat atttatctag ttaatgaatt 4620

taaaggatct atctttccct tcataaaata cctcttattt ccattaaagc cccccaagtt 4680

taattaattt aggattttga atgattattg acatccaata gttattttta atatttgtat 4740

tcttgttatt tctggaagaa agcctttgtg tagcacttgg tattttgcaa agtgctttta 4800

aaacattctt acttaccgta tttcatagaa gggaaggaaa aatgtaaggt ttaacagtaa 4860

gcacttgcat tgaacatgga ggcatgtggt atcatgatat tcttcactaa atttagctgt 4920

ccctaatcac agatcctaag gtaatataat ataattttag tgcatttctc ctcatcagga 4980

atgctggagg tgcattttaa gttttaataa taagtgctag aatgaccaaa ttgcagacta 5040

attgtttcca tattgtactt aaaatgagtt tttaaaagtg aaaaagaaat gactatatac 5100

aatcaatgct atttattgta cctctgggcc tactcttcta aaaattgtag cttatcgatt 5160

tttctctgtc aagcttgaac taatgtaaat aattgaaata atgtaaagtt atattttcat 5220

gtttttatag atacaacatg acaagaatac ataatgtaag agtatttcaa ctatggataa 5280

tgttgattgg ataatgcaca tctcagttac aagcagtact catagtttaa tatccatgta 5340

acggtgcatc aatatattgc tatataaata tgtctgtgtg catataagtg aaaagtggtc 5400

aaacaagagt gatgacagct gtctaaaggt ttttttattc attttatata aaaactgtta 5460

tggaaagacc aaaatgttta tgaactattc ttatgtaaat ttacaattgt cctttactgt 5520

acttttttgt ttacagtata gtaccttatt ttctgctgtg ttaagtgggt gtcaaactcc 5580

aagaagacat acactttcta taacttctat tgaagatatt ggaatttcca atttttcatg 5640

tgtactatgt cagaaaatgc tttcgatttt atttttaaat ctaacatcgg atggcttttc 5700

cggagtgttg taaaaacttc aatcatacat aaaacatgtt cttacaaaag gcaaa 5755

<210> 4
<211> 5755
<212> DNA
<213> Homo sapiens

<400> 4
gagtcgcgca cgcgcgcccg ggactgcctg cccctctctg tgacttgcct gtgtgtgtgc 60

gtgtgtgtat gtgtgtgtgt gtgtgtgtgt gcgcgcgcgc gtgagtgaga gaggagagag 120

ggagaagaga gcgcgagaga gggtgagtgt gtgtgagtgc atgggagggt gctgaatatt 180

ccgagacact gggaccacag cggcagctcc gctgaaaact gcattcagcc agtcctccgg 240

acttctggag cggggacagg gcgcagggca tcagcagcca ccagcaggac ctgggaaata 300

gggattcttc tgcctccact tcaggtttta gcagcttggt gctaaattgc tgtctcaaaa 360

tgcagaggat ctaatttgca gaggaaaaca gccaaagaag gaagaggagg aaaaggaaaa 420

aaaaaggggt atattgtgga tgctctactt ttcttggaaa tgcaaaagat tatgcatatt 480

tctgtcctcc tttctcctgt tttatgggga ctgatttttg gtgtctcttc taacagcata 540

cagatagggg ggctatttcc taggggcgcc gatcaagaat acagtgcatt tcgagtaggg 600

atggttcagt tttccacttc ggagttcaga ctgacacccc acatcgacaa tttggaggtg 660

gcaaacagct tcgcagtcac taatgctttc tgctcccagt tttcgagagg agtctatgct 720

atttttggat tttatgacaa gaagtctgta aataccatca catcattttg cggaacactc 780

cacgtctcct tcatcactcc cagcttccca acagatggca cacatccatt tgtcattcag 840

atgagacccg acctcaaagg agctctcctt agcttgattg aatactatca atgggacaag 900

tttgcatacc tctatgacag tgacagaggc ttatcaacac tgcaagctgt gctggattct 960

gctgctgaaa agaaatggca agtgactgct atcaatgtgg gaaacattaa caatgacaag 1020

aaagatgaga tgtaccgatc actttttcaa gatctggagt taaaaaagga acggcgtgta 1080

attctggact gtgaaaggga taaagtaaac gacattgtag accaggttat taccattgga 1140

aaacatgtta aagggtacca ctacatcatt gcaaatctgg gatttactga tggagaccta 1200

ttaaaaatcc agtttggagg tgcaaatgtc tctggatttc agatagtgga ctatgatgat 1260

tcgttggtat ctaaatttat agaaagatgg tcaacactgg aagaaaaaga ataccctgga 1320

gctcacacaa caacaattaa gtatacttct gctctgacct atgatgccgt tcaagtgatg 1380

actgaagcct tccgcaacct aaggaagcaa agaattgaaa tctcccgaag ggggaatgca 1440

ggagactgtc tggcaaaccc agcagtgccc tggggacaag gtgtagaaat agaaagggcc 1500

ctcaaacagg ttcaggttga aggtctctca ggaaatataa agtttgacca gaatggaaaa 1560

agaataaact atacaattaa catcatggag ctcaaaacta atgggccccg gaagattggc 1620

tactggagtg aagtggacaa aatggttgtt acccttactg agctcccttc tggaaatgac 1680

acctctgggc ttgagaataa gactgttgtt gtcaccacaa ttttggaatc tccgtatgtt 1740

atgatgaaga aaaatcatga aatgcttgaa ggcaatgagc gctatgaggg ctactgtgtt 1800

gacctggctg cagaaatcgc caaacattgt gggttcaagt acaagttgac aattgttggt 1860

gatggcaagt atggggccag ggatgcagac acgaaaattt ggaatgggat ggttggagaa 1920

cttgtatatg ggaaagctga tattgcaatt gctccattaa ctattaccct tgtgagagaa 1980

gaggtgattg acttctcaaa gcccttcatg agcctcggga tatctatcat gatcaagaag 2040

cctcagaagt ccaaaccagg agtgttttcc tttcttgatc ctttagccta tgagatctgg 2100

atgtgcattg tttttgccta cattggggtc agtgtagttt tattcctggt cagcagattt 2160

agcccctacg agtggcacac tgaggagttt gaagatggaa gagaaacaca aagtagtgaa 2220

tcaactaatg aatttgggat ttttaatagt ctctggtttt ccttgggtgc ctttatgcgg 2280

caaggatgcg atatttcgcc aagatccctc tctgggcgca ttgttggagg tgtgtggtgg 2340

ttctttaccc tgatcataat ctcctcctac acggctaact tagctgcctt cctgactgta 2400

gagaggatgg tgtctcccat cgaaagtgct gaggatcttt ctaagcaaac agaaattgct 2460

tatggaacat tagactctgg ctccactaaa gagtttttca ggagatctaa aattgcagtg 2520

tttgataaaa tgtggaccta catgcggagt gcggagccct ctgtgtttgt gaggactacg 2580

gccgaagggg tggctagagt gcggaagtcc aaagggaaat atgcctactt gttggagtcc 2640

acgatgaacg agtacattga gcaaaggaag ccttgcgaca ccatgaaagt tggtggaaac 2700

ctggattcca aaggctatgg catcgcaaca cctaaaggat cctcattaag aaccccagta 2760

aatcttgcag tattgaaact cagtgagcaa ggcgtcttag acaagctgaa aaacaaatgg 2820

tggtacgata aaggtgaatg tggagccaag gactctggaa gtaaggaaaa gaccagtgcc 2880

ctcagtctga gcaacgttgc tggagtattc tacatccttg tcgggggcct tggtttggca 2940

atgctggtgg ctttgattga gttctgttac aagtcaaggg ccgaggcgaa acgaatgaag 3000

gtggcaaaga atgcacagaa tattaaccca tcttcctcgc agaattcaca gaattttgca 3060

acttataagg aaggttacaa cgtatatggc atcgaaagtg ttaaaattta ggggatgacc 3120

ttgaatgatg ccatgaggaa caaggcaagg ctgtcaatta caggaagtac tggagaaaat 3180

ggacgtgtta tgactccaga atttcccaaa gcagtgcatg ctgtccctta cgtgagtcct 3240

ggcatgggaa tgaatgtcag tgtgactgat ctctcgtgat tgataagaac cttttgagtg 3300

ccttacacaa tggttttctt gtgtgtttat tgtcaaagtg gtgagaggca tccagtatct 3360

tgaagacttt tctttcagcc aagaattctt aaatatgtgg agttcatctt gaattgtaag 3420

gaatgattaa ttaaaacaca acatcttttt ctactcgagt tacagacaaa gcgtggtgga 3480

catgcacagc taacatggaa gtactataat ttacctgaag tctttgtaca gacaacaaac 3540

ctgtttctgc agccactatt gttagtctct tgattcataa tgacttaagc acacttgaca 3600

tcaactgcat caagatgtga catgttttat aaaaaaagga aaaaaaacat ttaaaactaa 3660

aaaatatttt taggtatttt cacaaacaaa ctggctttta aataaatttg cttccatatt 3720

ggttgaataa gacaaaaaca attaaactga gtgggaagtg aataaaaaaa ggctttaggt 3780

atcgattcca tatttttcaa agccaaatat gtaaatgcta aggaaagtaa acaaagagga 3840

gattccaatc ttgtaattta atattgttat taaaacttta atgtatccta ttctttaaca 3900

tttggtgtta atataaaatt acttggcaat gcttgacatt tgaaataaac atttttctat 3960

tgttttattg caagtggtcc aattaatttt gcttagctac agtttggtca taaatcaagt 4020

gagtttaaag acactaccaa gttgttaggt gcccagagaa aatttctccc ttttaaaaag 4080

gccaggtgat ttttcaaatg taatcttgcc cccaaagtaa tatctgaata tctttttgac 4140

atgtctaaat atatatatat ataaagaaat atttgttaac acaaaagcat ttgatctatg 4200

tagataaatg ctaatagatt taaaaagcta atattaacaa ataccagaat acgtgaagtt 4260

ccatttttaa agtgtttgag cttacagaag agaaacattc attttaaatg aagtaaaaaa 4320

tgccttgaaa gtaattcttt agatagttgc ccattgatta aattccaaaa actaaatatg 4380

tttttagctt taaaattata aaagctgtca taaactttat atattatgaa ttttaaaata 4440

tgtttgagtc tcctgcaata tagtttcatc ccattgacat caattaaaaa taaccctaat 4500

atattatttt tatatttatt cctcaggtgg aatggctatt ttaatatgcc cagtgtggat 4560

aaaatgtcac atttctgtaa cttttgacta aagagcctat atttatctag ttaatgaatt 4620

taaaggatct atctttccct tcataaaata cctcttattt ccattaaagc cccccaagtt 4680

taattaattt aggattttga atgattattg acatccaata gttattttta atatttgtat 4740

tcttgttatt tctggaagaa agcctttgtg tagcacttgg tattttgcaa agtgctttta 4800

aaacattctt acttaccgta tttcatagaa gggaaggaaa aatgtaaggt ttaacagtaa 4860

gcacttgcat tgaacatgga ggcatgtggt atcatgatat tcttcactaa atttagctgt 4920

ccctaatcac agatcctaag gtaatataat ataattttag tgcatttctc ctcatcagga 4980

atgctggagg tgcattttaa gttttaataa taagtgctag aatgaccaaa ttgcagacta 5040

attgtttcca tattgtactt aaaatgagtt tttaaaagtg aaaaagaaat gactatatac 5100

aatcaatgct atttattgta cctctgggcc tactcttcta aaaattgtag cttatcgatt 5160

tttctctgtc aagcttgaac taatgtaaat aattgaaata atgtaaagtt atattttcat 5220

gtttttatag atacaacatg acaagaatac ataatgtaag agtatttcaa ctatggataa 5280

tgttgattgg ataatgcaca tctcagttac aagcagtact catagtttaa tatccatgta 5340

acggtgcatc aatatattgc tatataaata tgtctgtgtg catataagtg aaaagtggtc 5400

aaacaagagt gatgacagct gtctaaaggt ttttttattc attttatata aaaactgtta 5460

tggaaagacc aaaatgttta tgaactattc ttatgtaaat ttacaattgt cctttactgt 5520

acttttttgt ttacagtata gtaccttatt ttctgctgtg ttaagtgggt gtcaaactcc 5580

aagaagacat acactttcta taacttctat tgaagatatt ggaatttcca atttttcatg 5640

tgtactatgt cagaaaatgc tttcgatttt atttttaaat ctaacatcgg atggcttttc 5700

cggagtgttg taaaaacttc aatcatacat aaaacatgtt cttacaaaag gcaaa 5755

<210> 5
<211> 5266
<212> DNA
<213> Homo sapiens

<400> 5
gtgtgtgcgc gcgcgcgtga gtgagagagg agagagggag aagagagcgc gagagagggg 60

gggctatttc ctaggggcgc cgatcaagaa tacagtgcat ttcgagtagg gatggttcag 120

ttttccactt cggagttcag actgacaccc cacatcgaca atttggaggt ggcaaacagc 180

ttcgcagtca ctaatgcttt ctgctcccag ttttcgagag gagtctatgc tatttttgga 240

ttttatgaca agaagtctgt aaataccatc acatcatttt gcggaacact ccacgtctcc 300

ttcatcactc ccagcttccc aacagatggc acacatccat ttgtcattca gatgagaccc 360

gacctcaaag gagctctcct tagcttgatt gaatactatc aatgggacaa gtttgcatac 420
ctctatgaca gtgacagagg cttatcaaca ctgcaagctg tgctggattc tgctgctgaa 480
aagaaatggc aagtgactgc tatcaatgtg ggaaacatta acaatgacaa gaaagatgag 540
atgtaccgat cactttttca agatctggag ttaaaaaagg aacggcgtgt aattctggac 600
tgtgaaaggg ataaagtaaa cgacattgta gaccaggtta ttaccattgg aaaacatgtt 660
aaagggtacc actacatcat tgcaaatctg ggatttactg atggagacct attaaaaatc 720
cagtttggag gtgcaaatgt ctctggattt cagatagtgg actatgatga ttcgttggta 780
tctaaattta tagaaagatg gtcaacactg gaagaaaaag aataccctgg agctcacaca 840
acaacaatta agtatacttc tgctctgacc tatgatgccg ttcaagtgat gactgaagcc 900
ttccgcaacc taaggaagca aagaattgaa atctcccgaa gggggaatgc aggagactgt 960
ctggcaaacc cagcagtgcc ctggggacaa ggtgtagaaa tagaaagggc cctcaaacag 1020
gttcaggttg aaggtctctc aggaaatata aagtttgacc agaatggaaa aagaataaac 1080
tatacaatta acatcatgga gctcaaaact aatgggcccc ggaagattgg ctactggagt 1140
gaagtggaca aaatggttgt tacccttact gagctccctt ctggaaatga cacctctggg 1200
cttgagaata agactgttgt tgtcaccaca attttggaat ctccgtatgt tatgatgaag 1260
aaaaatcatg aaatgcttga aggcaatgag cgctatgagg gctactgtgt tgacctggct 1320
gcagaaatcg ccaaacattg tgggttcaag tacaagttga caattgttgg tgatggcaag 1380
tatggggcca gggatgcaga cacgaaaatt tggaatggga tggttggaga acttgtatat 1440
gggaaagctg atattgcaat tgctccatta actattaccc ttgtgagaga agaggtgatt 1500
gacttctcaa agcccttcat gagcctcggg atatctatca tgatcaagaa gcctcagaag 1560
tccaaaccag gagtgttttc ctttcttgat cctttagcct atgagatctg gatgtgcatt 1620
gtttttgcct acattggggt cagtgtagtt ttattcctgg tcagcagatt tagcccctac 1680
gagtggcaca ctgaggagtt tgaagatgga agagaaacac aaagtagtga atcaactaat 1740
gaatttggga tttttaatag tctctggttt tccttgggtg cctttatgcg gcaaggatgc 1800
gatatttcgc caagatccct ctctgggcgc attgttggag gtgtgtggtg gttctttacc 1860
ctgatcataa tctcctccta cacggctaac ttagctgcct tcctgactgt agagaggatg 1920
gtgtctccca tcgaaagtgc tgaggatctt tctaagcaaa cagaaattgc ttatggaaca 1980
ttagactctg gctccactaa agagtttttc aggagatcta aaattgcagt gtttgataaa 2040
atgtggacct acatgcggag tgcggagccc tctgtgtttg tgaggactac ggccgaaggg 2100
gtggctagag tgcggaagtc caaagggaaa tatgcctact tgttggagtc cacgatgaac 2160

gagtacattg agcaaaggaa gccttgcgac accatgaaag ttggtggaaa cctggattcc 2220

aaaggctatg gcatcgcaac acctaaagga tcctcattaa gaaccccagt aaatcttgca 2280

gtattgaaac tcagtgagca aggcgtctta gacaagctga aaaacaaatg gtggtacgat 2340

aaaggtgaat gtggagccaa ggactctgga agtaaggaaa agaccagtgc cctcagtctg 2400

agcaacgttg ctggagtatt ctacatcctt gtcgggggcc ttggtttggc aatgctggtg 2460

gctttgattg agttctgtta caagtcaagg gccgaggcga aacgaatgaa ggtggcaaag 2520

aatgcacaga atattaaccc atcttcctcg cagaattcac agaattttgc aacttataag 2580

gaaggttaca acgtatatgg catcgaaagt gttaaaattt aggggatgac cttgaatgat 2640

gccatgagga acaaggcaag gctgtcaatt acaggaagta ctggagaaaa tggacgtgtt 2700

atgactccag aatttcccaa agcagtgcat gctgtccctt acgtgagtcc tggcatggga 2760

atgaatgtca gtgtgactga tctctcgtga ttgataagaa ccttttgagt gccttacaca 2820

atggttttct tgtgtgttta ttgtcaaagt ggtgagaggc atccagtatc ttgaagactt 2880

ttctttcagc caagaattct taaatatgtg gagttcatct tgaattgtaa ggaatgatta 2940

attaaaacac aacatctttt tctactcgag ttacagacaa agcgtggtgg acatgcacag 3000

ctaacatgga agtactataa tttacctgaa gtctttgtac agacaacaaa cctgtttctg 3060

cagccactat tgttagtctc ttgattcata atgacttaag cacacttgac atcaactgca 3120

tcaagatgtg acatgtttta taaaaaaagg aaaaaaaaca tttaaaacta aaaaatattt 3180

ttaggtattt tcacaaacaa actggctttt aaataaattt gcttccatat tggttgaata 3240

agacaaaaac aattaaactg agtgggaagt gaataaaaaa aggctttagg tatcgattcc 3300

atatttttca aagccaaata tgtaaatgct aaggaaagta aacaaagagg agattccaat 3360

cttgtaattt aatattgtta ttaaaacttt aatgtatcct attctttaac atttggtgtt 3420

aatataaaat tacttggcaa tgcttgacat ttgaaataaa catttttcta ttgttttatt 3480

gcaagtggtc caattaattt tgcttagcta cagtttggtc ataaatcaag tgagtttaaa 3540

gacactacca agttgttagg tgcccagaga aaatttctcc cttttaaaaa ggccaggtga 3600

tttttcaaat gtaatcttgc ccccaaagta atatctgaat atctttttga catgtctaaa 3660

tatatatata tataaagaaa tatttgttaa cacaaaagca tttgatctat gtagataaat 3720

gctaatagat ttaaaaagct aatattaaca aataccagaa tacgtgaagt tccattttta 3780

aagtgtttga gcttacagaa gagaaacatt cattttaaat gaagtaaaaa atgccttgaa 3840

agtaattctt tagatagttg cccattgatt aaattccaaa aactaaatat gtttttagct 3900

ttaaaattat aaaagctgtc ataaacttta tatattatga attttaaaat atgtttgagt 3960

ctcctgcaat atagtttcat cccattgaca tcaattaaaa ataaccctaa tatattattt 4020

ttatatttat tcctcaggtg gaatggctat tttaatatgc ccagtgtgga taaaatgtca 4080

catttctgta acttttgact aaagagccta tatttatcta gttaatgaat ttaaaggatc 4140

tatctttccc ttcataaaat acctcttatt tccattaaag ccccccaagt ttaattaatt 4200

taggattttg aatgattatt gacatccaat agttattttt aatatttgta ttcttgttat 4260

ttctggaaga aagcctttgt gtagcacttg gtattttgca aagtgctttt aaaacattct 4320

tacttaccgt atttcataga agggaaggaa aaatgtaagg tttaacagta agcacttgca 4380

ttgaacatgg aggcatgtgg tatcatgata ttcttcacta aatttagctg tccctaatca 4440

cagatcctaa ggtaatataa tataatttta gtgcatttct cctcatcagg aatgctggag 4500

gtgcatttta agttttaata ataagtgcta gaatgaccaa attgcagact aattgtttcc 4560

atattgtact taaaatgagt ttttaaaagt gaaaaagaaa tgactatata caatcaatgc 4620

tatttattgt acctctgggc ctactcttct aaaaattgta gcttatcgat ttttctctgt 4680

caagcttgaa ctaatgtaaa taattgaaat aatgtaaagt tatattttca tgtttttata 4740

gatacaacat gacaagaata cataatgtaa gagtatttca actatggata atgttgattg 4800

gataatgcac atctcagtta caagcagtac tcatagttta atatccatgt aacggtgcat 4860

caatatattg ctatataaat atgtctgtgt gcatataagt gaaaagtggt caaacaagag 4920

tgatgacagc tgtctaaagg tttttttatt cattttatat aaaaactgtt atggaaagac 4980

caaaatgttt atgaactatt cttatgtaaa tttacaattg tcctttactg tacttttttg 5040

tttacagtat agtaccttat tttctgctgt gttaagtggg tgtcaaactc caagaagaca 5100

tacactttct ataacttcta ttgaagatat tggaatttcc aatttttcat gtgtactatg 5160

tcagaaaatg ctttcgattt tatttttaaa tctaacatcg gatggctttt ccggagtgtt 5220

gtaaaaactt caatcataca taaaacatgt tcttacaaaa ggcaaa 5266

<210> 6
<211> 5195
<212> DNA
<213> Homo sapiens

<400> 6
agagatcctg ggagcgagag ggagagagag ggagcaagaa aggaagagag agcgagcgag 60

agagagcgag cgaataagag agagagtaag agggagagag aagaagagga agaagaggag 120

gcggcggcag cggaggagga ggaggactag tgtggggtgg aaaggaagag tgagcgagag 180

caagttaagg ggagggggtg taagagccag cgaattcttt ttctttttct attattattt 240

tgacgactcc tgagttgcgc ccatgctctt gtcagcttcg ttttaggcgt agcatggcca 300

ggcagaagaa aatggggcaa agcgtgctcc gggcggtctt ctttttagtc ctggggcttt 360

tgggtcattc tcacggagga ttccccaaca ccatcagcat aggtggactt ttcatgagaa 420

acacagtgca ggagcacagc gctttccgct ttgccgtgca gttatacaac accaaccaga 480

acaccaccga gaagcccttc catttgaatt accacgtaga tcacttggat tcctccaata 540

gtttttccgt gacaaatgct ttctgctccc agttctcgag aggggtgtat gccatctttg 600

gattctatga ccagatgtca atgaacaccc tgacctcctt ctgtggggcc ctgcacacat 660

cctttgttac gcctagcttc cccactgacg cagatgtgca gtttgtcatc cagatgcgcc 720

cagccttgaa gggcgctatt ctgagtcttc tgggtcatta caagtgggag aagtttgtgt 780

acctctatga cacagaacga ggattttcca tcctccaagc gattatggaa gcagcagtgc 840

aaaacaactg gcaagtaaca gcaaggtctg tgggaaacat aaaggacgtc caagaattca 900

ggcgcatcat tgaagaaatg gacaggaggc aggaaaagcg atacttgatt gactgcgaag 960

tcgaaaggat taacacaatt ttggaacagg ttgtgatcct agggaaacac tcaagaggtt 1020

atcactacat gctcgctaac ctgggtttta ctgatatttt actggaaaga gtcatgcatg 1080

ggggagccaa cattacaggt ttccagattg tcaacaatga aaaccctatg gttcagcagt 1140

tcatacagcg ctgggtgagg ctggatgaaa gggaattccc tgaagccaag aatgcaccac 1200

taaagtatac atctgcattg acacacgacg caatactggt catagcagaa gctttccgct 1260

acctgaggag gcagcgagta gatgtgtccc ggagaggaag tgctggagac tgcttagcaa 1320

atcctgctgt gccctggagt caaggaattg atattgagag agctctgaaa atggtgcaag 1380

tacaaggaat gactggaaat attcaatttg acacttatgg acgtaggaca aattatacca 1440

tcgatgtgta tgaaatgaaa gtcagtggct ctcgaaaagc tggctactgg aatgagtatg 1500

aaaggtttgt gcctttctca gatcagcaaa tcagcaatga cagtgcatcc tcagagaatc 1560

ggaccatagt agtgactacc attctggaat caccatatgt aatgtacaag aagaaccatg 1620

agcaactgga aggaaatgaa cgatatgaag gctattgtgt agacctagcc tatgaaatag 1680

ccaaacatgt aaggatcaaa tacaaattgt ccatcgttgg tgacgggaaa tatggtgcaa 1740

gggatccaga gactaaaata tggaacggca tggttgggga acttgtctat gggagagctg 1800

atatagctgt tgctccactc actataacat tggtccgtga agaagtcata gatttttcaa 1860

agccattcat gagcctgggc atctccatca tgataaagaa gcctcagaaa tcaaaaccag 1920

gcgtattctc atttctggat cccctggctt atgaaatctg gatgtgcatt gtctttgctt 1980

acattggagt cagcgtagtt cttttcctag tcagcaggtt cagtccttat gaatggcact 2040

tggaagacaa caatgaagaa cctcgtgacc cacaaagtcc tcctgatcct ccaaatgaat 2100

ttggaatatt taacagtctt tggttttcct tgggtgcctt tatgcagcaa ggatgtgata 2160

tttctccaag atcactctcc gggcgcattg ttggaggggt ttggtggttc ttcaccctga 2220

tcataatttc ttcctatact gccaatctcg ctgctttcct gactgtggag aggatggttt 2280

ctcccataga gagtgctgaa gacttagcta aacagactga aattgcatat gggaccctgg 2340

actccggttc aacaaaagaa tttttcagaa gatccaaaat tgctgtgtac gagaaaatgt 2400

ggtcttacat gaaatcagcg gagccatctg tgtttaccaa aacaacagca gacggagtgg 2460

cccgagtgcg aaagtccaag ggaaagttcg ccttcctgct ggagtcaacc atgaatgagt 2520

acattgagca gagaaaacca tgtgatacga tgaaagttgg tggaaatctg gattccaaag 2580

gctatggtgt ggcaacccct aaaggctcag cattaggaaa tgctgttaac ctggcagtat 2640

taaaactgaa tgagcaaggc ctcttggaca aattgaaaaa caaatggtgg tacgacaaag 2700

gagagtgcgg cagcgggggc ggtgactcca aggacaagac cagcgctctg agcctgagca 2760

atgtggcagg cgttttctat atacttgtcg gaggtctggg gctggccatg atggtggctt 2820

tgatagaatt ctgttacaaa tcacgggcag agtccaaacg catgaaactc acaaagaaca 2880

cccaaaactt taagcctgct cctgccacca acactcagaa ttatgctaca tacagagaag 2940

gctacaacgt gtatggaaca gagagtgtta agatctaggg atcccttccc actggaggca 3000

tgtgatgaga ggaaatcacc gaaaacgtgg ctgcttcaag gatcctgagc cagatttcac 3060

tctccttggt gtcgggcatg acacgaatat tgctgatggt gcaatgacct ttcaatagga 3120

aaaactgatt tttttttcct tcagtgcctt atggaacact ctgagactcg cgacaatgca 3180

aaccatcatt gaaatctttt tgctttgctt gaaaaaaaat aattaaaata aaaaccaaca 3240

aaaatggaca tgcaagattc cagtatgcga aaaaaaatct tattaagtca attcaacaaa 3300

agccattctt tgataccact gcagagtata taaacaccat gttctttaat acacacacac 3360

acacacacac acacacacac acacatttaa attccaattc agcaaagagg cccatctaag 3420

ctaaaaaaat taattcttcc tgattaaaaa gaaaaaatct gtctcccagt gtttgggaag 3480

acggactggc atttcttcta ggatctgctg accagatgtt tttggtattt cctgttggtg 3540

gtgatgttct gtgcactcta tttcctttca atgttgctga aatgtgtata tctttagaat 3600

gtaaatgcaa cacttaagaa aattcaaaca ctttggaaaa gggactaaac agtgatttct 3660

ctgtgttctt gaaatggttt tgtgaaaatg ctttgataac ttcccactca aagaagagat 3720

ttacagagct ttcgaaattg actttgtgtg tagcaaggga cggggcacta tcaggatacc 3780

tcttggtgct ttcctaaaat ggatcccggg gctttccaag gagcctggaa tttcagctca 3840

cagatctgtt tttcttgctt cagtgtgcat tttaagtcaa tagagctgag tatctagcat 3900

tgaggtgagg gaaatgctgc ctatactccc agatgtgttt agaatatctc agaaacaaca 3960

ctgtgtttag ctcggctttc tctgctaagt atgcctttca agtgtacacc acggagacag 4020

gaccgcgttg caaggcggga cagcaggttc agaccacagt tctcagtctg actttactct 4080

tgctaggtct gtcctactag ctgttgcctg ctaccgccca tggctctcca tcggactgca 4140

tgtgtccttt tctagtttgc aaagactaaa atgcattccc aaacctactg ctaatctgag 4200

ggcctcagca tcacttccag atccttgctt ggagcagtct ctctattgac tctctcagat 4260

cgctccactg ctccatgggc tatcaagtaa ctaactgcat acctgccgtt ggcatcatca 4320

gaacagtccg aagaaatagt ctccactcac taattacctc ctatataacg acgtatgctt 4380

cctgtagttc agtagtttgc tctcatcgat aacgtgcatt gggaagtttc cagactgcaa 4440

aaactaggag ctcgcattca tttcccaagt gtgaccctta gatgcttagt tgactcgctg 4500

catatttgct cttgtcttca gaaaagaaag gaagaagtat cgttccaacg aaatgtttcc 4560

agaaaagtgt actataaact ttcattccaa aaatggtgtc ataagcaaac aactcacttg 4620

tcaaatttca aatggtattg aacaaaaaaa gaaagctgtt gtgtttttgt tttgttttgt 4680

tttcatgaaa ctgtgatttt caacttatga atgctataat gtcccagcgc gggaagctca 4740

cgctgtgtga acatgaagtt gtataaaaca aaccaaccaa cctacacaca aatgttttca 4800

taggcactgt ataaagaaaa atgtatgttt attaactcaa atcagttttt cagagaggaa 4860

acgtcactga gatgaagagg cgggtaaatt ggtttgttat tttttaaaaa aaacttgcat 4920

gtttaaaaaa aagttgattg cttcaaattt ctgctactaa cttcaagcta tgggagtttg 4980

gcagtagtca cttgaggatt ttttttccaa ttcttttctt tttgttgtta aagctgtact 5040

tcagtgaaca gaaaaattgc caagcaaact aatggctata aaagcgtaat ttgcatgtgt 5100

gggcataaac tacagagcct cattgccatg aggtattgta caaagtttta atacattttg 5160

taaataaaat tgtaaagaaa gaaaaaaaaa aaaaa 5195

<210> 7
<211> 5195
<212> DNA
<213> Homo sapiens

<400> 7
agagatcctg ggagcgagag ggagagagag ggagcaagaa aggaagagag agcgagcgag 60

agagagcgag cgaataagag agagagtaag agggagagag aagaagagga agaagaggag 120

gcggcggcag cggaggagga ggaggactag tgtggggtgg aaaggaagag tgagcgagag 180

caagttaagg ggagggggtg taagagccag cgaattcttt ttctttttct attattattt 240

tgacgactcc tgagttgcgc ccatgctctt gtcagcttcg ttttaggcgt agcatggcca 300

ggcagaagaa aatggggcaa agcgtgctcc gggcggtctt ctttttagtc ctggggcttt 360

tgggtcattc tcacggagga ttccccaaca ccatcagcat aggtggactt ttcatgagaa 420

acacagtgca ggagcacagc gctttccgct ttgccgtgca gttatacaac accaaccaga 480

acaccaccga gaagcccttc catttgaatt accacgtaga tcacttggat tcctccaata 540

gtttttccgt gacaaatgct ttctgctccc agttctcgag aggggtgtat gccatctttg 600

gattctatga ccagatgtca atgaacaccc tgacctcctt ctgtggggcc ctgcacacat 660

cctttgttac gcctagcttc cccactgacg cagatgtgca gtttgtcatc cagatgcgcc 720

cagccttgaa gggcgctatt ctgagtcttc tgggtcatta caagtgggag aagtttgtgt 780

acctctatga cacagaacga ggattttcca tcctccaagc gattatggaa gcagcagtgc 840

aaaacaactg gcaagtaaca gcaaggtctg tgggaaacat aaaggacgtc caagaattca 900

ggcgcatcat tgaagaaatg gacaggaggc aggaaaagcg atacttgatt gactgcgaag 960

tcgaaaggat taacacaatt ttggaacagg ttgtgatcct agggaaacac tcaagaggtt 1020

atcactacat gctcgctaac ctgggtttta ctgatatttt actggaaaga gtcatgcatg 1080

ggggagccaa cattacaggt ttccagattg tcaacaatga aaaccctatg gttcagcagt 1140

tcatacagcg ctgggtgagg ctggatgaaa gggaattccc tgaagccaag aatgcaccac 1200

taaagtatac atctgcattg acacacgacg caatactggt catagcagaa gctttccgct 1260

acctgaggag gcagcgagta gatgtgtccc ggagaggaag tgctggagac tgcttagcaa 1320

atcctgctgt gccctggagt caaggaattg atattgagag agctctgaaa atggtgcaag 1380

tacaaggaat gactggaaat attcaatttg acacttatgg acgtaggaca aattatacca 1440

tcgatgtgta tgaaatgaaa gtcagtggct ctcgaaaagc tggctactgg aatgagtatg 1500

aaaggtttgt gcctttctca gatcagcaaa tcagcaatga cagtgcatcc tcagagaatc 1560

ggaccatagt agtgactacc attctggaat caccatatgt aatgtacaag aagaaccatg 1620

agcaactgga aggaaatgaa cgatatgaag gctattgtgt agacctagcc tatgaaatag 1680

ccaaacatgt aaggatcaaa tacaaattgt ccatcgttgg tgacgggaaa tatggtgcaa 1740

gggatccaga gactaaaata tggaacggca tggttgggga acttgtctat gggagagctg 1800

atatagctgt tgctccactc actataacat tggtccgtga agaagtcata gatttttcaa 1860

agccattcat gagcctgggc atctccatca tgataaagaa gcctcagaaa tcaaaaccag 1920
gcgtattctc atttctggat cccctggctt atgaaatctg gatgtgcatt gtctttgctt 1980
acattggagt cagcgtagtt cttttcctag tcagcaggtt cagtccttat gaatggcact 2040
tggaagacaa caatgaagaa cctcgtgacc cacaaagtcc tcctgatcct ccaaatgaat 2100
ttggaatatt taacagtctt tggttttcct tgggtgcctt tatgcagcaa ggatgtgata 2160
tttctccaag atcactctcc gggcgcattg ttggaggggt ttggtggttc ttcaccctga 2220
tcataatttc ttcctatact gccaatctcg ctgctttcct gactgtggag aggatggttt 2280
ctcccataga gagtgctgaa gacttagcta aacagactga aattgcatat gggaccctgg 2340
actccggttc aacaaaagaa tttttcagaa gatccaaaat tgctgtgtac gagaaaatgt 2400
ggtcttacat gaaatcagcg gagccatctg tgtttaccaa aacaacagca gacggagtgg 2460
cccgagtgcg aaagtccaag ggaaagttcg ccttcctgct ggagtcaacc atgaatgagt 2520
acattgagca gagaaaacca tgtgatacga tgaaagttgg tggaaatctg gattccaaag 2580
gctatggtgt ggcaacccct aaaggctcag cattaggaac gcctgtaaac cttgcagtat 2640
tgaaactcag tgaacaaggc atcttagaca agctgaaaaa caaatggtgg tacgataagg 2700
gggaatgtgg agccaaggac tccgggagta aggacaagac cagcgctctg agcctgagca 2760
atgtggcagg cgttttctat atacttgtcg gaggtctggg gctggccatg atggtggctt 2820
tgatagaatt ctgttacaaa tcacgggcag agtccaaacg catgaaactc acaaagaaca 2880
cccaaaactt taagcctgct cctgccacca acactcagaa ttatgctaca tacagagaag 2940
gctacaacgt gtatggaaca gagagtgtta agatctaggg atcccttccc actggaggca 3000
tgtgatgaga ggaaatcacc gaaaacgtgg ctgcttcaag gatcctgagc cagatttcac 3060
tctccttggt gtcgggcatg acacgaatat tgctgatggt gcaatgacct ttcaatagga 3120
aaaactgatt tttttttcct tcagtgcctt atggaacact ctgagactcg cgacaatgca 3180
aaccatcatt gaaatctttt tgctttgctt gaaaaaaaat aattaaaata aaaaccaaca 3240
aaaatggaca tgcaagattc cagtatgcga aaaaaaatct tattaagtca attcaacaaa 3300
agccattctt tgataccact gcagagtata taaacaccat gttctttaat acacacacac 3360
acacacacac acacacacac acacatttaa attccaattc agcaaagagg cccatctaag 3420
ctaaaaaaat taattcttcc tgattaaaaa gaaaaaatct gtctcccagt gtttgggaag 3480
acggactggc atttcttcta ggatctgctg accagatgtt tttggtattt cctgttggtg 3540
gtgatgttct gtgcactcta tttcctttca atgttgctga aatgtgtata tctttagaat 3600

gtaaatgcaa cacttaagaa aattcaaaca ctttggaaaa gggactaaac agtgatttct 3660

ctgtgttctt gaaatggttt tgtgaaaatg ctttgataac ttcccactca aagaagagat 3720

ttacagagct ttcgaaattg actttgtgtg tagcaaggga cggggcacta tcaggatacc 3780

tcttggtgct ttcctaaaat ggatcccggg gctttccaag gagcctggaa tttcagctca 3840

cagatctgtt tttcttgctt cagtgtgcat tttaagtcaa tagagctgag tatctagcat 3900

tgaggtgagg gaaatgctgc ctatactccc agatgtgttt agaatatctc agaaacaaca 3960

ctgtgtttag ctcggctttc tctgctaagt atgcctttca agtgtacacc acggagacag 4020

gaccgcgttg caaggcggga cagcaggttc agaccacagt tctcagtctg actttactct 4080

tgctaggtct gtcctactag ctgttgcctg ctaccgccca tggctctcca tcggactgca 4140

tgtgtccttt tctagtttgc aaagactaaa atgcattccc aaacctactg ctaatctgag 4200

ggcctcagca tcacttccag atccttgctt ggagcagtct ctctattgac tctctcagat 4260

cgctccactg ctccatgggc tatcaagtaa ctaactgcat acctgccgtt ggcatcatca 4320

gaacagtccg aagaaatagt ctccactcac taattacctc ctatataacg acgtatgctt 4380

cctgtagttc agtagtttgc tctcatcgat aacgtgcatt gggaagtttc cagactgcaa 4440

aaactaggag ctcgcattca tttcccaagt gtgaccctta gatgcttagt tgactcgctg 4500

catatttgct cttgtcttca gaaaagaaag gaagaagtat cgttccaacg aaatgtttcc 4560

agaaaagtgt actataaact ttcattccaa aaatggtgtc ataagcaaac aactcacttg 4620

tcaaatttca aatggtattg aacaaaaaaa gaaagctgtt gtgtttttgt tttgttttgt 4680

tttcatgaaa ctgtgatttt caacttatga atgctataat gtcccagcgc gggaagctca 4740

cgctgtgtga acatgaagtt gtataaaaca aaccaaccaa cctacacaca aatgttttca 4800

taggcactgt ataaagaaaa atgtatgttt attaactcaa atcagttttt cagagaggaa 4860

acgtcactga gatgaagagg cgggtaaatt ggtttgttat tttttaaaaa aaacttgcat 4920

gtttaaaaaa aagttgattg cttcaaattt ctgctactaa cttcaagcta tgggagtttg 4980

gcagtagtca cttgaggatt ttttttccaa ttcttttctt tttgttgtta aagctgtact 5040

tcagtgaaca gaaaaattgc caagcaaact aatggctata aaagcgtaat ttgcatgtgt 5100

gggcataaac tacagagcct cattgccatg aggtattgta caaagtttta atacattttg 5160

taaataaaat tgtaaagaaa gaaaaaaaaa aaaaa 5195

<210> 8
<211> 5508
<212> DNA
<213> Homo sapiens

<400>  8
agtggcagaa gagggctagg ctgagaggga agccaggact gtaggagagg gaggcagccc      60

gtcctcctca cgaacctgca aggatgcggc aggggcctgg gggcatgggg aggtactaac     120

cccccggagc cccgattggg ggcttgcaga cctggcccgt gggcggattt tctgcctagc     180

gcagccgaga agcagaggtg ccaggaaaac caagagaggg gcgctggggg tgcccatccc     240

cagagtcggt ccctctgcga accgaggaag aaaagaggag ggagtcagcg agtggtcaga     300

agggaaaacc tgacaccaga ctggctccgg agcgtccggg agactggggc gctccgcgcc     360

atcgtcttca atgcttctct gaacagcctt taggaagagt gcgagagaaa gagagagagc     420

gcgcgccagg gagaggagaa aagaagatga ggattatttc cagacagatt gtcttgttat     480

tttctggatt ttggggactc gccatgggag cctttccgag cagcgtgcaa ataggtggtc     540

tcttcatccg aaacacagat caggaataca ctgcttttcg attagcaatt tttcttcata     600

acaccagccc caatgcgtcg gaagctcctt ttaatttggt acctcatgtg gacaacattg     660

agacagccaa cagttttgct gtaacaaacg ccttctgttc ccagtattct agaggagtat     720

ttgccatttt tggactctat gataagaggt cggtacatac cttgacctca ttctgcagcg     780

ccttacatat ctccctcatc acaccaagtt tccctactga gggggagagc cagtttgtgc     840

tgcaactaag accttcgtta cgaggagcac tcttgagttt gctggatcac tacgaatgga     900

actgttttgt cttcctgtat gacacagaca ggggatactc gatactccaa gctattatgg     960

aaaaagcagg acaaaatggt tggcatgtca gcgctatatg tgtggaaaat tttaatgatg    1020

tcagctatag gcaacttcta gaagaacttg acagaagaca agagaagaag tttgtaatag    1080

actgtgagat agagagactt caaaacatat tagaacagat tgtaagtgtt ggaaagcatg    1140

ttaaaggcta ccattatatc attgcaaact tgggattcaa ggatatttct cttgagaggt    1200

ttatacatgg tggagccaat gttactggat tccagttggt ggattttaat acacctatgg    1260

taatcaaact aatggatcgc tggaagaaac tagatcagag agagtatcca ggatctgaga    1320

ctcctccaaa gtacacctct gctctgactt atgatggagt ccttgtgatg gctgaaactt    1380

tccgaagtct taggaggcag aaaattgata tctcaaggag aggaaatgct ggggattgtc    1440

tggcaaatcc tgctgctcca tggggccagg gaattgacat ggagaggaca ctcaaacagg    1500

ttcgaattca agggctgaca gggaatgttc agtttgacca ctatggacgt agagtcaatt    1560

acacaatgga tgtgtttgag ctgaaaagca caggacctag aaaggttggt tactggaatg    1620

atatggataa gttagtcttg attcaagatg taccaactct tggcaatgac acagctgcta    1680

ttgagaacag aacagtggtt gtaaccacaa ttatggaatc cccatatgtt atgtacaaga    1740

aaaatcatga aatgtttgaa ggaaatgaca agtatgaagg atactgtgta gatttggcat 1800

ctgaaattgc aaaacatatt ggtatcaagt ataaaattgc cattgtccct gatggaaaat 1860

atggagcaag ggatgcagac acaaaaatct ggaatgggat ggtaggagaa cttgtttatg 1920

ggaaagcaga gattgctatt gcccctctga caatcacttt ggtacgagag gaggtcattg 1980

acttttctaa gcccttcatg agtttgggca tatctatcat gatcaaaaag cctcagaaat 2040

ccaaaccagg agtgttttcc ttcttggatc ctctggccta tgagatttgg atgtgcatag 2100

tctttgccta cattggtgtc agcgtggtct tattcctagt tagtagattt agtccatatg 2160

agtggcacac agaagagcca gaggacggaa aggaaggacc cagcgaccag cctcccaatg 2220

agtttggcat ctttaacagc ctctggtttt ccctgggtgc ttttatgcag caaggatgtg 2280

acatttcacc cagatccctc tcaggtcgaa ttgttggagg tgtttggtgg ttctttacac 2340

tcatcattat atcatcttat actgctaacc tcgctgcttt cctgacggtt gagcgaatgg 2400

tctctcccat agaaagtgca gaagacctgg ccaaacaaac agaaattgcc tatggaacac 2460

tggattcagg atcaacaaaa gaattcttca gaagatcaaa aatagcagtg tatgaaaaga 2520

tgtggaccta catgcgatca gcagagccat cagtattcac taggactaca gctgagggag 2580

tagctcgtgt ccgcaaatcc aagggcaaat ttgcctttct cctggagtcc actatgaatg 2640

aatacattga gcagcgaaag ccatgtgaca cgatgaaagt gggaggaaat ctggattcca 2700

aaggctatgg agtagcaacg cccaagggtt cctcattagg aactcctgta aaccttgccg 2760

ttttgaaact cagtgaggca ggcgtcttag acaagctgaa aaacaaatgg tggtacgata 2820

aaggtgaatg tggacccaag gactctggaa gcaaggacaa gacgagtgcc ttgagcctga 2880

gcaatgtagc aggcgtcttc tacattctgg ttggcggctt gggcttggca atgctggtgg 2940

ctttgataga gttctgttac aagtccaggg cagaagcgaa gagaatgaag ctgacctttt 3000

ctgaagccat aagaaacaaa gccagattat ccatcactgg gagtgtggga gagaatggcc 3060

gcgtcttgac gcctgactgc ccaaaggctg tacacactgg aactgcaatc agacaaagtt 3120

caggattggc tgtcattgca tcggacctac cataaaaacc aaaaaaataa ttgagtgcct 3180

taattaaact gttggtgact ggtggaaacg cagccctgag ggacacgcca cgcgcgggtc 3240

tttgctaaac caatcctttg gctgagagcg ggaagtccgt cctaacgcgc tggccggaca 3300

tcagcagcag caacgtgtgc atgagctcag ctcggaaacc caaactcaga ttttatatca 3360

ggaaaactca caattgaggt ttttttcggg gagtgggtgg gggagggatc tgggatgggt 3420

gtattaacag caacaaattt cattcgagtg gactcaaaaa ctaatcagac ttatgagtta 3480

gcgcattaaa ctgtgaagtt cttgctcaga aaggcctttg tcttcaccgg aaaggataaa 3540

atagttgtag aagtccgtga acatgctaac ctgtgtctcc agaacatcca tatagtccat 3600

ggaagaaaat ccagctgaga aaacaaatca ctaaactgtg ataagaaaat aatgaacaaa 3660

catgtaaaac ctgtgggaaa aaaaaataaa ggaagtatgt acacttactt tggagaaaac 3720

aaatactgaa acatgcttgc tttttaactg acgtaaattc agtagaggac aacacaattc 3780

ttttttctaa ccatcttagg gaacaataca ttgcaataat tgatataaat gccatcactg 3840

taataaactt tagagacttt tttttataaa agttgttggt catcttcttg tttgctgtaa 3900

ccttcactat gtcacatgag tcgattcacc gattgcattt gtctcacaac caggaagaaa 3960

agcaaaagga agaaaacgtt taggttcaat catcagtctg cggtgtagac tcgaaagaga 4020

tgacaggtca ctcatgttaa tggtattatt tataatctca ttctgtgtac aacattgtgg 4080

tttttgtacc caccaaaaag aataaaacag cagatgttct tacaatatct acagagctta 4140

aaagtttttt cttatcgtta taaaagttat ttgagaaatt ataagactat aagagagatt 4200

gtattagtgg tgggccatag tggaaaatgt agctagccct cattattttt tgcatactaa 4260

gctacccctc cttttcagat ctttgactca ttaacagatt aaactgtcaa agatggagtc 4320

tttgagttgg ggaatgaatc actgtcctaa caacaacata ccttgtaatt gtgtgttgaa 4380

attttacttg actgtatttt gctgcataaa attatgtgtc tcttgggctt cttcccttat 4440

tcctattgtt ccctttaaat catatgaagg cattcataat agcttggggt agataacaaa 4500

tgaagaatta gtctttgttt tcaactggaa attgtaaaga aaattatact catgtttatt 4560

tataaaaatc accttatgta tgaattaaac taacatggtt caaaagaagg tttggttcat 4620

ttgaaataat aaataagtac tctaatacag ataaaaatca tgtacttagg gtattggcag 4680

aaagcacaag ttaggatgat ttcagaagtc tggccttgaa ggatgagttg agttttaaca 4740

ggaggagaag gtgttaagag ccatatgagt gagcagtggc ccaaagccat gcacatcagt 4800

ggctcattta aggaatgaat gccattagat gggctactga gagtacaggg atattatgga 4860

agataaagtt ggaaaagctg aaggattgat tttcttccat caactctcaa gatcccattc 4920

gccattcaat ctctgtgctg cagtaagagc aatcttaaac agtataaatc acacacacac 4980

acacacacac acacacacac acacacaagt ccctcaggaa aaattccaag ctcttgagaa 5040

gatcacatga gccccttcat gacctggcgc ttgcttattt cttccaggac ttctctcact 5100

tctatccagc tattcccgtc agcaaatgaa cctccaaagc agcacatgga gcactgcata 5160

gactatttcc tcagtgcgta actcctccct gtctcctctt tacctgagta acttgtactc 5220

atccttcaat actccaactg aattttactt accctgaaaa gatttccatg gctatccacc 5280

acccccctgc ctgtgagact gagttaggtg ccctttttca tgtctttccc ccatcacggc 5340

acttaccata ctgcgttgta attgcctgtg tactcgtctg tataactact agactgtaag 5400

ctccttgagg gcagggactg tgtctatctt gttcacagtt gtatccccag cacccagcac 5460

agtgcctggc atattgtagg tgcttaataa atatttgttg aatgaatg 5508

<210> 9
<211> 5621
<212> DNA
<213> Homo sapiens

<400> 9
agtggcagaa gagggctagg ctgagaggga agccaggact gtaggagagg gaggcagccc 60

gtcctcctca cgaacctgca aggatgcggc aggggcctgg gggcatgggg aggtactaac 120

cccccggagc ccccgattgg ggcttgcaga cctggcccgt gggcggattt tctgcctagc 180

gcagccgaga agcagaggtg ccaggaaaac caagagaggg gcgctggggg tgcccatccc 240

cagagtcggt ccctctgcga accgaggaag aaaagaggag ggagtcagcg agtggtcaga 300

agggaaaacc tgacaccaga ctggctccgg agcgtccggg agactggggc gctccgcgcc 360

atcgtcttca atgcttctct gaacagcctt taggaagagt gcgagagaaa gagagagagc 420

gcgcgccagg gagaggagaa aagaagatga ggattatttc cagacagatt gtcttgttat 480

tttctggatt ttggggactc gccatgggag cctttccgag cagcgtgcaa ataggtggtc 540

tcttcatccg aaacacagat caggaataca ctgcttttcg attagcaatt tttcttcata 600

acaccagccc caatgcgtcg gaagctcctt ttaatttggt acctcatgtg gacaacattg 660

agacagccaa cagttttgct gtaacaaacg ccttctgttc ccagtattct agaggagtat 720

ttgccatttt tggactctat gataagaggt cggtacatac cttgacctca ttctgcagcg 780

ccttacatat ctccctcatc acaccaagtt tccctactga gggggagagc cagtttgtgc 840

tgcaactaag accttcgtta cgaggagcac tcttgagttt gctggatcac tacgaatgga 900

actgttttgt cttcctgtat gacacagaca ggggatactc gatactccaa gctattatgg 960

aaaaagcagg acaaaatggt tggcatgtca gcgctatatg tgtggaaaat tttaatgatg 1020

tcagctatag gcaacttcta gaagaacttg acagaagaca agagaagaag tttgtaatag 1080

actgtgagat agagagactt caaaacatat tagaacagat tgtaagtgtt ggaaagcatg 1140

ttaaaggcta ccattatatc attgcaaact tgggattcaa ggatatttct cttgagaggt 1200

ttatacatgg tggagccaat gttactggat tccagttggt ggattttaat acacctatgg 1260

taatcaaact aatggatcgc tggaagaaac tagatcagag agagtatcca ggatctgaga 1320

ctcctccaaa gtacacctct gctctgactt atgatggagt ccttgtgatg gctgaaactt 1380

tccgaagtct taggaggcag aaaattgata tctcaaggag aggaaatgct ggggattgtc 1440

tggcaaatcc tgctgctcca tggggccagg gaattgacat ggagaggaca ctcaaacagg 1500

ttcgaattca agggctgaca gggaatgttc agtttgacca ctatggacgt agagtcaatt 1560

acacaatgga tgtgtttgag ctgaaaagca caggacctag aaaggttggt tactggaatg 1620

atatggataa gttagtcttg attcaagatg taccaactct tggcaatgac acagctgcta 1680

ttgagaacag aacagtggtt gtaaccacaa ttatggaatc cccatatgtt atgtacaaga 1740

aaaatcatga aatgtttgaa ggaaatgaca agtatgaagg atactgtgta gatttggcat 1800

ctgaaattgc aaaacatatt ggtatcaagt ataaaattgc cattgtccct gatggaaaat 1860

atggagcaag ggatgcagac acaaaaatct ggaatgggat ggtaggagaa cttgtttatg 1920

ggaaagcaga gattgctatt gcccctctga caatcacttt ggtacgagag gaggtcattg 1980

acttttctaa gcccttcatg agtttgggca tatctatcat gatcaaaaag cctcagaaat 2040

ccaaaccagg agtgttttcc ttcttggatc ctctggccta tgagatttgg atgtgcatag 2100

tctttgccta cattggtgtc agcgtggtct tattcctagt tagtagattt agtccatatg 2160

agtggcacac agaagagcca gaggacggaa aggaaggacc cagcgaccag cctcccaatg 2220

agtttggcat ctttaacagc ctctggtttt ccctgggtgc ttttatgcag caaggatgtg 2280

acatttcacc cagatccctc tcaggtcgaa ttgttggagg tgtttggtgg ttctttacac 2340

tcatcattat atcatcttat actgctaacc tcgctgcttt cctgacggtt gagcgaatgg 2400

tctctcccat agaaagtgca gaagacctgg ccaaacaaac agaaattgcc tatggaacac 2460

tggattcagg atcaacaaaa gaattcttca gaagatcaaa aatagcagtg tatgaaaaga 2520

tgtggaccta catgcgatca gcagagccat cagtattcac taggactaca gctgagggag 2580

tagctcgtgt ccgcaaatcc aagggcaaat ttgcctttct cctggagtcc actatgaatg 2640

aatacattga gcagcgaaag ccatgtgaca cgatgaaagt gggaggaaat ctggattcca 2700

aaggctatgg agtagcaacg cccaagggtt cctcattagg aaatgctgtt aacctcgcag 2760

ttttaaaact gaatgaacaa ggcctcttgg acaaattgaa aaacaaatgg tggtacgaca 2820

aaggagaatg tggcagcggg ggaggtgact ccaaggacaa gacgagtgcc ttgagcctga 2880

gcaatgtagc aggcgtcttc tacattctgg ttggcggctt gggcttggca atgctggtgg 2940

ctttgataga gttctgttac aagtccaggg cagaagcgaa gagaatgaag gtggcaaaga 3000

gtgcacagac ttttaaccca acttcctcgc agaataccca gaatttagca acctatagag 3060

aaggttacaa cgtatatgga accgaaagta ttaaaattta gggctgacct tttctgaagc 3120

cataagaaac aaagccagat tatccatcac tgggagtgtg ggagagaatg gccgcgtctt 3180

gacgcctgac tgcccaaagg ctgtacacac tggaactgca atcagacaaa gttcaggatt 3240

ggctgtcatt gcatcggacc taccataaaa accaaaaaaa taattgagtg ccttaattaa 3300

actgttggtg actggtggaa acgcagccct gagggacacg ccacgcgcgg gtctttgcta 3360

aaccaatcct ttggctgaga gcgggaagtc cgtcctaacg cgctggccgg acatcagcag 3420

cagcaacgtg tgcatgagct cagctcggaa acccaaactc agattttata tcaggaaaac 3480

tcacaattga ggtttttttc ggggagtggg tgggggaggg atctgggatg ggtgtattaa 3540

cagcaacaaa tttcattcga gtggactcaa aaactaatca gacttatgag ttagcgcatt 3600

aaactgtgaa gttcttgctc agaaaggcct ttgtcttcac cggaaaggat aaaatagttg 3660

tagaagtccg tgaacatgct aacctgtgtc tccagaacat ccatatagtc catggaagaa 3720

aatccagctg agaaaacaaa tcactaaact gtgataagaa aataatgaac aaacatgtaa 3780

aacctgtggg aaaaaaaaat aaaggaagta tgtacactta ctttggagaa aacaaatact 3840

gaaacatgct tgctttttaa ctgacgtaaa ttcagtagag gacaacacaa ttcttttttc 3900

taaccatctt agggaacaat acattgcaat aattgatata aatgccatca ctgtaataaa 3960

ctttagagac ttttttttat aaaagttgtt ggtcatcttc ttgtttgctg taaccttcac 4020

tatgtcacat gagtcgattc accgattgca tttgtctcac aaccaggaag aaaagcaaaa 4080

ggaagaaaac gtttaggttc aatcatcagt ctgcggtgta gactcgaaag agatgacagg 4140

tcactcatgt taatggtatt atttataatc tcattctgtg tacaacattg tggtttttgt 4200

acccaccaaa aagaataaaa cagcagatgt tcttacaata tctacagagc ttaaaagttt 4260

tttcttatcg ttataaaagt tatttgagaa attataagac tataagagag attgtattag 4320

tggtgggcca tagtggaaaa tgtagctagc cctcattatt ttttgcatac taagctaccc 4380

ctccttttca gatctttgac tcattaacag attaaactgt caaagatgga gtctttgagt 4440

tggggaatga atcactgtcc taacaacaac ataccttgta attgtgtgtt gaaattttac 4500

ttgactgtat tttgctgcat aaaattatgt gtctcttggg cttcttccct tattcctatt 4560

gttcccttta aatcatatga aggcattcat aatagcttgg ggtagataac aaatgaagaa 4620

ttagtctttg ttttcaactg gaaattgtaa agaaaattat actcatgttt atttataaaa 4680

atcaccttat gtatgaatta aactaacatg gttcaaaaga aggtttggtt catttgaaat 4740

aataaataag tactctaata cagataaaaa tcatgtactt agggtattgg cagaaagcac 4800

aagttaggat gatttcagaa gtctggcctt gaaggatgag ttgagtttta acaggaggag 4860

aaggtgttaa gagccatatg agtgagcagt ggcccaaagc catgcacatc agtggctcat 4920

ttaaggaatg aatgccatta gatgggctac tgagagtaca gggatattat ggaagataaa 4980

gttggaaaag ctgaaggatt gattttcttc catcaactct caagatccca ttcgccattc 5040

aatctctgtg ctgcagtaag agcaatctta aacagtataa atcacacaca cacacacaca 5100

cacacacaca cacacacaca agtccctcag gaaaaattcc aagctcttga gaagatcaca 5160

tgagcccctt catgacctgg cgcttgctta tttcttccag gacttctctc acttctatcc 5220

agctattccc gtcagcaaat gaacctccaa agcagcacat ggagcactgc atagactatt 5280

tcctcagtgc gtaactcctc cctgtctcct ctttacctga gtaacttgta ctcatccttc 5340

aatactccaa ctgaatttta cttaccctga aaagatttcc atggctatcc accacccccc 5400

tgcctgtgag actgagttag gtgccctttt tcatgtcttt cccccatcac ggcacttacc 5460

atactgcgtt gtaattgcct gtgtactcgt ctgtataact actagactgt aagctccttg 5520

agggcaggga ctgtgtctat cttgttcaca gttgtatccc cagcacccag cacagtgcct 5580

ggcatattgt aggtgcttaa taaatatttg ttgaatgaat g 5621

<210> 10
<211> 3345
<212> DNA
<213> Homo sapiens

<400> 10
agtggcagaa gagggctagg ctgagaggga agccaggact gtaggagagg gaggcagccc 60

gtcctcctca cgaacctgca aggatgcggc aggggcctgg gggcatgggg aggtactaac 120

cccccggagc cccgattggg ggcttgcaga cctggcccgt gggcggattt tctgcctagc 180

gcagccgaga agcagaggtg ccaggaaaac caagagaggg gcgctggggg tgcccatccc 240

cagagtcggt ccctctgcga accgaggaag aaaagaggag ggagtcagcg agtggtcaga 300

agggaaaacc tgacaccaga ctggctccgg agcgtccggg agactggggc gctccgcgcc 360

atcgtcttca atgcttctct gaacagcctt taggaagagt gcgagagaaa gagagagagc 420

gcgcgccagg gagaggagaa aagaagatga ggattatttc cagacagatt gtcttgttat 480

tttctggatt ttggggactc gccatgggag cctttccgag cagcgtgcaa ataggtggtc 540

tcttcatccg aaacacagat caggaataca ctgcttttcg attagcaatt tttcttcata 600

acaccagccc caatgcgtcg gaagctcctt ttaatttggt acctcatgtg gacaacattg 660

agacagccaa cagttttgct gtaacaaacg ccttctgttc ccagtattct agaggagtat 720

ttgccatttt tggactctat gataagaggt cggtacatac cttgacctca ttctgcagcg 780

ccttacatat ctccctcatc acaccaagtt tccctactga gggggagagc cagtttgtgc 840
tgcaactaag accttcgtta cgaggagcac tcttgagttt gctggatcac tacgaatgga 900
actgttttgt cttcctgtat gacacagaca ggggatactc gatactccaa gctattatgg 960
aaaaagcagg acaaaatggt tggcatgtca gcgctatatg tgtggaaaat tttaatgatg 1020
tcagctatag gcaacttcta gaagaacttg acagaagaca agagaagaag tttgtaatag 1080
actgtgagat agagagactt caaaacatat tagaacagat tgtaagtgtt ggaaagcatg 1140
ttaaaggcta ccattatatc attgcaaact tgggattcaa ggatatttct cttgagaggt 1200
ttatacatgg tggagccaat gttactggat tccagttggt ggattttaat acacctatgg 1260
taatcaaact aatggatcgc tggaagaaac tagatcagag agagtatcca ggatctgaga 1320
ctcctccaaa gtacacctct gctctgactt atgatggagt ccttgtgatg gctgaaactt 1380
tccgaagtct taggaggcag aaaattgata tctcaaggag aggaaatgct ggggattgtc 1440
tggcaaatcc tgctgctcca tggggccagg gaattgacat ggagaggaca ctcaaacagg 1500
ttcgaattca agggctgaca gggaatgttc agtttgacca ctatggacgt agagtcaatt 1560
acacaatgga tgtgtttgag ctgaaaagca caggacctag aaaggttggt tactggaatg 1620
atatggataa gttagtcttg attcaagatg taccaactct tggcaatgac acagctgcta 1680
ttgagaacag aacagtggtt gtaaccacaa ttatgcctct gatgaagaat cctattttaa 1740
gaaattgatc aagaaagaaa agagttccgc gctgttcgac cattcctaac taaggctcaa 1800
gtcttgttct ccagtgtagt aaatttaagc ttatttttca tgtgggattc ttcttggatg 1860
accaactctg gactaccaga aaaaaaaaat tttaagttct gtgacttttc tgagatacta 1920
gaacaaaaga agaattaatc ttcatctttc tcaagaaata gatgttgaca aagaatcact 1980
tagcgattct gacatatcaa ttcccctatc ttgaaatgag gtcactgtat gtaaatgatg 2040
gaattatatc actccatttc caagggtaga ttttctataa gtaaatatct cggaatttgt 2100
gtgcttgttt tctgaatata tacagttgtt ttctttaaag atctcttgga attttgcctg 2160
ttctgtgtga aataaagtgt tttaatgtgc attataggta tgatatagag aatctccttt 2220
ccatccttgt tactaaaggg actggacaaa taaatcttaa aaccaaaata ctgaattaat 2280
tttgcaagca tggctagttt ttaggaagca tgctatcaaa aaaaaaaaga ctaaaaatga 2340
ctgaaaaaat ccaactgttt tatatatata taaatatata tatatttata tatatatata 2400
aaggatattc tgtaaagtta tatgttgttt gacagtaaag ccatcaatat ttttgctatc 2460
aaaatagtat aatactagta tctttttgta tgaaaatgta atctttatat aaataatacc 2520

tctgatattt gcaactgcat aatcgttcag taattcaaaa agacatacta gaatcctttt 2580

tctgaaagtg ttccttcaat ttgcttttgt tgaaaacggt agtccaggac ctatgatatc 2640

cctccacttc attcattatg aaagaaatcc cttgtagata aacaagatat tggcatctgc 2700

atgtaattat ccccagattc agctgaaaac tcccaacaca gatggaattg gctagacatt 2760

ttaatatatg tgatacctat atctagatat agaaggctga gagtgagcac tggatataat 2820

tcattttgat tgaaattgat atggtgttat tgttcttcca gttgtctgtc ctttgtgtat 2880

gttcttattt atatgttgat acactgtaac actatatgct attgctaaat aaaattgatt 2940

gagaaattca gttattcata aatatttatt gagcgtctgc tatgtgctag gcacagttct 3000

aggccctggg gatatgtcac agacaaaaat cctgcactca atgaaactta tagtatattg 3060

agagaaagca gaccagaaac ataattaaga attatattag ctatctttat taaatataat 3120

gtagtgttag cttttatggc tgttgaaagt tattttttct tgtaacagtg ttgtatatct 3180

acaatgtgat tttcatttta ataatgaatt tattctacct gaatataatc atactgaata 3240

taccacagca aaatctaata gaaaataaaa ttaatatcat catttttatc tttaagtctt 3300

gttgactaaa aatgttataa aatcaataaa atttataaga ctgtg 3345

<210> 11
<211> 3213
<212> DNA
<213> Homo sapiens

<400> 11
agccactaga cgctccacca ccatcttttg catgtgcaac atttgcagcc ggacagaaaa 60

cctctcccag ggctatggag actgcgggaa aaatctggcg gctcgcgatg gattgctaag 120

gagaactagt cataatctta aaccaccgaa acctctttcc ttttttttct ttcttttctt 180

tcttttcttt tttttttttt ttttttggtt gattttaatt ttagcgccat cgtcttcaat 240

gcttctctga acagccttta ggaagagtgc gagagaaaga gagagagcgc gcgccaggga 300

gaggagaaaa gaagatgagg attatttcca gacagattgt cttgttattt tctggatttt 360

ggggactcgc catgggagcc tttccgagca gcgtgcaaat aggtggtctc ttcatccgaa 420

acacagatca ggaatacact gcttttcgat tagcaatttt tcttcataac accagcccca 480

atgcgtcgga agctcctttt aatttggtac ctcatgtgga caacattgag acagccaaca 540

gttttgctgt aacaaacgcc ttctgttccc agtattctag aggagtattt gccatttttg 600

gactctatga taagaggtcg gtacatacct tgacctcatt ctgcagcgcc ttacatatct 660

ccctcatcac accaagtttc cctactgagg gggagagcca gtttgtgctg caactaagac 720

cttcgttacg aggagcactc ttgagtttgc tggatcacta cgaatggaac tgttttgtct 780

tcctgtatga cacagacagg ggatactcga tactccaagc tattatggaa aaagcaggac 840

aaaatggttg gcatgtcagc gctatatgtg tggaaaattt taatgatgtc agctataggc 900

aacttctaga agaacttgac agaagacaag agaagaagtt tgtaatagac tgtgagatag 960

agagacttca aaacatatta gaacagattg taagtgttgg aaagcatgtt aaaggctacc 1020

attatatcat tgcaaacttg ggattcaagg atatttctct tgagaggttt atacatggtg 1080

gagccaatgt tactggattc cagttggtgg attttaatac acctatggta atcaaactaa 1140

tggatcgctg gaagaaacta gatcagagag agtatccagg atctgagact cctccaaagt 1200

acacctctgc tctgacttat gatggagtcc ttgtgatggc tgaaactttc cgaagtctta 1260

ggaggcagaa aattgatatc tcaaggagag gaaatgctgg ggattgtctg gcaaatcctg 1320

ctgctccatg gggccaggga attgacatgg agaggacact caaacaggtt cgaattcaag 1380

ggctgacagg gaatgttcag tttgaccact atggacgtag agtcaattac acaatggatg 1440

tgtttgagct gaaaagcaca ggacctagaa aggttggtta ctggaatgat atggataagt 1500

tagtcttgat tcaagatgta ccaactcttg gcaatgacac agctgctatt gagaacagaa 1560

cagtggttgt aaccacaatt atgcctctga tgaagaatcc tattttaaga aattgatcaa 1620

gaaagaaaag agttccgcgc tgttcgacca ttcctaacta aggctcaagt cttgttctcc 1680

agtgtagtaa atttaagctt atttttcatg tgggattctt cttggatgac caactctgga 1740

ctaccagaaa aaaaaaattt taagttctgt gacttttctg agatactaga acaaaagaag 1800

aattaatctt catctttctc aagaaataga tgttgacaaa gaatcactta gcgattctga 1860

catatcaatt cccctatctt gaaatgaggt cactgtatgt aaatgatgga attatatcac 1920

tccatttcca agggtagatt ttctataagt aaatatctcg gaatttgtgt gcttgttttc 1980

tgaatatata cagttgtttt ctttaaagat ctcttggaat tttgcctgtt ctgtgtgaaa 2040

taaagtgttt taatgtgcat tataggtatg atatagagaa tctcctttcc atccttgtta 2100

ctaaagggac tggacaaata aatcttaaaa ccaaaatact gaattaattt tgcaagcatg 2160

gctagttttt aggaagcatg ctatcaaaaa aaaaaagact aaaaatgact gaaaaaatcc 2220

aactgtttta tatatatata aatatatata tatttatata tatatataaa ggatattctg 2280

taaagttata tgttgtttga cagtaaagcc atcaatattt ttgctatcaa aatagtataa 2340

tactagtatc tttttgtatg aaaatgtaat ctttatataa ataatacctc tgatatttgc 2400

aactgcataa tcgttcagta attcaaaaag acatactaga atcctttttc tgaaagtgtt 2460

ccttcaattt gcttttgttg aaaacggtag tccaggacct atgatatccc tccacttcat 2520

tcattatgaa agaaatccct tgtagataaa caagatattg gcatctgcat gtaattatcc 2580

ccagattcag ctgaaaactc ccaacacaga tggaattggc tagacatttt aatatatgtg 2640

atacctatat ctagatatag aaggctgaga gtgagcactg gatataattc attttgattg 2700

aaattgatat ggtgttattg ttcttccagt tgtctgtcct ttgtgtatgt tcttatttat 2760

atgttgatac actgtaacac tatatgctat tgctaaataa aattgattga gaaattcagt 2820

tattcataaa tatttattga gcgtctgcta tgtgctaggc acagttctag gccctgggga 2880

tatgtcacag acaaaaatcc tgcactcaat gaaacttata gtatattgag agaaagcaga 2940

ccagaaacat aattaagaat tatattagct atctttatta aatataatgt agtgttagct 3000

tttatggctg ttgaaagtta ttttttcttg taacagtgtt gtatatctac aatgtgattt 3060

tcattttaat aatgaattta ttctacctga atataatcat actgaatata ccacagcaaa 3120

atctaataga aaataaaatt aatatcatca tttttatctt taagtcttgt tgactaaaaa 3180

tgttataaaa tcaataaaat ttataagact gtg 3213

<210> 12
<211> 5434
<212> DNA
<213> Mus musculus

<400> 12
gaagaatcaa agggagggga gggaagacca aatctatggt tggaccaggg cttctttttc 60

gccaatgtaa aaaggaatat gccgtacatc tttgcctttt tctgcaccgg ttttctaggt 120

gcggttgtgg gtgccaattt ccccaacaat atccagatag gggattattt tccaaaccaa 180

caatcacagg aacatgcggc ttttaggttt gctttgtcac aactcacgga gccccccaag 240

ctgcttcccc agatcgatat tgtgaacatc agcgacagct ttgagatgac ttaccgattc 300

tgttcccagt tctccaaagg agtgtacgcc atctttggat tttatgaacg aaggactgtc 360

aacatgctga cctccttctg tggggccctc catgtgtgct tcatcactcc aagttttccc 420

gttgacacat ccaatcagtt tgtccttcag ctgcgcccgg aactacagga agctctcatt 480

agcattatcg accattacaa gtggcagact tttgtctaca tttatgatgc tgaccggggc 540

ctgtcagtcc tgcagagagt cttggataca gccgccgaga agaactggca ggtgacggct 600

gtcaacattc taacaaccac ggaggaagga taccggatgc tctttcagga cctggagaag 660

aaaaaggaga ggctggtggt ggtggactgt gaatcagaac gcctcaacgc catcctgggc 720

cagattgtga agctagaaaa gaacggcatc gggtaccact acatcctcgc caacctgggc 780

ttcatggaca ttgacttaaa taagttcaag gagagtggag ccaatgtgac aggtttccaa 840

ctggtgaact acacagacac gatcccagcc agaatcatgc agcagtggag gacaagtgac 900
gctcgggacc acaccagggt ggactggaag aggccaaagt acacttctgc tcttacctat 960
gatggtgtga aggtgatggc ggaggccttc cagagcctgc ggaggcagag gattgacata 1020
tcccggcgag ggaatgctgg ggactgtctg gctaacccag ctgtgccctg gggccaaggg 1080
atcgacatcc agagagccct gcagcaggtg cgctttgaag gtttgacagg aaatgtgcag 1140
tttaacgaga aagggcgccg gaccaactac accctccatg tgatcgaaat gaagcatgat 1200
ggaatccgca agattggtta ctggaatgaa gatgataaat ttgtccccgc agccacggac 1260
gctcaggctg gaggggacaa ctcaagcgtc cagaatagaa cctacatcgt cacgactatc 1320
ctcgaagatc cttacgtgat gcttaaaaag aatgccaacc aatttgaagg caatgaccgc 1380
tatgagggct actgcgtgga actggctgcg gagatcgcca agcacgtggg ctattcctac 1440
cgacttgaga ttgtcagcga cggcaaatac ggagcccggg atcctgacac aaaggcctgg 1500
aatggcatgg tgggagagct agtctatgga agagcagatg tggcggtggc ccccttgacc 1560
ataaccttgg tccgggagga agtcatcgac ttctccaagc cattcatgag tttgggaatc 1620
tccattatga ttaagaagcc acagaagtcc aagccaggtg tcttctcctt tcttgaccct 1680
ttggcctacg agatctggat gtgtatagtg tttgcctaca ttggagtgag cgtcgtcctc 1740
ttcctggtca gccgtttcag tccttatgaa tggcacagtg aagagtttga agaaggacga 1800
gatcagacaa ccagtgacca gtcaaatgag tttggcatat tcaacagcct gtggttctcg 1860
ctgggggcct tcatgcagca aggatgtgac atttccccca ggtccctgtc tggacgcatc 1920
gtcggcggtg tctggtggtt cttcactttg attatcatct cctcatacac agccaacctg 1980
gctgccttcc tgactgtgga aaggatggtg tctcccatcg agagtgcaga ggacctggca 2040
aagcagacgg aaattgctta tgggacattg gaagcaggat ccactaagga gttcttcagg 2100
aggtctaaaa tcgctgtgtt tgagaagatg tggacataca tgaagtctgc agaaccgtct 2160
gtgtttgttc ggaccacaga ggagggcatg atcagagtga gaaagtctaa aggcaaatat 2220
gcctacctcc tggagtccac catgaatgag tacattgagc aacgcaagcc ctgtgacacc 2280
atgaaagtgg gaggtaactt ggattccaaa ggctatggca ttgcaacacc caaggggtcc 2340
gccctgagaa atccagtaaa cctggcagtg ttaaaactga acgagcaggg gcttttggac 2400
aaattgaaaa acaaatggtg gtacgacaag ggcgagtgcg gcagcggggg aggtgactcc 2460
aaggacaaga ccagtgctct gagcctgagc aatgtggcag gcgtgttcta catcctgatt 2520
ggagggctgg gattggccat gctggttgcc ttaatcgagt tctgctacaa atcccgtagc 2580

gagtcgaagc ggatgaaggg tttctgtttg attccacagc aatccatcaa tgaagccata 2640
cggacatcga ccctccccag gaacagcggg gcaggagcca gcggaggaag tggcagtgga 2700
gagaatggca gagtggtcag ccaggacttc cccaagtcca tgcaatccat tccctgcatg 2760
agccacagtt cagggatgcc cttgggagcc acaggattgt aactggagca gacaggaaac 2820
ccttggggag caggctcagg cttccccagc cccatcccaa gcccttcagt gccaaaaaca 2880
aagaaatgaa acaccgcctc caaccaccac aaccatatgg agggcaattc agccaatgtc 2940
cctgaagaat taaaaaaaaa aaaaaaattc cactagacca ccatcccttt tgttttggga 3000
ttttttttca accttttcca tttgctgagt gaagatggtc actaactcta tgctgcaata 3060
acgggagaga tcctgtccaa ggaagtctaa cgtctataaa aatggagtca ctgggatatc 3120
aatgaagaaa tcaaactgtt taattttaat tcagttgtta atgtgtctta gtgtgtgcat 3180
tattttctta ctattaaccc atggtttgca ggctctgttc aacccttttc cttcttccta 3240
tccccagccc ccacccccac cccacccctt cccatcactt ttcagtttca gattgaagac 3300
tcacgattgg ttccacctta caagcaagga aaggaatatt gaccttcaaa ctcattcagt 3360
gttggagctc tctatgaggc cttctatcct ttggcccatg ccttggatgg agacagacac 3420
tactggagaa gggggctgcc ttccccagaa agaatactac ctaaatgctg gttcatcaga 3480
gagtaactgt gaagagcatc tcagataagg gttcttaaaa ggggggatgg gcagagtaca 3540
gtagaggagg acttgttagg ttcattgttc cccggaagaa gcttcctgca atgcccagga 3600
gcatcccaca ggcaatagag taccaatatg gtagacctct ttcacagtca atctcagccc 3660
tggaaattat gttctactat gctcaggccc aaaggcaatg tgggggccaa agggcaactg 3720
gccttctgac ccactagagc cttgcaaaga gttggcagtc aggggggtgt catacatgat 3780
tcttgccttt tcctttgctt gatgtctata ctggatcccc atggtggagt aggactctct 3840
gtggagcctc tggcataaga ggaaatgttg cccgagctaa gagcaaaagg aaggtgatag 3900
ggaattatgg aagtccatcc aagcccctga cacagagcct tctgtgctgt ttcctgtcct 3960
aggtttgaga gaccaaatat gaacttgtga gttgtgtcaa tgcagagaac cggggttttg 4020
tgttaataga gtattgtttt agcatgtgat cagatcttaa gaaagaaaga aagaaagaca 4080
gcaaagcctg aattcttttt gagagatgga gatgaggtac atcaggggac cttaagatgc 4140
ctgagcccat cttttaactc taacacaggg atactaacct ttttccctgg ctaagacaga 4200
ctgagggcat tgcattacaa tggccagaaa ctacacccat gagtgcaacg accaaggtga 4260
aggacagcca gacaggaaag gtgacaaaag ccaccctgag tgatactagg ctgcctgaac 4320
ctttgggtgg gagtggaagt ccctagcaca cagctttgtg gagacctcca ctctcactct 4380

tggtgcaact gacttccact tcttatgcag ccactcccag gtgcatgctt cagtgagtgc 4440

cacatgccac tagcttttac caataaccat attctggctt attctcctac cctctgcttc 4500

cattcaatct tccccactag gggttatcat tggcaatgga cgagctgaag attttggagc 4560

ctatccatgg ttcagggcag tagaactggc ttcctctatg gatgcttcca gcggacaacc 4620

accatctctg tgtctctctc attctctctt cacctgccat agcaaagaaa catcccagaa 4680

tcgacccttc tcccattgat ccatgtctcc cgtgtcacca tgggcatggc atgtgaagca 4740

atgtcctgca gatggattac caccttttat ttctgccagc taacatcctc ccagcctcct 4800

gcctactact gtagactccg gtgtgaccca ccactgggga gttcacacct agaaagagat 4860

ggaactaaac tctactatca gctgcttcaa aaaccttttg tctacggcct gggaagatgt 4920

tctccagccc ctgagcctat tggttggata gagagagact gtggtggaaa tgagggtctt 4980

gctgggaggg gtccatgact ccggggaaga cgcttctcaa cgagggatgt gtggaaagaa 5040

aacagaagtg agaaagaagt taccttgtat tatgtatttt tactacactt ttcttaaaaa 5100

tggagcagtg ggaaaactct gaaagagatt gacatttttc tcaacaggaa tccatactta 5160

acagttctgg ctttcattaa attttgctct ttggtacctg ggccttttat ttaacatata 5220

tatttatttt aactctcttg gaagatgtgt gagggaaaaa aataactctc atttgatcag 5280

acactaaata tttggggtag ggatttctgt cttattttca aatagccagg gtttttcttc 5340

atttttttgg tatttgcata aagtgaacaa tatcacaaga attaaatcac tgtggaccca 5400

ttaaaaaaaa aaaaaaaaaa aaaaaaaaaa aaaa 5434

<210> 13
<211> 6841
<212> DNA
<213> Mus musculus

<400> 13
tttcctgtgt gtgtgcgtgt gtgtgtgtgt atgtgtgtgt atgtgtgtgc gcgcgcgtgt 60

gagagaggag agagggagaa gagagctcga gagagggtga ttgtgtgagt gcacgggagg 120

gtgctgatat tcccagacac cgggaccaca gcagcagctc agctaaaaac tgcattcagc 180

cagtcctcgg acttcaggag cagggacagg acgcaggcat caacagccac cagctaaacc 240

tgggagataa ggattcttct gccttcactt cgtggtttta gcagctcctt gctaaatttc 300

gacctcaaaa tgcagaggat ctaatttgct gaggaaaacg gtcaaagaag gaaaaggagg 360

aaaggaaaac gaggggatat tttgtggatg ctctactttt cttggaaatg caaaagatta 420

tgcatatttc tgtcctcctt tctcctgttt tatggggact gatttttggt gtctcttcta 480

acagcataca gatagggggg ctatttccaa ggggcgctga tcaagaatac agtgcatttc 540
gggtagggat ggttcagttt tccacttcgg agttcagact gacaccccat atcgacaatt 600
tggaggtagc caacagtttc gcagtcacca atgctttctg ctcccagttt tcaagaggcg 660
tctatgcgat tttgggtttt tacgacaaga agtctgtaaa taccatcaca tcattctgtg 720
ggacactgca tgtatccttc atcacaccaa gcttcccaac agatggcacg catccatttg 780
tcatccagat gcgacctgac ctcaaaggag cactccttag cttgattgag tactaccaat 840
gggataagtt cgcatacctc tatgacagtg acagaggctt atcaacactg caagctgtgc 900
tggattctgc tgcggagaag aagtggcagg tgactgctat caatgtgggg aacattaaca 960
atgacaagaa agatgagacc tacagatcac tctttcaaga tctggagtta aaaaaagaac 1020
ggcgtgtaat ccttgactgc gaaagggata aagtcaatga cattgtggac caggttatta 1080
ccattggaaa gcatgttaaa gggtaccatt atatcattgc aaatctggga tttactgatg 1140
gagacctgct gaaaattcag tttggaggag caaatgtctc tggatttcag attgtagact 1200
acgacgactc cctggtgtct aaatttatag aaagatggtc aacactcgaa gagaaagaat 1260
accctggagc acacacagcg acaattaagt atacttcggc cctgacttat gatgctgtcc 1320
aagtgatgac tgaagcattc cgcaatcttc ggaagcagag gattgaaatc tccaggagag 1380
gaaatgcagg agattgtttg gccaacccag ctgtgccttg gggacaaggc gtggaaatag 1440
aaagggccct caagcaggtt caagttgaag gtctctctgg aaatataaaa tttgaccaga 1500
acggaaaacg aataaactac acaattaaca tcatggagct caaaacaaat ggaccccgga 1560
agattgggta ctggagtgaa gtggataaaa tggttgtcac cctaaccgag ctcccctctg 1620
gaaatgacac atctgggctt gaaaacaaaa ctgtggttgt caccacaata ttggaatctc 1680
catatgttat gatgaagaaa aatcatgaaa tgcttgaagg gaatgagcgt tatgagggct 1740
actgtgttga cttagctgca gaaattgcca aacattgtgg attcaagtac aagctgacta 1800
ttgttgggga tggcaagtat ggggccaggg atgcagacac caaaatttgg aatggtatgg 1860
ttggagaact tgtatatggg aaagctgata ttgccattgc tccattaact atcactctcg 1920
tgagagaaga ggtgattgac ttctcgaagc cattcatgag ccttggaatc tctatcatga 1980
tcaagaagcc tcagaagtcc aaaccaggag tgttttcctt tcttgatcct ttagcctatg 2040
agatctggat gtgcattgtg tttgcctaca ttggggtcag tgtagtttta ttcctggtca 2100
gcagatttag cccctacgag tggcacactg aggaatttga agatggaaga gaaacacaaa 2160
gtagtgaatc aactaatgaa tttgggattt ttaatagtct ctggttttcc ttgggtgcct 2220

ttatgcggca aggatgcgat atttcgccaa gatctctctc tgggcgcatt gttggaggtg 2280

tgtggtggtt ctttaccctc atcatcatct cctcctacac ggctaactta gctgccttcc 2340

tgactgtaga gaggatggtg tcgcccatcg aaagtgctga ggatctgtct aagcaaacag 2400

aaattgctta tggaacatta gactctggct ccactaaaga gtttttcagg agatctaaaa 2460

ttgcagtgtt tgataaaatg tggacttata tgaggagtgc agagccctct gtgtttgtga 2520

ggactacggc agaaggagta gccagagtca ggaaatccaa agggaagtat gcctacttgc 2580

tggagtccac aatgaatgag tacatcgagc agaggaagcc ttgcgacacc atgaaagtgg 2640

gcggcaacct ggattccaaa ggctacggca tcgccacacc taaaggatcc tcattaagaa 2700

ccccagtaaa tcttgcagta ttgaaactca gtgagcaagg cgtcttagac aagctgaaaa 2760

acaaatggtg gtacgataaa ggtgaatgtg gagccaagga ctcgggaagt aaggaaaaga 2820

ccagtgccct cagtctgagc aacgttgctg gagtattcta catccttgtc ggggggcctg 2880

gtttggcaat gctggtggct ttgattgagt tctgttacaa gtcaagggcc gaggcgaaac 2940

gaatgaaggt ggcaaagaat gcacagaata ttaacccatc ttcctcgcag aattcccaga 3000

attttgcaac ttataaggaa ggttacaacg tatatggcat cgagagtgtt aaaatttagg 3060

ggatgacctt gagcgctgcc acgaggaaca aggcaaggct gtcaattaca ggaagtactg 3120

gagaaaatgg acgtgttatg actccagaat ttcccaaagc agtgcatgct gtcccttacg 3180

tgagtcctgg catgggaatg aatgtcagtg tgactgatct ctcgtgattg ataggaacct 3240

tctgagtgcc ttacacaatg gtttccttgt gtgtttattg tcaaagtggt gagaggcatc 3300

cgatatcttg aaggcttttc tttcagccaa gaattcttaa ctatgtggag ttcaccttga 3360

attgtaagga aagattaatt acaaacagag catccttttc tacttcgatg tcagaggaag 3420

cgtggtggac atgcacagct aacatggaag tactatcact taactgaagt ctttgtacag 3480

acaacaaacc cgtttccgca gccactattg ttagtctctt gattcataat gactaagcac 3540

acttgacatc aactgcatca agatgtgacc tgttttataa aaaaaaagag gaaaaaaaaa 3600

catttaaaat aaaaaaaata tttttaggta ttttcacaga caaactggct tttaaataaa 3660

tttgcttccg tactggttgg ttaagacaaa aacaaaaaca aaacaaaaca aaacaaaaaa 3720

aacaacaaaa aaacaaaaca aaacaaaaca aaaaaataaa gtcaaactgt gtgggaagtg 3780

atggggaaaa tggtccatgg tatcagttcc atatttttca aagccaaata tgtaaatgct 3840

gaggaaggaa acagaggaga ctccagtctt gtaatttaat attgttatta aaactttaat 3900

gtatcctgtt ctttaacatt tggtgttaat atgaaactac ttggcgatgc ttgacatttg 3960

aaataaacat tttttctatt gttttatttg caagtggtcc agttaatttt gcttagctac 4020

agtttggtca tacatccagt gatctaaaga cactaatgca tagtccttag gatcccctaa 4080

gaaaaagacc ccagttaaaa tacaggaata gtttttcttt gacaaataga atttacccca 4140

aatagtatat ctaaaactat tttttctgta catcatatat ataatagtaa aatatttgtt 4200

aattaaaaag cattaatcta tatagataaa tgcttgaaga tttaaaaatc agatttatta 4260

tcaaatgtca gattgtgtga ttttctgctc ttcaagtatt taaggttgtg taggagcagc 4320

aatcattata tataaagtga aaaaggcttg aaggtagctg ttcagatact tatacactgg 4380

ccaaaatcta taatctaagt atgtattttt agttttaaaa ttataagaac tgacatcagt 4440

ttttgaatac tatagtcacc tacaaatagt ttctttccac ttctatctat aaaatcaact 4500

ctaatatatt aattcttatt tatgcctcaa gtagaaggag cactttaatt tgtgtaatgt 4560

ggatactgtt ccatctctgt aactttccac taaagaaatc atactacact atttgattca 4620

tttaagccat ctatcttccc ttccttaaaa tatcttttat gtttatttcc actaatgtcc 4680

tccaaataaa attactttag agttaaaagc aactgacacc cagtagttat ctttaagatt 4740

tatatgttat ttttctctca taaaaaatct gtgtaatatt tgatgtctga ccaagtgctt 4800

ggtagagcat ccttactgac tatttttaag gatcagaaat gtaagattta acagtagcca 4860

cttgcattct acatggtaac atgtagtttc atgatgatct tcactaaatt ttaccatccc 4920

ttctataaat tccaagatca tacaaaataa tttgagtgca ttactcatca gggattctgg 4980

aggtacattt taagttttaa taataaatgc tagaatgacc aaattgcaga ctaattattt 5040

ccatattgta cttaaaatga gtttttaaaa attattttaa aagagatgac tatatacaat 5100

caaagctatt tattgtacct ctgggcctac tcttccaaaa attgagctta tcaatttttc 5160

tctgtcaagc ttgaactaat gtaaataatt ggaataatgt aaagttatat tttcatgttt 5220

ttatagttat gacaagaaca cttaatgtaa gagtatctcg agtatgaata atgttgattg 5280

gctaacacag agctcagttt taggcagtaa aatctagttc aatatccatg taacaatgca 5340

tcagtatatt gctgtataga tgtgtctgtg tgcatataag tgataagtgc tcaacaaaga 5400

gtggtgacag ctctctcaag gttttttttt taattcattt tctataaact gttatggaaa 5460

gaccaaaata attatgaact attcttatgt aaatttacaa ttgtccttta ctgtacattt 5520

tgtttacagt atagtacctt attctctgct gtgttaagtg ggtgtcatac tccaaggaga 5580

caaacacttt ctacaacttc tattgaagat tctgggattt ccaatttttc atgtgtacta 5640

tgtctgaaaa tgcttttgat tttattttta aatctaacat ctgatggatt tttcagagtg 5700

ttgtgaaacc ttcagtcata cataaaatat gttcttataa aatgtcaaca tctttttatt 5760

ttgtttttac ttaatggtat atcaatctaa taatgagaaa ccaaaagtgt atcatatatc 5820

ccccccccca aatctagatg tttttagtga gggagggcat cagtttgtca gcaaagtctg 5880

aatagtgtca aggcagaata ttttttaatt taaattaaga gaattagtaa ttctggtatt 5940

tcacacctat actcattagg ttctttggac atatatagaa atgtggctat tgattaaatc 6000

acttattttg taatagacaa cactggtaac ataccaggca ttgtgcactt agccctccta 6060

ctgtgcagtc atctcatttg ctgtggtact catattaaag agcaggaaag taacttgctt 6120

taaaatatgg tttgactttt aaaaattgac cacttattat ttaacttaaa aattctttcc 6180

actttaacta atttcttcac aaagctggtc atttgaataa ctacacaatc ctatttatat 6240

gtactttaaa gttattccaa tattatagtt ctcctaactt gaacataaat ttgtaaaaat 6300

ttttatactt aaatgtatca agtacttaac acaccaatat aaaggtgatt ctgcttaaaa 6360

atacaggaat tttacatttc taatctgtaa atactgtgga tattttcatg atcaaggtag 6420

tactttgaga ataagaagaa aattactagc aacattcctg taaaaacctt tagcacctac 6480

catatcttaa ggatatcatt aacttttaat tttaaatgaa gtgtatgtat gtgcgtgctg 6540

atgtgggaat atgcccatga gtgcaggtgt ctgctgataa caggggtggg cccaggtata 6600

tcagagctag agtggggtta tagatgcttg ccagctccta aaatggtttt tggagaaact 6660

agcctgggtc cactgcaagt gttctttctt aaccactgag caatcctcca gcaccaataa 6720

catcattaaa actttttaaa aagaacctct ttgcagttca ttaaactatg acataccaat 6780

ttttcatact ttacctaatg gcaatctgga ggtccatcta cataatagaa actattcgat 6840

g 6841

<210> 14
<211> 4993
<212> DNA
<213> Mus musculus

<400> 14
ccatgctctt gtcagcttcg ttttaggcgt agcacagcca ggcagaagaa aatggggcaa 60

agcgtgctcc gggcggtctt ctttttagtc ctggggcttt tgggtcattc tcacggagga 120

ttccccaaca ccatcagcat aggtggactt ttcatgagaa acacggtaca ggagcacagt 180

gctttccgct ttgctgtgca gttatacaac accaaccaga acaccactga gaagcccttc 240

catttgaact accacgtaga ccacttggat tcctccaata gtttttctgt gactaatgct 300

ttctgctccc agttctccag aggggtgtat gctatctttg gattctatga ccagatgtca 360

atgaacacac tgacctcctt ctgtggggcc ctgcatacat ctttcgtcac acctagcttt 420

cccactgatg ccgatgtgca gtttgtcatc cagatgcgcc cagccttaaa gggtgccatt 480
ctgagtcttc tgggttacta caagtgggag aagtttgtgt acctctatga cacagaacga 540
gggttttcca tcctgcaagc aattatggaa gcagcagtgc aaaacaactg gcaagtgaca 600
gcaaggtctg tgggaaacat aaaggacatc caggaattca gacgcatcat tgaagaaatg 660
gacagaaggc aggaaaaacg atacttgatt gactgtgaag tcgaaaggat taacacaatt 720
ttggaacagg ttgtgatcct ggggaaacat tcaagaggtt atcactacat gcttgctaac 780
ctgggtttta ctgacattgt actggaaaga gtcatgcatg ggggagccaa cattacaggt 840
ttccagattg tcaacaatga aaacccaatg gtccagcaat tcatacagcg ctgggtgaga 900
ctggatgaaa gggaattccc tgaagccaag aatgcaccac taaagtatat atctgcactg 960
acacacgacg caatactggt catagcagaa gccttccgat acctgaggag gcagcgagtg 1020
gatgtatccc gcagaggcag tgctggagac tgcttagcaa atcctgctgt gccctggagt 1080
caaggaattg atattgagag agctctgaaa atggtgcaag tacaaggaat gactggaaac 1140
atccaatttg acacttatgg acgtaggaca aattatacca ttgatgtata tgaaatgaaa 1200
gtctcaggct ctcgaaaagc tggttactgg aatgagtatg aaaggtttgt gcccttctca 1260
gatcaacaaa tcagcaatga cagctcatcc tccgagaaca ggaccatcgt agtgactacc 1320
attctggaat caccatatgt aatgtataaa aagaatcatg agcaactgga aggaaatgag 1380
cgctatgaag gctattgtgt cgatttagcc tatgaaatag ccaaacacgt aaggatcaaa 1440
tacaaattgt ccattgtcgg tgatgggaaa tatggcgcaa gggatccaga gactaaaata 1500
tggaatggca tggttgggga acttgtctat ggaagagctg atatagctgt tgctccactc 1560
actataacat tggtccgtga agaagtcata gatttttcaa agccatttat gagcctgggg 1620
atctccatca tgataaagaa gcctcagaaa tcaaagccag gcgtattttc attcctggat 1680
cctttagctt atgaaatctg gatgtgcatt gtcttcgctt acattggagt cagtgtagtt 1740
ctcttcctag tcagcagatt tagcccttat gagtggcact tggaagacaa caatgaagaa 1800
cctcgtgacc cacaaagccc tcctgatcct cccaatgaat ttggaatatt taacagtctt 1860
tggttttcct tgggtgcttt tatgcagcaa ggatgtgata tttctccaag atcactttct 1920
gggcgcattg ttggaggggt ttggtggttc ttcaccctga tcataatctc ttcctacact 1980
gcaaaccttg ctgctttcct gactgtggag aggatggtgt cccccataga gagcgctgaa 2040
gatttagcca agcagactga aattgcatac gggaccctgg actctggttc aacaaaagaa 2100
tttttcagaa gatccaaaat tgctgtgtat gagaaaatgt ggtcttacat gaaatccgca 2160
gagccatctg tgtttaccaa aacaacagct gatggggtag cccgagttcg gaagtccaag 2220

ggaaagttcg ccttcctgct ggagtcaacc atgaatgagt acattgagca gagaaagccg 2280

tgtgatacga tgaaagttgg tggaaatctg gattccaaag gctatggtgt ggcaacccct 2340

aaaggctcag cattaggaac gcctgtaaac cttgcagtat tgaaactcag tgaacaaggc 2400

atcttagaca agctgaaaaa caaatggtgg tacgataagg gggaatgtgg agccaaggac 2460

tccgggagta aggacaagac cagtgctcta agcctgagca atgtggcagg cgtgttctat 2520

atacttgtcg gaggtctggg gctggccatg atggtggctt tgatagaatt ctgttacaaa 2580

tcacgggcag agtccaaacg catgaaactc acaaagaaca cccaaaactt taagcctgct 2640

cctgccacca acactcagaa ttacgctaca tacagagaag gctacaacgt gtatggaaca 2700

gaaagtgtta agatctaggg gtacggttaa gatcccttcc caccggaagc atgcaatgag 2760

aggaaatcac tgaaaacgtg gctgcttcaa ggatcctgag ccagatttca ctctccttgg 2820

tgtcgggcat gacacgaata ttgctgatgg tgcaatgacc tttcaatagg aaaaactgat 2880

tttttttttc cttcagtgcc ttatggaaca ctctgagact tgcgacaatg caaaccatca 2940

ttgaaatctt tttgctttgc ttgaaaaaaa attaaaataa aaccaacaaa aaatggacat 3000

gcaagattcc agtatgcgaa aaaaattctt attaaaaaca ttaattcaac aaaagccatt 3060

ctttgatacc actgcacagt atacgaaacc atgttcgtta acacacacac aaacacacac 3120

acacacacac acacacacac actcacttat gtatacacat tcacacatac aaatttattc 3180

taattcagca aaatagaccc atcttagcta aaagaaataa ttcctcctga tttaaaataa 3240

aagaaatctg tctcccagtg ttcgggcaga cagactggca tttcttctag gatctgctga 3300

tcagatgttt ttggtatttc ctgttggtgg tgatgttctg tgcaccctat ttccttttga 3360

tgttgctgaa ttgtgtaaat ctttagaatg taaatgtgac acttaagaaa attcaaatgc 3420

tttgggaaag agactgaatg gtgacttagt gttcttgcaa tggttttgtg acaaaaaaaa 3480

aaatgtttgg agaactcccc atagtggagg tgaagggatt aatagagcct ttgaaattga 3540

catctgtgtg tagcatgggg cagggcactg tcaggcttac ctctcggtgc tttcctaaaa 3600

cggatcccgg agctttccaa gaagcctgga attacagccc acagatagat ctgtttttct 3660

taattccgtg tgcattttaa gtcaatatag ctgagtagtt gaatagtaag cactgaggtg 3720

agggaagtgc tgcctattct cccagacgtg tttggaatat ctttctcaga aacaacactg 3780

tgtttagctc tgctttctct gctaagtatg cctaagtgta caacacggag acagaaccgt 3840

gttgcaaggc aggccagtgg gttcagacca cagttctcaa tctgacttta ctcttgctag 3900

gtctgtttaa ccagctgttg cctgttaccg cctgcctgtg gctctccgtc agactgcatg 3960

tgtccttttc tagtttgcaa agactaagac gcattcctaa acctattgct aacctgaggg 4020

acctaagcat cactcccagg tccttgcttg gagcagtttc tctactgaac tgaagatcgc 4080

cccactgctc catgggctct caagtaacta attgcaaacc tgctgttgac gtcatcagac 4140

cagccagagg aaatagtctc cgctcactaa ttacctccta tataacaatg tatgctttct 4200

gtagttcagt agtatgctct catcagtaac gtgcattgga aagtttccag actgtacaaa 4260

ttaggagccc accttcattt cccaagtgtg acccttagat gcttagttga ctcgcgcata 4320

tttgctctcg tctttagaaa agaaaggaag aggtctcgtt ccaatgaaac atttctagaa 4380

aggtgtaata taaactttca ttccagaaag tgtgtcataa gcaaataact cacctgtcag 4440

attttaaatg gtattgaaca caaaagaaag ctgttgtgtt ttgttttgtt ttcatgaaac 4500

tgtgattttt caacttatga atgctataat gtcccagtgc gggaagctaa ctctgtgtga 4560

atatcaactt gtataaaaca aaccaactga cctatacaca aacatttttc ataggcactg 4620

tataaagaga aatgtatgtt tattgactca aatcagtttt tcagagagga aactttactg 4680

agatgaagaa gcgggtaaat tggtttgtta tttaaaaaaa aaaaaaactt gcatgtttaa 4740

aaaaatgttg attgcttcaa atttctgcta ttaacttcaa gctatgggag tttggcggta 4800

gtcaattgag gatttttttc catttctttt ctttttgttg ttaaagctgt acttcagtga 4860

acagaaaaat tgccaagcaa actaatggcc gtaaaagtgt aaattgcatg tgtgggcata 4920

attaccgaac ctcattgcca tgaggtatgt acaaagtttt aatacatttt gtaaataaaa 4980

ttgtaaagaa agc 4993

<210> 15
<211> 5458
<212> DNA
<213> Mus musculus

<400> 15
agcagaagcg gactaggctg ggcaaggctg agaggaaagg gctgggagcc gggaggcagc 60

ttgtcctccc gcgagcctgc aaggatgctg caggggactg agaccatggg gaggtgctaa 120

cttcctctcc aaccccccag cccgggatcc gggctggcat acccggcccc ccggggtagt 180

ttcctgccaa gctcaaccaa gaatccaaag gcgacttgga gggcataaaa agaaaaacac 240

tggttctgaa gaggggcccc cacctccaga gccggttcct cggcaaacgc gcggaaggta 300

ggaggaaggg gatcagatac tggagactgt acgccaaaac cccagcctga agactcgctc 360

cagagcctcc ctgaagacct gggcgatctg cgccatcatc ttcaatgcct ctctgaaaat 420

catttagcaa gagtgagaga aagagaggag agcgcgcacc agggagagga gcaaagaaga 480

tgaggattat ttgcaggcag attgtcttgt tgttttctgg attttgggga ctcgccatgg 540

gagcctttcc gagcagcgtt caaataggtg gtctctttat ccgaaacaca gaccaggaat 600

acactgcttt tcggctagct atctttcttc ataacaccag ccccaatgca tctgaagccc 660

ctttcaattt ggtacctcat gtggacaaca ttgagactgc caacagtttt gctgtgacaa 720

atgcattctg ttcccagtat tctagagggg tgtttgccat ttttggactc tatgacaaga 780

ggtcagtgca taccttgacc tccttctgca gtgctctgca catctctctc atcacaccaa 840

gcttccccac tgaaggagag agccagttcg tgcttcagct aagaccttca ttgagaggtg 900

cactcctgag cctcctggat cactatgaat ggaattgttt tgtcttcctg tatgatacag 960

acagggggta ttcaatactt caagctataa tggaaaaagc aggacagaat ggatggcatg 1020

tcagtgcgat atgtgtggaa aattttaacg atgtcagcta caggcaacta ctagaagagc 1080

ttgacagaag acaagagaag aaatttgtaa tagattgtga gatagaaagg cttcaaaaca 1140

tattagaaca aattgtgagt gttgggaagc acgtcaaagg ctaccattat atcatcgcaa 1200

atttgggttt caaagatatt tctcttgaga gatttataca tggaggagca aatgtcactg 1260

gattccagtt agtagatttt aatacgccca tggtgacgaa actaatggat cgctggaaga 1320

aactagatca acgagaatat ccaggatctg aaacacctcc aaagtacact tctgctctca 1380

cttacgatgg tgtcttggta atggctgaaa ctttccgaag tctcagaaga cagaaaattg 1440

atatttcaag gagaggaaat gccggggatt gtctggcaaa ccctgctgct ccctggggcc 1500

agggaattga catggagaga acactgaagc aggttcgaat tcaaggactg actgggaatg 1560

ttcaatttga ccactatgga cgtagagtta attacacaat ggatgtgttt gaattaaaaa 1620

gcacaggacc tcgaaaggtt ggctattgga acgatatgga taaattagtc ttgattcaag 1680

atgcgcctac tcttggcaat gacacagcag ctatcgagaa cagaacagtg gttgtaacca 1740

caattatgga atccccctac gttatgtaca agaaaaatca tgaaatgttt gaaggaaatg 1800

acaagtatga aggctactgt gtagacttgg catctgaaat tgcgaaacat atcggtatca 1860

aatataaaat tgccattgtc cctgatggaa aatatggagc aagggatgcg gacaccaaaa 1920

tttggaatgg gatggtagga gagcttgtgt atgggaaagc agagattgcc attgcacctc 1980

tgacaatcac gttggtgcga gaggaggtca tcgacttttc taagcctttt atgagtttag 2040

gcatctctat catgatcaaa aaacctcaga aatctaaacc aggagtgttt tccttcttgg 2100

accctctggc ctatgagatc tggatgtgca tagtgtttgc atacattggt gtcagcgtgg 2160

tcttgttcct agtcagtagg tttagcccat atgagtggca cacagaagag cctgaggatg 2220

gaaaagaagg acccagtgac caacctccca atgagtttgg catctttaac agcctctggt 2280

tttccctggg tgcctttatg caacaaggat gtgacatttc acccagatcc ctgtccggtc 2340

ggattgttgg aggcgtatgg tggttcttca ctctcatcat tatctcatcc tacactgcta 2400

atctggctgc attcctgaca gtggagagaa tggtctcccc catagaaagt gcagaagacc 2460

tggccaaaca aacagaaatt gcctatggaa cacttgattc gggatcaaca aaagaattct 2520

tcagaagatc aaaaatagca gtatatgaaa agatgtggac ctacatgcga tcggcagagc 2580

catctgtgtt cactagaact acagctgagg gcgtggcccg tgtccgcaag tccaagggca 2640

aatttgcctt cctcctggag tccacgatga atgaatacat tgagcagcga aagccctgtg 2700

acacgatgaa agtgggagga aacctggatt ccaaaggcta tggtgtagcg acgcccaagg 2760

gttcctcatt aggaactcct gtaaaccttg ccgttttgaa actcagtgag gcaggcgtct 2820

tagacaagct gaaaaacaaa tggtggtacg ataaaggtga atgtggaccc aaggactcgg 2880

gaagcaagga caagacgagt gccttgagcc tgagcaacgt agcaggcgtc ttctacattc 2940

tggttggcgg cttgggcttg gcaatgctgg tggctttgat agagttctgt tacaagtcca 3000

gggcagaggc gaagagaatg aagctgactt tttccgaagc cataagaaac aaagccaggt 3060

tatccatcac tgggagtgtg ggagaaaacg gccgtgtgct gacccccgac tgccccaagg 3120

ccgtacacac aggaactgcg attagacaga gctcgggatt ggctgtcatt gcatcggacc 3180

taccataaaa accaaaaaat taattgagtg ccttaatcaa actgtgttgg tgactgatgg 3240

aaacgcagcc ccgagggaca ggccaagcgt gggtcttggc taaacccatc ctttgactga 3300

gcaggaagga catcctgagt gccggacaga accagtgcat gagttccgca cggaaatgca 3360

gactcggatt tttttatcag gaaatctcac agtttagttt tttgggggga gtgggtgggg 3420

taccttaggt gtattaacag caacagatct cacttgagcg cacttaaaac taatcagact 3480

ggccagttag cgcattaaac tgtgaagttc tttctcagaa aggtcgtggt caccacagcc 3540

agggatcaac agttaataga ggtcaaagaa cctgctacct gtgtctccag aacacccata 3600

tagtccatgg aataatcttc cgctgagaca agccgtcact aacatgtgat aggaaaataa 3660

taaatagata aagcctgtga acaaacaatg agatttaaag aaagtattta cagttacttt 3720

ggaaataaaa caatactgaa acatgcttgc tttttaactg aagttaatcc agtagaggac 3780

aacgcaattg ttttttctaa ccatcttagg gaacaataca ttgcaataat tgataatagt 3840

gccatcactg taataaactt tagagacttt ttttaatgta aaagttgttg gtcaccttgt 3900

ttcctgtaac cttcactctg tcacacaagt tggctcatag gttgtgtttg tctatcagaa 3960

ataagaaaaa cacaagtgaa gaaaatgttg gcatgaagtc atccatctgc aatgaaaaac 4020

ctaaaagact acgggtcact catgttatca atataattta taatcctgtt cagtgtacaa 4080

aattgtgggt tttgtactca cccaaaagac taaaacacca gtttttctta cagtatctat 4140

ctacagagct tattctcccc tattatttgg gaaactctga gactccatat tgcagaagtc 4200

aaggaatagg ccatataaga aaatgtagct tgtttttatt atttctgcat atttatttct 4260

agatcttggg ctcatttgtt aacagaataa gttgtcaaag gtaaagtcct tgagtctggg 4320

aatgagccat cgttccaaaa ccaacacacc ctgtgtggaa attttacttg actctgtttt 4380

gctgcataga attcagtgtc tcttggccat tccccctcat tcctatacta aattctttga 4440

agacactggt aacagtttgt ggtagactac agttgaaaaa actcaatcct tatttctacc 4500

aagtaattgt gagtaaaatt ataccatgat catttttaag aaaagaaaac ccttatggtt 4560

caaaaggtct ggttcatttg aaatgatgaa tgagttcaat gatacagttt ttaaaaaacc 4620

aaaacaaaca aacaaacaaa aacaaatcac agattgaggg atctttgcag aaagcacaaa 4680

ttaggatgat gcccaaagtg tggccttgaa gaatgagttg agttttcaca ggagaaggtg 4740

ctggtaagat cctaggcgtg agcagcacac atagctgctt cttttatcag tgtttcatgt 4800

acaaggcttg tgcaaccagg tggcctggtg agggcacagg gtgtgacacc atggaagctc 4860

tgtcagaaat atggagaatt ggtttccttc cctcaatact tacaatccca ttcaataact 4920

gtgtgtcact acaatgtaat ccttataata catgtatgca ctgtcctttg ggaaaggtta 4980

cttctctgtt gttgagcaat gattcatttc acaacctagg ccactccctg gtatcttcct 5040

cttcctctct cactcctacc caagctctca gccagcaaat ggacctccac agcacacgga 5100

acacggcatc tactgtgtct ttgatctgta gatctttttt gtctttacct gactgtcaca 5160

catccttcag ccctgaaatt gaattttatg tgtactgaga agatttccat gattacccac 5220

caccctgccc atgacactga gttacctgcc attcttaatg tttcttaccc atcatggcac 5280

tcatcgtact gcattgtaat tgcctgtgta cttgtctata aactactaga ctgtcagctc 5340

cttgagggca gggactgtgt ctattttgtt cacagttgta tccccagcac ccagcacagt 5400

gcctggcata ttgtaggtgc ttaataaata tttgttgaat gaaagaaaaa aaaaaaaa 5458

<210> 16
<211> 2992
<212> DNA
<213> Rattus norvegicus

<400> 16
aattcggcac gagctcggct ccccttccaa gagaaacaag agaaacctca cggaaggaag 60

ggaggaagga aagaagcaag caaggaactg caggaagaaa agagccggca gagcatcaag 120

aagaatcgaa gggaggggag ggaagaccaa atctatggtt ggaccagggc ttctttttcg 180

ccaatgtaaa aaggaatatg ccgtacatct ttgccttttt ctgcaccggt tttctaggtg 240

cggttgtggg tgccaatttc cccaacaata tccagatagg ggggttattt ccaaaccaac 300

aatcacagga acatgcggct tttaggtttg ctttgtcaca actcacggag ccccccaagc 360

tgcttcccca gatcgatatt gtgaacatca gcgacagctt tgagatgact taccgtttct 420

gttcccagtt ctccaaagga gtctatgcca tctttggatt ttatgaacga aggactgtca 480

acatgctgac ctccttctgt ggggccctcc atgtgtgctt cattactcca agttttcctg 540

ttgacacatc caatcaattt gtccttcagc tacgcccgga actacaggaa gctctcatta 600

gcattatcga ccattacaag tggcaaacct ttgtctacat ttatgatgct gaccggggcc 660

tgtcagtcct gcagagagtc ttggatacag ccgcagagaa gaactggcag gtaacggctg 720

tcaacattct gacaaccacc gaggaaggat accggatgct ctttcaggac ctggagaaga 780

aaaaggagag gctggtggtg gttgactgtg aatcagaacg cctcaacgcc atcctgggcc 840

agatcgtgaa gctagaaaag aatggcatcg ggtaccacta catcctcgcc aatctgggct 900

tcatggacat tgacttaaat aagttcaagg agagcggagc caatgtgaca ggtttccagc 960

tggtgaacta cacagacacg atcccagcca gaatcatgca gcaatggagg acaagtgact 1020

cccgagacca taccagggtg gactggaaga ggccaaagta cacttctgct ctcacctatg 1080

atggtgtcaa ggtgatggct gaggccttcc aaagcctgcg gaggcagagg attgacatat 1140

cccgccgggg gaatgctggg gactgtctgg ctaacccagc tgtgccctgg ggtcaaggga 1200

tcgacatcca gagagccctg cagcaggtgc gcttcgaagg tttgacagga aatgtgcagt 1260

tcaacgagaa agggcgccgg accaattaca ccctccacgt gatcgaaatg aaacatgatg 1320

gaatccgaaa gattggttac tggaatgaag acgataaatt tgtccccgca gccaccgacg 1380

ctcaggctgg aggggacaac tcaagcgtcc agaataggac ctacatcgtc actactatcc 1440

tcgaagatcc ttacgtgatg cttaaaaaga atgccaacca gtttgagggc aatgaccgct 1500

atgagggcta ctgtgtggag ctggctgcag agatcgccaa gcacgtgggc tactcctacc 1560

gacttgagat tgtcagcgac ggcaaatatg gagcccggga tcccgacaca aaggcttgga 1620

atggcatggt gggagaactg gtctatggaa gagcagacgt ggctgtggct cccttgacca 1680

taaccttggt ccgggaggaa gtcatcgact tctccaagcc attcatgagt ttgggaatct 1740

ccattatgat taagaagcca cagaagtcca agccaggtgt cttctccttt cttgaccctt 1800

tggcctatga gatctggatg tgtatagtgt ttgcctacat tggagtgagc gtcgtcctct 1860

tcctggtcag ccgtttcagc ccctacgaat ggcacagcga agagtttgaa gagggacgag 1920

accagacaac cagtgaccag tcaaatgagt ttggcatatt caacagcctg tggttctccc 1980

tgggggcctt catgcagcaa ggatgtgaca tttcccccag gtccctgtcc ggacgcatcg 2040

tcggcggcgt ctggtggttc ttcactttga tcatcatctc ctcgtacaca gccaacctgg 2100

ctgccttcct gactgtggag aggatggtgt ctcccattga gagtgcagag gacctggcaa 2160

agcagacgga aattgcttat gggacattgg aagcaggctc cactaaggag ttcttcagga 2220

gatctaaaat cgctgtgttt gagaagatgt ggacatacat gaagtctgca gaaccatccg 2280

tgtttgttcg gaccacagag gaaggcatga tcagagtgag aaaatctaaa ggcaaatacg 2340

cctacctcct ggagtccacc atgaatgagt atattgagca acgaaagccc tgtgacacca 2400

tgaaagtggg aggtaacttg gattccaaag gctatggcat tgcgacaccc aaggggtccg 2460

ccctgagaaa tccagtaaac ctggcagtgt taaaactgaa cgagcagggg cttttggaca 2520

aattgaaaaa caaatggtgg tacgacaagg gcgagtgcgg cagcggggga ggtgactcca 2580

aggacaagac cagcgctttg agcctcagca atgtggcagg cgtgttctac atcctgattg 2640

gagggctggg actggccatg ctggttgcct taatcgagtt ctgctacaaa tcccgtagcg 2700

agtcgaagcg gatgaagggt ttctgtttga tcccacagca atccatcaat gaagccatac 2760

ggacatcgac cctcccccgg aacagtgggg caggagccag cggaggaggc ggcagtggag 2820

agaatggccg ggtggtcagc caggacttcc ccaagtccat gcaatccatt ccctgcatga 2880

gtcacagttc agggatgccc ttgggagcca caggattgta actggagcag acaggaaacc 2940

cttggggagc aggctcaggc ttccacagcc ccatcccaag cccttcagtg cc 2992

<210> 17
<211> 6745
<212> DNA
<213> Rattus norvegicus

<400> 17
   gagactgcct gcccctctct gtgactttcc tgtgtgtgtg cgtgtgtgtg tatgtgtgtg 60
   tatgtgtgtg cgcgctcgtg tgagagagga gagagggaga agagagctcg agagagggtg 120
   attgtgtgag tgcatgggag ggtgctgata ttcccagaca ccaggactac agcggcagct 180
   cagctaaaaa ctgcattcag ccagtcctcg ggacttcggg agcaggacaa ggacgcaagg 240
   catcaacagc caccagctac aactgggaaa taaggattct tctgccttca cttcgtgttt 300
   ttagcagctc cttgctaaat atcgacctca caatgcagag gatctaattt gctgaggaaa 360
   acagtcaaag aaggaagagg aagaaaggga aacgagggga tattttgtgg atgctctact 420
   tttcttggaa atgcaaaaga ttatgcatat ttctgtcctc ctttctcctg ttttatgggg 480
   actgattttt ggtgtctctt ctaacagcat acagataggg gggctatttc caaggggcgc 540
   tgatcaagaa tacagtgcat ttcgggtagg gatggttcag ttttccactt cggagttcag 600
   actgacaccc catatcgaca atttggaggt agccaacagt ttcgcagtca ccaatgcttt 660
   ctgctcccag ttttcaagag gagtctacgc aatttttgga ttttatgaca agaagtctgt 720
   aaataccatc acatcattct gtgggacact ccatgtgtcc ttcatcacac ctagcttccc 780
   aacagatggc acacatccat ttgtcatcca gatgcgacct gacctcaaag gagcactcct 840
   tagcttgatt gagtactacc aatgggacaa gttcgcatac ctctatgaca gtgacagagg 900
   cttatcaaca ctgcaagctg ttctggattc tgctgcagag aagaagtggc aggtgactgc 960
   tatcaatgtg gggaacatca acaatgacaa gaaagatgag acctacagat cgctctttca 1020
   agatctggag ttaaaaaaag aacggcgtgt aatcctggac tgtgaaaggg ataaagtaaa 1080
   tgacattgtg gaccaggtta ttaccattgg aaaacatgtt aaagggtacc attatatcat 1140
   tgcaaatctg ggattcactg atggggacct gctgaaaatt cagtttggag gagcaaatgt 1200
   ctctggattt cagattgtag actacgacga ttccctggtg tctaaattta tagaaagatg 1260
   gtcaacactg gaagagaaag aataccctgg agcacacaca gcgacaatta agtatacttc 1320
   ggccctgacg tatgatgctg tccaagtgat gactgaagca ttccgtaacc ttcggaagca 1380
   gaggattgaa atatcccgga gaggaaatgc aggagattgt ttggccaacc cagctgtgcc 1440
   ctggggacaa ggggtcgaaa tagaaagggc cctcaagcag gttcaagttg aaggcctctc 1500
   tggaaatata aagtttgacc agaatggaaa acgaataaac tacacaatta acatcatgga 1560
   gctcaaaaca aatggacccc ggaagattgg gtactggagt gaagtggata aaatggttgt 1620
   caccctaact gagctcccat caggaaatga cacgtctggg cttgaaaaca agactgtggt 1680
   ggtcaccaca atattggaat ctccatatgt tatgatgaag aaaaatcatg aaatgcttga 1740
   agggaatgag cgttacgagg gctactgtgt tgacttagct gcagaaattg ctaaacactg 1800
   tgggttcaag tacaagctga ctattgttgg ggatggcaag tatggggcca gggatgccga 1860
   caccaaaatt tggaatggta tggttggaga gcttgtctac gggaaagctg acattgcaat 1920
   tgctccatta actatcactc tcgtgagaga agaggtgatt gacttctcca agcccttcat 1980
   gagtcttgga atctctatca tgatcaagaa gcctcagaag tccaaaccag gagtgttttc 2040
   ctttcttgat cctttagcct atgagatctg gatgtgcatt gtgtttgcct acattggggt 2100
   cagtgtagtt ttattcctgg tcagcagatt tagcccctac gagtggcaca ctgaggaatt 2160
   tgaagatgga agagaaacac aaagtagtga atcaactaat gaatttggga tttttaatag 2220
   tctctggttt tccttgggtg cctttatgcg gcaaggatgc gatatttcgc caagatccct 2280
   ctctgggcgc attgttggag gtgtgtggtg gttctttacc ctgatcataa tctcctccta 2340
   cacggctaac ttagctgcct tcctgactgt agagaggatg gtgtctccca tcgaaagtgc 2400
   tgaggatctg tctaagcaaa cagaaattgc ttatggaaca ttagactctg gctccactaa 2460
   agagtttttc aggagatcta aaatcgcagt gtttgataaa atgtggactt atatgaggag 2520
   tgcagagccc tctgtgtttg tgaggactac cgcagaagga gtagccagag tccggaaatc 2580
   caaaggaaag tatgcctact tgctggagtc cacaatgaac gagtacatcg agcagaggaa 2640
   gccttgtgac accatgaaag tgggaggaaa cttggattcc aaaggctacg gcatcgccac 2700
   acctaaagga tcctcattag gaaccccagt aaatcttgca gtattgaaac tcagtgagca 2760
   aggcgtctta gacaagctga aaaacaaatg gtggtacgat aaaggtgaat gtggagccaa 2820
   ggactcggga agtaaggaaa agaccagtgc cctcagtctg agcaacgttg ctggagtatt 2880
   ctacatcctt gtcgggggcc ttggtttggc aatgctggtg gctttgattg agttctgtta 2940
   caagtcaagg gccgaggcga aacgaatgaa ggtggcaaag aatccacaga atattaaccc 3000
   atcttcctcg cagaattccc agaattttgc aacttataag gaaggttaca acgtatatgg 3060
   catcgagagt gttaaaattt aggggatgac cttgagtgat gtcatgagga gcaaggcaag 3120
   gctgtcaatt acaggaagta ctggagaaaa tggacgtgtt atgactccag aatttcccaa 3180
   agcagtgcat gctgtccctt acgtgagtcc tggcatggga atgaatgtca gtgtgactga 3240
   tctctcgtga ttgataggaa ccttctgagt gccttacaca atggtttcct tgtgtgttta 3300
   ttgtcaaagt ggtgagaggc atccgatatc ttgaaggctt ttctttcagc caagaattct 3360
   taactatgtg gagttcacct tgaattgtaa ggaaagataa attacaaaca gagcatcatt 3420
   ttctactccg atatcagagg aagcgtggtg gacatgcaca gctaacatgg aaatactatc 3480
   atttaactga agtctttgta cagacaacaa acccgtttcc gcagccacta ttgttagtct 3540
   cttgattcat aatgacttaa gcacacttga catcaactgc atcaagatgt gacctgtttt 3600
   ataaataaaa aggaaaaaaa catttaaaat aaaaaaatat ttttaggtat tttcacagac 3660
   aaactggctt ttaaataaat ttgcttccgt actggttggt taagacaaat acaaaaacaa 3720
   aaacaaaaca aaacaaaaac caaaaaaaac aaaacaaaac aaaacaaaaa ataaaaaaat 3780
   gataaagtca aactgagtgg gaagtgattg aaaaaaaggt ctgtggtatc agttccgtat 3840
   tttttcaaag ccaaatatgt aaatggtgag gaaggaaaca gaggagaatc cagtcgtgta 3900
   atttaatatt gttattaaaa ctttaatgta tcctattctt taacctttgg tgttaatatg 3960
   aaactacttg gtgatgcttg acatttgaaa taaacatttt tctattgttt tatttgcaag 4020
   tggtccaatt aattttgctt agctacagtt tggtcataaa tccagtgatc taaagacagg 4080
   aatgcatagt ccctaagatt cctaagaaaa ggcccccagt taaaagactg gaatattttt 4140
   tctctgacaa gtagaatttc ccccaaatag tctatgtaaa gctatttttt ctgtacatca 4200
   tactgtataa tagtaaaata tttgttaatt aaaaagcatt taatctatat agataaatgc 4260
   ttatagttta aaaatcagat ttattatcaa atgtcagaag gcgtgatttt ctgctcttga 4320
   agtatttaag gttgtgtagg agcagtaatc aatatagata aagtcagaaa tggcttgaag 4380
   gtatcaactg ttcagacacc cgtactggcc aaattctgta atctaagtat gcatcttcag 4440
   ttttaaaatt ataaaaacca acatcagttt ttgaatactt tagtgaccta gaaatagttt 4500
   ctttccattt ccatctataa aatcagccct aatttttatt tatgccccaa atagaagggg 4560
   cactttaata agtgtaatat ggatactctc ccatctctgt aactgttcac tgaagagatt 4620
   atactgtttt atttgatgaa tttaagccat ccatcttccc ttcattaaaa tgccttttat 4680
   ttttatttcc actaatgtgt tccaaatgag attagtttag ggttaaaagc acctgacaca 4740
   cagtagctat ctttaagatt tatgttattt ttttctcata aaaattctat gtaatatttg 4800
   gtgtctgacc aagtggttgg tagagcatcc ttattgacta tttttagaga tcagaaatgt 4860
   aagatttaac agtagccact tacattctac atggtaacat gtagttcgtg atgatcttca 4920
   ctaaatttta ccatccctcc tataaattcc gagatcattc aaaataattt gagtgcatta 4980
   ctcatcaggg atgctggagg tacattttaa gttttaataa taaatgctag aatgaccaaa 5040
   ttgcagacta attatttcca tattgtactt aaaattagtt tttaaaaatt attttaaaaa 5100
   aagatgacta tatacaatca atgctattta ttgtacctct gggcgtactc ttccaaaaat 5160
   tgagcttatc aatttttctc tgtcaagctt gaactaatgt aaataatcga aataatgtaa 5220
   agttatattt tcatgttttt atagatatga catgacaaga acatataatg taagagtatc 5280
   tcaagtgtgg ataatgttga ttggctaaca cagagctcag tcttaggcag taaaacctag 5340
   ttcaatatcc atgtaacaat gcatcagtat attgctgtat acatgtgtct gtgcatagaa 5400
   gtgataaatg ctcaaataag agtggtgaca gctatctcaa ggtttttttt aattcatttt 5460
   atataaactg ttatggaaag accaaaataa ttatgaacta ttcttatgta aatttacaat 5520
   tgtcctttaa tgtacatttt gtttacagta tagtacctta ttctctgctg tgttaagtgg 5580
   gtgtcgtact ccaaggagac aaacactttc tacaacttct attgaagaca ctgggatttc 5640
   caattttttc atgtgtacta tgtcagaaaa tgcttttgat tttattttta aatctaacat 5700
   ctgatggctt tttcagagtg ttgtggaaac ttcagtcata cataaaatat gttcttataa 5760
   aatgccaaca tctttttatt ctgtttttcc tcaatggtac aacaatctaa taatgagaaa 5820
   acagaaagtg tatcttatat tcttcccaaa taaatctaga tgtttgtagt gaaggagggc 5880
   atctctgctc agtttgttag aaaagtatga tagagtcaag gcagaatgtc tttttaattt 5940
   aaatgaagag aattggtcgt tgtggtacgt catgctcctt aagcactttg gacatagaga 6000
   gaaatgtggc aattgattaa atcaccaatt ctgcatacag gcattgtgta cttcagcctt 6060
   ccttgtgcag tcctctcatt tggtatggta ctcatattaa agagcaggga agtaacttgc 6120
   tttaaaatat ggtttgattt ttaaaaactg accatttatt atttgactta aaaattcttt 6180
   ccattttaac cagttttgac aaagctggtc atttgaatat ttacacaatc ctatttacat 6240
   gttctccaaa attattctaa catgacagtt ctaactttca cataaatttg tcaaagtttt 6300
   atatttaaat gtatcaggta tttaacacac caatataaag gcgaatctgc ttaaaataca 6360
   gtaattttac atttctaatc tgtaagtact atggatattt tgatgatcat gatggtattc 6420
   ggagaaaaag aagagaatta ctagcaatat tccggtaaaa aaaagttacc accaaccatt 6480
   tcttaatgat atcattaatt tttatttttc aattaagtgt acgtatgtgt gtgcctgctg 6540
   tgggaatgtg ccagtgagtg caggtgcctg ctgataacag gggtgaggcc aattatatca 6600
   gagctagagt tggggtgttc ataggtgttt gggagctccc aaatggtttg cgggaaacta 6660
   gcctgggtcc actgcaagtg ttctttctca aaccactgaa caattctcca gtaccaacaa 6720
   tatcattaaa ttttttttta aaaaa 6745

<210> 18
<211> 5052
<212> DNA
<213> Rattus norvegicus

<400> 18
tgcgcccatg ctcttgtcag cttcgtttta ggcgtagcac agccaggcag aagaaaatgg 60

ggcaaagcgt gctccgggcg gtcttctttt tagtcctggg gcttttgggt cattctcacg 120

gaggattccc caacaccatc agcataggtg gacttttcat gagaaacacg gttcaggagc 180

acagcgcttt ccgctttgct gtgcagttat acaacaccaa ccagaacacc actgagaagc 240

ccttccattt gaattaccac gtagaccact tggattcctc caatagtttt tctgtgacta 300

atgctttctg ctcccagttc tccagagggg tgtatgctat ctttggattc tatgaccaga 360

tgtcaatgaa caccctgacc tccttctgtg gggccctgca cacatctttt gtcacaccta 420

gctttcccac tgatgcagat gtgcagtttg tcatccagat gcgcccagcc ttgaagggtg 480

ccattctgag tcttctcggt tactacaagt gggagaagtt tgtgtacctc tatgacacag 540

aacgagggtt ttctgtccta caagcaatta tggaggcagc agtgcaaaac aactggcaag 600

tgacagcaag gtctgtggga aacataaagg acgtccagga attcagacgc atcattgaag 660

aaatggacag aaggcaggaa aaacgatact tgattgactg tgaagtcgaa aggattaaca 720

caattttgga acaggttgtg atcctgggga agcattcaag aggctatcac tacatgcttg 780

ctaacctggg ttttactgac attttactgg aaagagtcat gcatggggga gccaacatta 840

caggtttcca gattgtcaac aatgaaaacc caatggttca gcagttcata cagcgctggg 900

tgagactgga tgaaagggaa ttccctgaag ccaagaatgc accactgaag tatacatctg 960

cgctgacaca tgacgcaata ttggtcatag cagaagcctt ccgatacctg aggagacaga 1020

gagtggatgt ctcccgcaga ggcagtgctg gagactgctt agcaaatcct gctgtgccct 1080

ggagtcaagg aattgatatt gagagagctc tgaaaatggt gcaagtacaa ggaatgactg 1140

gaaacatcca atttgacact tatggacgta ggacaaatta taccattgat gtctatgaaa 1200

tgaaagtctc gggttctcga aaagctggtt actggaacga atatgaaagg tttgtgccct 1260

tctcagatca acaaatcagc aatgacagct catcctcaga gaaccggacc attgtagtga 1320

ctaccattct ggaatcacca tatgtgatgt ataaaaagaa tcatgagcag ctggaaggaa 1380

atgagcgcta tgaaggctac tgtgttgatt tagcctatga aatagccaaa cacgtaagga 1440

tcaaatacaa attgtccatt gtcggtgatg ggaaatatgg cgccagagat ccagagacta 1500

aaatatggaa tggcatggtt ggggaacttg tctatggaag agctgatata gctgttgctc 1560

cactcactat aacattggtc cgtgaagaag tcatagattt ctcaaagcca tttatgagcc 1620

tgggaatctc catcatgata aagaagcctc agaaatcaaa gccaggcgtc ttttcattcc 1680

tggatccttt ggcttatgaa atctggatgt gcattgtctt cgcttacatt ggagtcagtg 1740

tagttctctt cctagtcagc agatttagcc cttatgaatg gcacttggaa gacaacaatg 1800

aagaacctcg tgacccacaa agccctcctg atcctcccaa tgaatttgga atatttaaca 1860

gtctttggtt ttccttgggt gctttcatgc agcaaggatg tgatatttct ccaagatcac 1920

tttctgggcg cattgttgga ggggtttggt ggttcttcac cctgatcata atctcttcct 1980

acactgcaaa ccttgctgct ttcctgactg tggagaggat ggtgtcccct atagagagcg 2040

ctgaagactt agccaagcag actgaaattg catatgggac cctggactct ggttcaacaa 2100

aagaattttt cagacgatcc aaaattgctg tgtatgagaa aatgtggtct tacatgaaat 2160

ccgcagagcc atctgtgttt accaaaacaa cagctgacgg ggtagcccga gttcggaagt 2220

ccaagggaaa gttcgccttc ctgctggagt cgaccatgaa cgagtacatt gagcagagaa 2280

agccgtgcga tacgatgaaa gttggtggaa atctggattc caaaggctat ggtgtggcaa 2340

cccctaaagg ctcagcatta ggaacgcctg taaaccttgc agtattgaaa ctcagtgaac 2400

aaggcatctt agacaagctg aaaaacaaat ggtggtacga taagggggaa tgtggagcca 2460

aggactccgg gagtaaggac aagaccagtg ctctaagcct gagcaatgtg gcaggcgtgt 2520

tctatatact tgtcggaggt ctgggcctgg ccatgatggt ggctttgata gaattctgtt 2580

acaaatcacg ggcagagtcc aaacgcatga aactcacaaa gaacacccaa aactttaagc 2640

ctgctcctgc caccaacact cagaattacg ctacatacag agaaggctac aacgtgtatg 2700

gaacagaaag tgttaagatc tagggatccc ttcccaccag aagcatgcaa tgagaggaaa 2760

tcactgaaaa cgtggctgct tcaaggatcc tgagccggat ttcactctcc ctggtgtcgg 2820

gcatgacacg aatattgctg atggtgcaat gacctttcaa taggaaaaac tgattttttt 2880

tttccttcag tgccttatgg aacactctga gacttgcgac aatgcaaacc atcattgaaa 2940

tctttttgct ttgcttgaaa aaaaattaaa ataaaaccaa caaaaaatgg acatgcaaga 3000

ttccagtatg cgaaaaaaat cttattaaaa acatcaattc aacaaaagcc attctttgat 3060

accactgcac agtacacaaa accatgtttg ttaacacaca cacatgcaca cactctctca 3120

cacacacacg cacacactct cacacacaca cactcacaca tacacacaca cacacacgca 3180

gacatgcacg tacgtataca cactcacaca tacaaattta ttcgaattca gcaaatagac 3240

ccatcttagc taaaagaaga attcctcctg atttaaaaga aaaaatctgt ctcccagtgt 3300

tcgggcagac agactggcat ttcttctagg atctgcagat cagatgtttt tggtatttcc 3360

tgttggtggt gatgttctgt gcacctattt ccttttgatg ttgctgaatt gtgtaaatct 3420

ttagaatgta aatgtgacac ttaagaaaat tcaaatgctt tgggaaagag actgaatggt 3480

gacttagtat tcttgcaatg gttttatggg aaaaaaatgt ttggagaact ccccatagtt 3540

gaggtgaagg gattaataga gcttttgaaa ttggcttctg tgtgtaccca ggggcagggc 3600

actatcaggc atacctctcg gtgctttcct aaaacggatc ccggagcttt cctagaagcc 3660

tggaattaga gctcacagat ctgtttttct tgcttcagtg tgcattttaa gtcaatatag 3720

ccgagtagtc gaatagtaag cattgaggtg agggaaatgt tgcctattct cccagatgtg 3780

tttggaatat ctttctcaga aaacaacact gtgtttagct ctgctttctc tgctaagtat 3840

gccttttaag tgtacaacac ggagacagga ccgtgttgca aggcaggcca gtggattcag 3900

accacagttc tcaatctgac tttactcttg ctaggtctgt tttaccagct gttgcctgtt 3960

accgcctgcc tgtggctctc cgtcagactg catgtgtcct tttctagttt gcaaagacta 4020

agatgctttc ctaaacctat cgctaacctg agggacctaa gtaagcatca ctcccaggtc 4080

cttgcttgga gcagtttctc tactgactca ccgaagatcg ccccactgct ccatgggctc 4140

tcaagtaact aattgcaaac ctgctgttgg catcaccaga ccagtcagag gaaatagtct 4200

ccgctcacta attacctcct atataacgac gtatgcttta tgtagttcag tagtatgctc 4260

tcatcagtaa cgtgcattgg aaagtttcca gactgtacaa attaggagcc cacctccatt 4320

tcccaagtgt gacccttaga tgcttagttg actcgctgca catttgctct cgtctttaga 4380

aaagaaagga agaggtctcg ttccagtgaa acatttccag aaaggtgtaa tataaacttt 4440

cattccagaa agtgtgtcat gagcaaataa ctcacctgtc agatttttaa atggtattga 4500

acacaaaaga aagctgttgt gttttgtttt gttttgttct catgaaactg tgatctttca 4560

acttatgaat gctgtaattg tcccagtgcg ggaagctaac gctgtgtgaa tatgaagttg 4620

tataaagcaa accaaccgac ctatacacaa acacttttca taggcactgt ataaagagaa 4680

atgtatgttt attgactcaa atcagttttt cagagaggaa actttactga gattaagaag 4740

cgggtaaatt ggtttgttat ttaaaaaaaa aacttgcatg tttaaaaaaa tgttgattgc 4800

ttcaaatttc tgctattaac ttcaagctat gggagtttgg cggtagtcaa ttgaggattt 4860

ttttccaatt cttttctttt tgttgttaaa gctgtacttc aatgaacaga aaaattgcca 4920

agcaaactaa tggccgtaaa agtgtaaatt gcatgtgtgg gcataattac cgaacctcat 4980

tgccatgagg tattgtacaa agttttaata cattttgtaa ataaaattgt aaagaaagaa 5040

aaaaaaaaaa aa 5052

<210> 19
<211> 4141
<212> DNA
<213> Rattus norvegicus

<400> 19
gctgagtgga aagagctagg agccgggagg cagcctgtcc tccctcaagc ctgcaaggat 60

gcggcagggg actgagacca tggggaggtg ctaacttcct cttcaacccc ccagcccggg 120

atccgggctg gcatacccgg ccccccgggc tagtttcctg ccaagcgcaa ccaagaagcc 180

aaaggcgatt tggagggcat aaaaagaaaa agagtggttc tggagagggg tgcccacctc 240

cagagccggt ccctctgcaa acaggcaaag ataggagaaa ggggatcaga tactggagtg 300

tacgcaaaat cctctgtctg tggactcgca ccagagcctc ccagaaaacc tgggcgatct 360

gcgccatcgt cttcaatgcc tctctgaaaa gcctttagca agactgagag aaagagaaaa 420

gagagcgcgc cagagagagg agcaaagaag atgaggatta tttgcaggca gattgtcttg 480

ttgttttctg gattttgggg actcgccatg ggagcctttc caagcagcgt tcaaataggt 540

ggtctcttca tccgaaacac agaccaggaa tacactgctt ttagactggc aatctttctt 600
cataacacca gccccaatgc atcggaagct cctttcaatt tggtacctca tgtggacaac 660
attgagactg ccaacagttt tgctgtgaca aacgccttct gttcccagta ttctagaggg 720
gtgtttgcca tttttggact ctatgacaag agatccgtgc ataccttgac ctcgttctgc 780
agtgctctgc acatctctct catcacacca agctttccca ctgaagggga gagccagttt 840
gtgctgcagc taagaccttc actgagaggt gcactcctga gcctcctgga tcactatgag 900
tggaactgtt tcgtcttcct gtatgataca gacagggggt attcaatact tcaagctata 960
atggaaaaag caggacaaaa tggatggcat gtcagtgcaa tatgtgtgga aaattttaat 1020
gatgtcagct acaggcaact gctagaagag cttgacagaa gacaagagaa gaaatttgtg 1080
atagattgtg agatagagag gcttcaaaac attttagaac aaattgtgag tgttgggaag 1140
catgtcaaag gctaccatta tatcatcgca aatttgggtt tcaaggatat ttctcttgag 1200
agatttatac atggaggagc aaatgtaaca ggattccagt tggtagattt taatacaccc 1260
atggtaacca aactaatgga tcggtggaag aaactagatc agagagaata tccaggttct 1320
gaaacacctc caaagtacac ctctgctctc acttatgatg gagtcctggt gatggctgaa 1380
actttccgaa gtctcagaag acagaaaatt gatatttcaa ggagaggaaa tgctggggac 1440
tgtctggcaa accctgctgc tccctggggc cagggaattg acatggagag gacactgaag 1500
caggttcgaa ttcaagggct gactgggaat gttcaatttg accattatgg acgtagagtt 1560
aattacacaa tggatgtgtt tgaactgaaa agcacaggac ctcgaaaggt tggctactgg 1620
aatgatatgg ataaattagt cttgattcaa gatatgccta ctcttggcaa tgacacagca 1680
gctattgaga acagaacagt ggttgtaacc acaattatgg aatctcccta tgttatgtac 1740
aagaaaaatc atgaaatgtt tgaaggaaat gacaagtacg aaggctactg tgtagatctg 1800
gcatcggaaa ttgcaaaaca tattggtatc aaatataaaa ttgccattgt tcctgatgga 1860
aaatatggag caagggacgc agacactaag atctggaatg ggatggtagg agagcttgtg 1920
tatgggaaag cagagattgc tattgcccct ctgacaatca cattggttcg agaggaagtc 1980
atcgattttt ctaagccttt tatgagttta ggcatctcta tcatgatcaa aaaacctcag 2040
aaatctaaac caggagtctt ttccttcttg gaccctctgg cctatgagat ctggatgtgc 2100
atagtgtttg catacattgg tgtcagtgtg gtcttgttcc tagtcagtag gtttagccca 2160
tatgagtggc acacagaaga acctgaggat gggaaggaag gacccagtga ccagcctccc 2220
aatgaatttg gcatctttaa cagcctttgg ttttccctgg gtgcctttat gcaacaagga 2280
tgtgacattt cacccagatc cctgtcaggt cggattgttg gaggcgtgtg gtggttcttc 2340

acactcatca ttatatcgtc ctacactgct aatctggctg cattccttac tgtggagaga 2400
atggtctccc ccatagaaag tgcagaagac ctggccaaac aaacagaaat tgcctatgga 2460
acacttgatt ctgggtcaac aaaagaattc ttcagaagat caaaaatagc agtgtatgaa 2520
aagatgtgga cctacatgcg atcggcagag ccgtctgtgt tcactagaac tacagctgag 2580
ggcgtggctc gtgtccgcaa gtccaagggc aaatttgcct ttctcctgga gtccacgatg 2640
aatgaataca ttgagcagcg aaagccctgt gacacgatga aagtgggagg aaacctggat 2700
tccaaaggct atggtgtagc aacgcccaag ggttcctcat taagaactcc tgtaaacctt 2760
gccgttttga aactcagtga ggcaggcgtc ttagacaagc tgaaaaacaa atggtggtac 2820
gataaaggtg aatgtggacc caaggactcg ggaagcaagg acaagacgag tgccttgagc 2880
ctgagcaacg tagcaggcgt cttctacatt ctggttggcg gcctgggctt ggcaatgctg 2940
gtggctttga tagagttctg ttacaagtcc agggcagagg cgaagagaat gaagctgact 3000
ttttccgaag ccataagaaa caaagccagg ttatccatca ctgggagtgt gggagaaaac 3060
ggccgtgtgc tgacccctga ctgccccaag gccgtacaca caggaactgc aattagacaa 3120
agttcgggat tggctgtcat tgcatcggac ctaccataaa aaccaaaaaa ataattgagt 3180
gccttaatca aactgtgttg gtgactgact gaaacgcagc cctgagggaa aggccaagag 3240
tgggtcttgg ctaaatccat cctttggctt gagagcagga agagcatact gagtgccgga 3300
cagaaccagt gcatgatttc agcacggaaa tgcagactcg gatttttttt tatcaggaaa 3360
actcacaatt tagttttttt ggggatagag ggtgggggat agctgggata ggtgtattaa 3420
cagcaacaaa tttcacatga gcgcacttaa aactaatcag actggtcagt tagtgcatta 3480
aactgtgaag ttctttctca gaaaggtctt agtcactaca gcaacagatt caacagttac 3540
tagaggtcaa ggaaccttct acctgtgtct ccagaacacc catttagtcg atggcataat 3600
ctgcagctga aacaagcagt cactaagatg tgataagaaa ataataaata tgtaaatcct 3660
gtgaaaaaaa acaacaaaat ttaaagaaag tatttacagt tactttggaa ataaaacaat 3720
actgaaacat gcttgctttt taactgaagt aaatccagta gaggacaaca caattgtttt 3780
ttctaaccat cttagggaac aatacattgc aataattgat aataatgcca tcactgtaat 3840
aaactttaga gacttttttt taatgtaaaa gttgtttgtc accttgtttc ctgtaacctt 3900
cactctgtca cacaagttgg ctcataggtt gtgtttgtct atcagaaaca acaacaaaaa 3960
aaatgaagaa aatgttggca tgaagtcatc catctgcaac gtagaattta aaagactacg 4020
ggtcattcat gttatcaata taatttataa tcctgttctg tgtacaaaat tgtggttttg 4080

tacccaccca aaagaataaa acaacagttt ttcttacagt acctaaaaaa aaaaaaaaaa 4140

a 4141

<210> 20
<211> 5648
<212> DNA
<213> Pan troglodytes

<400> 20
aggagaggga gtgggggagc cagcgctcca gctagcatga ggacgggctt cttttcccgt 60

gctcagttaa tctggctgtc agttggtgtt aacgctgcag tttaagtgct cggattccaa 120

gggaaacaga caaacctcac ggaaggaagg aagcaagcaa gcaaggaagg aactgcagga 180

gggaaagaac aggcagaaca gcgagaagaa taaagggaaa gggggggaaa caccaaatct 240

atgattggac ctgggcttct ttttcgccaa tgcaaaaagg aatatgcagc acatttttgc 300

cttcttctgc accggtttcc taggcgcggt agtaggtgcc aatttcccca acaatatcca 360

gatcggggga ttatttccaa accagcagtc acaggaacat gctgctttta gatttgcttt 420

gtcgcaactc acagagcccc cgaagctgct cccccagatt gatattgtga acatcagcga 480

cagctttgag atgacctata gattctgttc ccagttctcc aaaggagtct atgccatctt 540

tgggttttat gaacgtagga ctgtcaacat gctgacctcc ttttgtgggg ccctccacgt 600

ctgcttcatt acgccgagct ttcccgttga tacatccaat cagtttgtcc ttcagctgcg 660

ccctgaactg caggatgccc tcatcagcat cattgaccat tacaagtggc agaaatttgt 720

ctacatttat gatgccgacc ggggcttatc cgtcctgcag aaagtcctgg acacagctgc 780

tgagaagaac tggcaggtga cagcagtcaa cattttgaca accacagagg agggataccg 840

gatgctcttt caggacctgg agaagaaaaa ggagcggctg gtggtggtgg actgtgaatc 900

agaacgcctc aatgctatct tgggccagat tataaagctg gagaagaatg gcatcggcta 960

ccactacatt cttgcaaatc tgggcttcat ggacattgac ttaaacaaat tcaaggagag 1020

tggcgccaat gtgacaggtt tccagctggt gaactacaca gacactattc cggccaagat 1080

catgcagcag tggaagaata gtgatgctcg agaccacaca cgggtggact ggaagagacc 1140

caagtacacc tctgcgctca cctacgatgg ggtgaaggtg atggctgagg ctttccagag 1200

tctgcggagg cagagaattg atatatctcg ccgggggaat gctggggatt gtctggctaa 1260

cccagctgtt ccctggggcc aagggatcga catccagaga gctctgcagc aggtgcgatt 1320

tgaaggttta acaggaaacg tgcagtttaa tgagaaagga cgccggacca actacacgct 1380

ccacgtgatt gaaatgaaac atgacggcat ccgaaagatt ggttactgga atgaagatga 1440

taagtttgtc cctgcagcca ccgatgccca agctgggggc gataattcaa gtgttcagaa 1500

cagaacatac atcgtcacaa caatcctcga agatccttat gtgatgctca agaagaacgc 1560

caatcagttt gagggcaatg accgttacga gggctactgt gtagagctgg cggcagagat 1620

tgccaagcac gtgggctact cctaccgtct ggagattgtc agtgatggaa aatatggagc 1680

ccgagaccct gacacgaagg cctggaatgg catggtggga gagctggtct atggaagagc 1740

agatgtggct gtggctccct taactatcac tttggtccgg gaagaagtta tagatttctc 1800

caaaccattt atgagtttgg ggatctccat catgattaaa aaaccacaga aatccaagcc 1860

gggtgtcttc tccttccttg atcctttggc ttatgagatt tggatgtgca ttgtttttgc 1920

ctacattgga gtgagcgttg tcctcttcct ggtcagccgc ttcagtccct atgaatggca 1980

cagtgaagag tttgaggaag gacgggacca gacaaccagt gaccagtcca atgagtttgg 2040

gatattcaac agtttgtggt tctccctggg agccttcatg cagcaaggat gtgacatttc 2100

tcccaggtcc ctgtctggtc gcatcgttgg tggcgtctgg tggttcttca ccttaatcat 2160

catctcctca tatacagcca atctggccgc cttcctgacc gtggagagga tggtgtctcc 2220

cattgagagt gcagaggacc tagcgaagca gacagaaatt gcctacggga cgctggaagc 2280

aggatctact aaggagttct tcaggaggtc taaaattgct gtgtttgaga agatgtggac 2340

atacatgaag tcagcagagc catcagtttt tgtgcggacc acagaggagg ggatgattcg 2400

agtgaggaaa tccaaaggca aatatgccta cctcctggag tccaccatga atgagtacat 2460

tgagcagcgg aaaccctgtg acaccatgaa ggtgggaggt aacttggatt ccaaaggcta 2520

tggcattgcg acacccaagg ggtctgccct gagaggtccc gtaaacctag cggttttgaa 2580

actcagtgag caaggcgtct tagacaagct gaaaagcaaa tggtggtacg ataaagggga 2640

atgtggaagc aaggactccg gaagtaagga caagacaagc gctctgagcc tcagcaatgt 2700

ggcaggcgtg ttctacatcc tgattggagg acttggacta gccatgctgg ttgccttaat 2760

cgagttctgc tacaaatccc gtagtgaatc caagcggatg aagggttttt gtttgatccc 2820

acagcaatcc atcaacgaag ccatacggac atcgaccctc ccccgcaaca gcggggcagg 2880

agccagcagc ggcggcagtg gagagaatgg tcgggtggtc agccatgact tccccaagtc 2940

catgcaatcg attccttgca tgagccacag ttcagggatg cccttgggag ccacgggatt 3000

gtaactggag cagatggaga ccccttgggg agcaggctcg ggctccccag ccccatccca 3060

aacccttcag tgccaaaaac aacaacaaaa tgaaacgcaa ccaccaccaa ccactgcgac 3120

cacaagaagg atgattcaac aggttttcct gaagaattga aaaccatttt tgctgtccct 3180

tttccttttt tgatgttctt tcaccctttt ctgtttgcta agtgaggatg aaaaaataac 3240

actgtactgc aataagggga gagtaaccct gtctaatgaa acctgtgtct ctgagagtag 3300

agtcactgga acactaatga ggaaactgca ctgttttatt ttaattcagt tgttagtgtg 3360

tcttagtgtg tgcaattttt tttcttacta atatccatgg tttgcaggtt ctgttaggcc 3420

ctttccttct ccttacttct tatccccaac tccctaccca cccctcttca gttttcagat 3480

tggagattca agatttgttc cactttataa gcaagaggaa aaaaaagcaa ccttcaaact 3540

aattctccat gggggctctc catgttaccc tccactcctt ggccccaagc ctttgatgga 3600

gacagacatt gttggagaag tgggctgcct tccccaagtg gggcactgct taagcactta 3660

tttagtggag aacacaggtg aaaagcaact caggatgagg gtggtggaga gggcaggggc 3720

agatgtgcag tcagagaagg actcctgaag ttactgctgc tcagaaaaac agttccttta 3780

atgtggaaga gccatttcat aggtcatagg tggtatggta tatttcttca gagtcaacct 3840

tggccctgag aagtatgtcc tcctggtgtg ctcaggttca acggcagtct ggtggctgaa 3900

ggcacttggc ctcctaaacc aagcagaatt ttgggaagag ttaacagcca gggagatatt 3960

gcccatgatt ctcacttttt ctttgcctgg catctaagca ggaacccatt gtggagtaga 4020

ctctcttctt ctatggagcc tctgacatgg ggagcaatgc taagcaagct aagtgtaaaa 4080

gaaaagtgac agaataattt tggaagagga agcctcatca aaagctcaca caaaatagag 4140

cttcccatgg tgtgccctat cctaggttta agaaaacacg tatgaagttt atgctgatgc 4200

aaagaacttg ggtttttatg ttaatataaa gtgttgtttt agcatgtggc cagataatgc 4260

tctgtcatct ttagaaagtg agatacccaa ggaaataatt gaagaagtat agggagatgg 4320

attaagttga taatgacatt tagggcaact taagaccttt gatcccaggt tctaactcaa 4380

agaggctgac ctttccccag ctaagatagc atgaggacgt tgtattccaa tatacctatg 4440

attggggcta caaagctgaa ctaaagcaag attggtgaag tggcagggtt tatagagaga 4500

agcccaggct gagttcagct tttgttggaa gtgagaatcc ctgacatata gctttcttgg 4560

agatcccaac tctcattctt ggtgcaactg gcttccagct ctccagcagt cactctccta 4620

ggtgcatgat tcagtgcgtg ccatgtgtca ttagctttta ctgataaccg tactctggct 4680

tgttccctta ccccctactt ctatccagtt ttctctgcta ggggttatca ttagcaattc 4740

acatgctaaa ggttttggag cctacctagg ggtaggtgca gctttattgg cttttctgtg 4800

gattctctca gtggacccac accatctcta tgtctctcca ctctcctgcc ttcagccata 4860

gcaaagaatc cttccaaaat caaactcttc acttttttga ctcaagtgtt gttgttcagt 4920

ctctcgcgtg tcaatgtggg catggttcat gaaaccggac cctcaagatg gatgattgct 4980

tttaactact gccagctgat gtctctcagc ccctgccctc acacaagatt tttctcagcc 5040

ttcagcctac cactgcggaa tccgatgtga cccaccatta gggagtctgc atcttggaag 5100

agttggaaat aaccctttaa catcaacatg cttcaaagac tttttgcctt tggcctggta 5160

agacgcctct ccagctactg agcccacaag taacatgagc ggataaaaag agacttgttt 5220

gtgctagaaa tgagggtcta tgctatgagg gggtccaaga ctctggcgaa atatgctttt 5280

tcatcaatgg agaaatgaaa ggaaaacaaa agcaagaaaa aagttaactt gtattatgta 5340

tttttactac acttttctta aaaatagagc attgggaaaa ctctgaaaga gactgacatt 5400

tttctcaaca ggaatccata cttaacagtt ctggctttca ttaaattttg ctctttggta 5460

cctgggcctt ttatttaaca tctatatttg ttttaactct cttggcagat gtgtgaaagg 5520

attcttgctt gatcaaacac taagtttttt tttggttcct gtttttcttt caaatagcca 5580

ggtttttttt cttttggtat ttgcataaaa tgaaaatatc accgaatatt aaatcactgt 5640

ggatccat 5648

<210> 21
<211> 5753
<212> DNA
<213> Pan troglodytes

<400> 21
gcgagtcgcg cacgcgcgcc cgggactgcc tgcccctctc tgtgacttgc cggtgtgtgt 60

gcgtgtgtgt atgtgtgtgt gtgtgcgcgc gcgcgtgagt gagagaggag agagggagaa 120

gagagcgcga gagagggtga gtgtgtgtga gtgcatggga gggtgctgaa tattccgaga 180

cactgggacc acagcggcag ctccgctgaa aactgcattc agccagtcct ccggacttct 240

ggagcgggga cagggcgcag ggcatcagca gccaccagca ggacctggga aatagggatt 300

cttctgcctc cacttcaggt tttagcagct tggtgctaaa ttgctgtctc aaaatgcaga 360

ggatctaatt tgcagaggaa aacagccaaa gaaggaagag gaggaaaagg aaaaaaaaag 420

gggtatattg tggatgctct acttttcttg gaaatgcaaa agattatgca tatttctgtc 480

ctcctttctc ctgttttatg ggactgattt tttggtgtct cttctaacag catacagata 540

ggggggctat ttcctagggg cgccgatcaa gaatacagtg catttcgagt agggatggtt 600

cagttttcca cttcggagtt cagactgaca ccccacatcg acaatttgga ggtggcaaac 660

agcttcgcag tcactaatgc tttctgctcc cagttttcga gaggagtcta tgctattttt 720

ggattttatg acaagaagtc tgtaaatacc atcacatcat tttgcggaac actccacgtc 780

tccttcatca ctcccagctt cccaacagat ggcacacatc catttgtcat tcagatgaga 840

cccgacctca aaggagcact ccttagcttg attgaatact atcaatggga caagtttgca 900

tacctctatg acagtgacag aggcttatca acactgcaag ctgtgctgga ttctgctgct 960

gaaaagaaat ggcaagtgac tgctatcaat gtgggaaaca ttaacaatga caagaaagat 1020

gagatgtacc gatcactttt tcaagatctg gagttaaaaa aggaacggcg tgtaattctg 1080

gactgtgaaa gggataaagt aaacgacatt gtagaccagg ttattaccat tggaaaacat 1140

gttaaagggt accactacat cattgcaaat ctgggattta ctgatggaga cctattaaaa 1200

atccagtttg gaggtgcaaa tgtctctgga tttcagatag tggactatga tgattcgttg 1260

gtatctaaat ttatagaaag atggtcaaca ctggaagaaa aagaataccc tggagctcac 1320

acaacgacaa ttaagtatac ttctgctctg acctatgatg ccgttcaagt gatgactgaa 1380

gccttccgca acctaaggaa gcaaagaatt gaaatctccc gaagggggaa tgcaggagac 1440

tgtctggcga acccagcagt gccctgggga caaggtgtag aaatagaaag ggccctcaaa 1500

caggttcagg ttgaaggtct ctcaggaaat ataaagtttg accagaatgg aaaaagaata 1560

aactatacaa ttaacatcat ggagctcaaa actaatgggc cccggaagat tggctactgg 1620

agtgaagtgg acaaaatggt tgttactctt actgagctcc cttctggaaa tgacacctct 1680

gggcttgaga ataagactgt tgttgtcacc acaattttgg aatctccgta tgttatgatg 1740

aagaaaaatc atgaaatgct tgaaggcaat gagcgctatg agggctactg tgttgacctg 1800

gctgcagaaa tcgccaaaca ttgtgggttc aagtacaagt tgacaattgt tggtgatggc 1860

aagtatgggg ccagggatgc agacacgaaa atttggaatg ggatggttgg agaacttgta 1920

tatggaaagg ctgatattgc aattgctcca ttaactatta cccttgtgag agaagaggtg 1980

attgacttct caaagccctt catgagcctc gggatatcta tcatgatcaa gaagcctcag 2040

aagtccaaac caggagtgtt ttcctttctt gatcctttag cctatgagat ctggatgtgc 2100

attgtttttg cctacattgg ggtcagtgta gttttattcc tggtcagcag atttagcccc 2160

tacgagtggc acactgagga gtttgaagat ggaagagaaa cacaaagtag tgaatcaact 2220

aatgaatttg ggatttttaa tagtctctgg ttttccttgg gtgcctttat gcagcaagga 2280

tgcgatattt cgccaagatc cctctctggg cgcattgttg gaggtgtgtg gtggttcttt 2340

accctgatca taatctcctc ctacacggct aacttagctg ccttcctgac tgtagagagg 2400

atggtgtctc ccatcgaaag tgctgaggat ctttctaagc aaacagaaat tgcttatgga 2460

acattagact ctggctccac taaagagttt ttcaggagat ctaaaattgc ggtgtttgat 2520

aaaatgtgga cctacatgcg gagtgcggaa ccctctgtgt tcgtgaggac tacggccgaa 2580

ggggtggcta gagtgcggaa gtccaaaggg aaatatgcct acttgttgga gtccacgatg 2640

aacgagtaca ttgagcaaag aaagccttgc gacaccatga aagttggtgg aaacctggat 2700

tccaaaggct atggcatcgc aacacctaaa ggatcctcat taagaaatgc ggttaacctc 2760

gcagtactaa aactgaatga acaaggcctg ttggacaaat tgaaaaacaa atggtggtac 2820

gacaaaggag agtgcggcag cgggggaggt gattccaagg tcagccccat aaatcttgca 2880

gtattgaaac tcagtgagca aggcgtctta gacaagctga aaaacaaatg gtggtacgat 2940

aaaggtgaat gtggagccaa ggactctgga agtaaggaaa agaccagtgc cctcagtctg 3000

agcaacgttg ctggagtatt ctacatcctt gtcgggggcc ttggtttggc aatgctggtg 3060

gctttgattg agttctgtta caagtcaagg gccgaggcga aacgaatgaa gatgaccttg 3120

aatgatgcca tgaggaacaa ggcaaggctg tcaattacag gaagtactgg agaaaatgga 3180

tgtgttatga ctccagaatt tcccaaagca gtgcatgctg tcccttacgt gagtcctggc 3240

atgggaatga atgtcagtgt gactgatctc tcgtgattga taagaacctt ttgagtgcct 3300

tacacaatgg ttttcttgtg tgtttattgt caaagtggtg agaggcatcc agtatcttga 3360

agacttttct ttcagccaag aattcttaaa tatgtggagt tcatcttgaa ttgtaaggaa 3420

tgattaatta aaacacaaca tctttttcta ctcgagttac agacaaagcg tggtggacat 3480

gcacagctaa catggaagta ctataattta cctgaagtct ttgtacagac atcaaacctg 3540

tttctgcagc cactattgtt agtctcttga ttcataatga cttaagcaca cttgacatca 3600

actgcatcaa gatgtgacat gttttataaa aaaaggaaaa aaaaacattt aaaactaaaa 3660

aatattttta ggtattttca caaacaaact ggcttttaaa taaatttgct tccatattgg 3720

ttgaataaga caaaaacaat taaactgagt gggaagtgaa taaaaaaagg ctttaggtat 3780

cggttccata tttttcaaag ccaaatatgt aaatgctaag gaaagtaaac aaagaggaga 3840

ttccaatctt gtaatttaat attgttatta aaactttaat gtatcctatt ctttaacatt 3900

tggtgttaat ataaaattac ttggcaatgc ttgacatttg aaataaacat ttttctattg 3960

ttttattgca agtggtccaa ttaattttgc ttagctacag tttggtcata aatcaagtga 4020

gtttaaagac actaccaagt tgttaggtgc ccagagaaaa tttctccctt ttaaaaaggc 4080

caggtgattt ttcaaatgta atcttgcccc caaagtaata tctgaatatg tttttgacat 4140

gtctaaatat atatatatat aaagaaatat ttgttaacac aaaagcattt gatctatgta 4200

gataaatgct aatagattta aaaagctaat attaacaaat accagaatat gtgaagttcc 4260

atttttaaag tgtttgagct tacagaagag aaacattcat tttaaatgaa gtaaaaaatg 4320

ccttgaaagt aattctttag atagttgccc attgattaaa ttccaaaaac tgaatatgtt 4380

tttagcttta aaattataaa agctgtcata aactttatat attatgaatt ttaaaatatg 4440

tttgagtcac ctgcaatata gtttcatccc attgacatca attaaaaata accctaatat 4500

attattttta tatttattcc tcaggtggaa tggctatttt aatatgccca gtgtggataa 4560

aatgtcacat ttctgtaact tttgactaaa gagcctatat atatctagtt aatgaattta 4620

aaggatctat ctttcccttc ataaaatacc tcttatttcc attaaagccc cccaagttta 4680

attaatttag gattttgaat gattattgac atccaatagt tatttttaat atttgtattc 4740

ttgttatttc tggaagaaag cctttgtgta gcacttggta ttttgcaaag tgcttttaaa 4800

acattcttac ttaccgtatt tcatagaagg gaaggaaaaa tgtaaggttt aacagtaacc 4860

acttgcattg aacttggagg catgtggtat catgatattc ttcactaaat ttagctgtcc 4920

ctaatcacag atcctaaggt aatataatat aattttagtg catttctcct catcaggaat 4980

gctggaggtg cattttaagt tttaataata agtgctagaa tgaccaaatt gcagactaat 5040

tgtttccata ttgtacttaa aatgagtttt taaaaatgaa aaagaaatga ctatatacaa 5100

tcaatgctat ttattgtacc tctgggccta ctcttctaaa aattgtagct tatcgatttt 5160

tctctgtcaa gcttgaacta atgtaaataa ttgaaataat gtaaagttat attttcatgt 5220

ttttatagat acaacatgac aagaatacat aatgtaagag tatttcaact atggataatg 5280

ttgattggat aatgcacatc tcagttacaa gcagtactca tagtttaata tccatgtaac 5340

ggtgcatcaa tatattgcta tataaatatg tctgtgtgca tataagtgaa aagtggtcaa 5400

acaagagtga tgacagctgt ctaaaggttt ttttattcat tttatataaa aactgttatg 5460

gaaagaccaa aatgtttatg aactattctt atgtaaattt acaattgtcc tttactgtac 5520

tttttttgttt acagtatagt accttatttt ctgctgtgtt aagtgggtgt caaactccaa 5580

gaagacatac actttctata acttctattg aagatattgg aatttccaat ttttcatgtg 5640

tactatgtca gaaaatgctt tcgattttat ttttaaatct aacatcggat ggcttttccg 5700

gagtgttgta aaaacttcaa tcatacataa aacatgttct tacaaaaggc aaa 5753

<210> 22
<211> 2990
<212> DNA
<213> Pan troglodytes

<220>
<221> misc_feature
<222> (2549)..(2549)
<223> n is a, c, g, or t

<220>

<221>  misc_feature
<222>  (2581)..(2581)
<223>  n is a, c, g, or t

<400>  22
gagggggtgt gagagatcct gggagcgaga gggagagaga gggagcaaga aaggaagaga      60

gagcgagcga gagagagcga gcgaataaga gagagagtaa gagagagaga gaagaagagg     120

aagaagagga ggcggcggca gcggaggagg aggaggacta gtgtggggtg gaaaggaaga     180

gtgagcgaga gcaagttaag gggagggggt gtaagagcca gcgaattctt tttctttttc     240

tattattatt ttgacgactc ctgagttgcg cccatgctct tgtcagcttc gttttaggcg     300

tagcatggcc aggcagaaga aaatggggca aagcgtgctc cgggcggtct tctttttagt     360

cctggggctt ttgggtcatt ctcacggagg attccccaac accatcagca taggtggact     420

tttcatgaga aacacagtgc aggagcacag cgctttccgc tttgccgtgc agttatacaa     480

caccaaccag aacaccaccg agaagccctt ccatttgaat taccacgtag atcacttgga     540

ttcctccaat agtttttccg tgacaaatgc tttctgctcc cagttctcga gaggggtgta     600

tgccatcttt ggattctatg accagatgtc aatgaacacc ctgacctcct tctgtggggc     660

cctgcacaca tcctttgtta cgcctagctt ccccactgac gcagatgtgc agtttgtcat     720

ccagatgcgc ccagccttga agggcgctat tctgagtctt ctgggtcatt acaagtggga     780

gaagtttgtg tacctctatg acacagaacg aggattttcc atcctccaag cgattatgga     840

agcagcagtg caaaacaact ggcaagtaac agcaaggtct gtgggaaaca taaaggacgt     900

ccaagaattc aggcgcatca ttgaagaaat ggacaggagg caggaaaagc gatacttgat     960

tgactgcgaa gtcgaaagga ttaacacaat tttggaacag gttgtgatcc tagggaaaca    1020

ctcaagaggt tatcactaca tgctcgctaa cctgggtttt actgatattt tactggaaag    1080

agtcatgcat gggggagcca acattacagg tttccagatt gtcaacaatg aaaaccctat    1140

ggttcagcag ttcatacagc gctgggtgag gctggatgaa agggaattcc ctgaagccaa    1200

gaatgcacca ctaaagtata catctgcatt gacacacgac gcaatactgg tcatagcaga    1260

agctttccgc tacctgagga ggcagcgagt agatgtgtcc cggagaggaa gtgctggaga    1320

ctgcttagca aatcctgctg tgccctggag tcaaggaatt gatattgaga gagctctgaa    1380

aatggtgcaa gtacaaggaa tgactggaaa tattcaattt gacacttatg gacgtaggac    1440

aaattatacc atcgatgtgt atgaaatgaa agtcagtggc tctcgaaaag ctggctactg    1500

gaatgagtat gaaaggtttg tgcctttctc agatcagcaa atcagcaatg acagtgcatc    1560

ctcagagaat cggaccatag tagtgactac cattctggaa tcaccatatg taatgtacaa    1620

gaagaaccat gagcaactgg aaggaaatga acgatatgaa ggctattgtg tagacctagc 1680

ctatgaaata gccaaacatg taaggatcaa atacaaattg tccatcgttg gtgacgggaa 1740

atatggtgca agggatccag agactaaaat atggaacggc atggttgggg aacttgtcta 1800

tgggagagct gatatagctg ttgctccact cactataaca ttggtccgtg aagaagtcat 1860

agatttttca aagccattca tgagcctggg catctccatc atgataaaga agcctcagaa 1920

atcaaaacca ggcgtattct catttctgga tcccctggct tatgaaatct ggatgtgcat 1980

tgtctttgct tacattggag tcagcgtagt tcttttccta gtcagcagat tcagtcctta 2040

tgaatggcac ttggaagaca acaatgaaga acttcgtgac ccacaaagtc ctcctgatcc 2100

tccaaatgaa tttggaatat ttaacagtct ttggttttcc ttgggtgcct ttatgcagca 2160

aggatgtgat atttctccaa gatcactctc cgggcgcatt gttggagggg tttggtggtt 2220

cttcaccctg atcataattt cttcctatac tgccaatctc gctgctttcc tgactgtgga 2280

gaggatggtt tctcccatag agagtgctga agacttagct aaacagactg aaattgcata 2340

tgggaccctg gactccggtt caacaaaaga atttttcaga agatccaaaa ttgctgtgta 2400

cgagaaaatg tggtcttaca tgaaatcagc ggagccatct gtgtttacca aaacaacagc 2460

agacggagtg gcccgagtgc gaaagtccaa gggaaagttc gccttcctgc tggagtcaac 2520

catgaatgag tacattgagc agagaaaanc catgtgatac gatgaaagtt ggggagaatc 2580

nggagagtgc ggcagcgggg gcggtgactc caaggtcagc ctcaatgaga acgcctgtaa 2640

accttgcagt attgaaactc agtgaacaag gcatcttaga caagctgaaa aacaaatggt 2700

ggtacgataa gggggaatgt ggagccaagg actccgggag taaggacaag accagcgctc 2760

tgagcctgag caatgtggca ggcgttttct atatacttgt cggaggtctg gggctggcca 2820

tgatggtggc tttgatagaa ttctgttaca aatcacgggc agagtccaaa cgcatgaaac 2880

tcacaaagaa cacccaaaac tttaagcctg ctcctgccac caacactcag aattatgcta 2940

catacagaga aggctacaac gtgtatggaa cagagagtgt taagatctag 2990

<210> 23
<211> 3193
<212> DNA
<213> Pan troglodytes

<220>
<221> misc_feature
<222> (13)..(13)
<223> n is a, c, g, or t

<400> 23

atgggagcct ttncgagcag cgtgcaaata ggtggtctct tcatccgaaa cacagatcag 60

gaatacactg cttttcgatt agcaattttt cttcataaca ccagccccaa tgcgtcggaa 120

gctcctttta atttggtacc tcatgtggac aacattgaga cagccaacag ttttgctgta 180

acaaacgcct tctgttccca gtattctaga ggagtatttg ccatttttgg actctatgat 240

aagaggtcgg tacatacctt gacctcattc tgcagcgcct tacatatctc cctcatcaca 300

ccaagtttcc ctactgaggg ggagagccag tttgtgctgc aactaagacc ttcgttacga 360

ggagcactct tgagtttgct ggatcactac gaatggaact gttttgtctt cctgtatgac 420

acagacaggg gatactcgat actccaagct attatggaaa aagcaggaca aaatggttgg 480

catgtcagcg ctatatgtgt ggaaaatttt aatgatgtca gctataggca acttctagaa 540

gaacttgata gaagacaaga gaagaagttt gtaatagact gtgagataga gagacttcaa 600

aacatattag aacagattgt aagtgttgga aagcatgtta aaggctacca ttatatcatt 660

gcaaacttgg gattcaagga tatttctctt gagaggttta tacatggtgg agccaatgtt 720

actggattcc agttggtgga ttttaataca cctatggtaa tcaaactaat ggatcgctgg 780

aagaaactag atcagagaga gtatccagga tctgagactc ctccaaagta cacctctgct 840

ctgacttatg atggagtcct tgtgatggct gaaactttcc gaagtcttag gaggcagaaa 900

attgatatct caaggagagg aaatgctggg gattgtctgg caaatcctgc tgctccatgg 960

ggccagggaa ttgacatgga gaggacactc aaacagataa atttcaatga ccaaaagaga 1020

aagaagatgg atggtaagat tattattagg ctgagaggga gagaaacatc taagttgttc 1080

aggaaggtgc tgagaaagaa tagtggaaaa agaaagctgg ttggttactg gaatgatatg 1140

gataagttag tcttgattca agatgtacca actcttggca atgacacagc tgctattgag 1200

aacagaacag tggttgtaac cacaattatg gaatccccat atgttatgta caagaaaaat 1260

catgaaatgt ttgaaggaaa tgacaagtat gaaggatact gtgtagattt ggcatctgaa 1320

attgcaaaac acattggtat caagtataaa attgccattg tccctgatgg aaaatatgga 1380

gcaagggatg cagacacaaa aatctggaat gggatggtag gagaacttgt ttatgggaaa 1440

gcagagattg ctattgcccc tctgacaatc actttggtac gagaggaggt cattgacttt 1500

tctaagccct tcatgagttt gggcatatct atcatgatca aaaagcctca gaaatccaaa 1560

ccaggagtgt tttccttctt ggatcctctg gcctatgaga tttggatgtg catagtcttt 1620

gcctacattg gtgtcagcgt ggtcttattc ctagttagta gatttagtcc atatgagtgg 1680

catacagaag agccagagga tggaaaggaa ggacccagcg accagcctcc caatgagttt 1740

ggcatcttta acagcctctg gttttccctg ggtgctttta tgcagcaagg atgtgacatt 1800

tcacccagat ccctctcagg tcgaattgtt ggaggtgttt ggtggttctt tacactcatc 1860

attatatcat cttatactgc taacctcgct gctttcctga cggttgagcg aatggtctct 1920

cccatagaaa gtgcagaaga cctagccaaa caaacagaaa ttgcctatgg aacattggat 1980

tcagggtcaa caaaagaatt cttcagaaga tcaaaaatag cagtgtatga aaagatgtgg 2040

acctacatgc gatcagcaga gccatcagtg ttcactagga ctacagctga gggagtagct 2100

cgtgtccgca aatccaaggg caaatttgcc tttctcctgg agtccactat gaatgaatac 2160

attgagcagc gaaagccatg tgacacgatg aaagtgggag gaaatctgga ttccaaaggc 2220

tatggagtag caacgcccaa gggttcctca ttaagaaatg ctgttaacct cgcagtttta 2280

aaactgaatg aacaaggcct cttggacaaa ttgaaaaaca aatggtggta cgacaaagga 2340

gaatgtggca gcgggggagg tgactccaag gacaagacga gtgccttgag cctgagcaat 2400

gtagcaggcg tcttctacat tctggttggc ggcttgggct tggcaatgct ggtggctttg 2460

atagagttct gttacaagtc cagggcagaa gcgaagagaa tgaagctgac cttttctgaa 2520

gccataagaa acaaagccag attatccatc actgggagtg tgggagagaa tggccgcgtc 2580

ttgacgcctg actgcccaaa ggctgtacac actggaactg caatcagaca aagttcagga 2640

ttggctgtca ttgcatcgga cctaccataa aaaccaaaaa aaaaattgag tgccttaatt 2700

aaactgttgg tgactggtgg aaacgcagcc ctgagggaca cgccacgcgc gggtctttgc 2760

taaaccaatc ctttggctga gagcgggaag tccgtcctaa cgcgctggcc ggacatcagc 2820

agcagcaacg tgtgcatgag ctcagctcgg aaacccaaac tcagatttta tatcaggaaa 2880

actcacaatt gaggtttttt tcggggagtg ggtgggggag ggatctggga tgggtgtatt 2940

aacagcaaca aatttcattc gagtggactc aaaaactaat cagacttgtg agttagcgca 3000

ttaaactgtg aagttcttgc tcagaaaggc ctttgtcttc accggaaagg ataaaatagt 3060

tgtagaagtc cgtgaacatg ctaacctgtg tctccagaac atccatgtag tccatggaag 3120

aaaatccagc tgagaaaaca aatcactaaa ctgtgataag aaaataatga acaaacatgt 3180

aaaactctgt ggg 3193

<210> 24
<211> 64
<212> DNA
<213> Artificial Sequence

<220>
<223> GluR2 siRNA1

<400> 24

gatccccgga gcactcctta gcttgattca agagatcaag ctaaggagtg ctcctttttg        60

gaaa                                                                  64


<210>  25
<211>  63
<212>  DNA
<213>  Artificial Sequence

<220>
<223>  GluR2 siRNA 2

<400>  25
agcttttcca aaaaggagca ctcctagctt gatctcttga atcaagctaa ggagtgctcc        60

ggg                                                                   63


<210>  26
<211>  12
<212>  PRT
<213>  Artificial Sequence

<220>
<223>  GluR1(Ser831) rat

<400>  26

Leu Ile Pro Gln Gln Ser Ile Asn Glu Ala Ile Lys
1               5                   10


<210>  27
<211>  12
<212>  PRT
<213>  Artificial Sequence

<220>
<223>  GluR1(Ser831) rat2

<400>  27

Leu Ile Pro Gln Gln Ser Ile Asn Glu Ala Ile Lys
1               5                   10


<210>  28
<211>  21
<212>  PRT
<213>  Artificial Sequence

<220>
<223>  GluR4 rat

<400>  28

Lys His Thr Gly Thr Ala Ile Arg Gln Ser Ser Gly Leu Ala Val Ile
1               5                   10                  15

Ala Ser Asp Leu Pro
            20


<210>  29
<211>  26
<212>  PRT
<213>  Artificial Sequence

<220>
<223>  Conantokin-R


<220>
<221>  MOD_RES
<222>  (3)..(4)
<223>  Carboxylation

<220>
<221>  MOD_RES
<222>  (11)..(11)
<223>  Carboxylation

<220>
<221>  MOD_RES
<222>  (15)..(15)
<223>  Carboxylation

<220>
<221>  DISULFID
<222>  (21)..(25)

<400>  29

Gly Glu Glu Glu Val Ala Lys Met Ala Ala Glu Leu Ala Glu Asn Ile
1               5                   10                  15


Ala Leu Gly Cys Leu Val Asn Cys Tyr Pro
            20                  25


<210>  30
<211>  21
<212>  PRT
<213>  Artificial Sequence

<220>
<223>  Conantokin-T


<220>
<221>  MOD_RES
<222>  (3)..(4)
<223>  carboxylation

<220>

<221>  MOD_RES
<222>  (10)..(10)
<223>  carboxylation

<220>
<221>  MOD_RES
<222>  (14)..(14)
<223>  carboxylation

<400>  30

Gly Glu Glu Glu Tyr Gln Lys Met Leu Glu Asn Leu Arg Glu Ala Glu
1               5                   10                  15

Val Lys Lys Asn Ala
            20

## Description

### FIELD OF THE INVENTION

The invention relates to the field of Alzheimer disease, more precisely to compounds for the treatment of Alzheimer disease.

### BACKGROUND OF THE INVENTION

Amyloid diseases or amyloidoses include a number of disease states having a wide variety of outward symptoms. These disorders have in common the presence of abnormal extracellular deposits of protein fibrils, known as "amyloid fibrils", "amyloid deposits" or "amyloid plaques" that are usually about 10-100 nm in diameter and are localized to specific organs or tissue regions. Such plaques are composed primarily of a naturally occurring soluble protein or peptide. These insoluble deposits are composed of generally lateral aggregates of fibrils that are approximately 10-15 nm in diameter. Though diverse in their occurrence, all amyloid deposits have common morphologic properties, stain with specific dyes (e.g. Thioflavin T, Congo red), and have a characteristic red-green birefringent appearance in polarized light after staining.

Amyloid-related diseases are characterized by the type of protein present in the deposit. For example, neurodegenerative diseases such as scrapie, bovine spongiform encephalitis, Creutzfeldt-Jakob disease and the like are characterized by the appearance and accumulation of a protease-resistant form of a prion protein (referred to as AScr or PrP-27) in the central nervous system. Similarly, Alzheimer's disease, another neurodegenerative disorder, is characterized by the deposition of amyloid plaques and neurofibrillary tangles. In this case, the plaque and blood vessel amyloid is formed by the deposition of fibrillar amyloid beta protein. Other diseases such as adult-onset diabetes (Type 11 diabetes) are characterized by the localized accumulation of amyloid in the pancreas.

Each amyloidogenic protein has the ability to fold into beta-sheets and to form insoluble fibrils, which get deposited extracellularly or intracellularly. Each amyloidogenic protein, although different in amino acid sequence, has the same property of forming fibrils and binding to other elements such as proteoglycan, amyloid P and complement component. Moreover, each amyloidogenic protein has amino acid sequences, which, although different, can catalyze the formation of beta-sheet cells. As per example, the amyloid beta fibrils have been associated with dead neuronal cells and microgliosis in patients with Alzheimer's disease. When tested in vitro, the amyloid beta peptide was shown to be capable of triggering an activation process of microglia (brain macrophages), which would explain the presence of microgliosis and brain inflammation found in the brain of patients with Alzheimer's disease.

In another type of amyloidosis seen in patients with Type II diabetes, the amyloidogenic protein IAPP has been shown to induce beta-islet cell toxicity in vitro. Hence, appearance of IAPP fibrils in the pancreas of Type II diabetic patients could contribute to the loss of the beta islet cells (Langerhans) and organ dysfunction.

One of the most prominent amyloid diseases is Alzheimer's disease, which is a progressive neurodegenerative disease affecting approximately 0.5-1% of the total population in the western world. Alzheimer's disease is characterized by the deposition of large numbers of amyloid plaques in the brain.

The molecular mechanisms responsible for the development of the idiopathic cases of Alzheimer disease (AD) are unknown. Growing evidence indicates that cerebral elevation and accumulation of Aβ peptide mediate many aspects of AD pathogenesis. The main constituent of the amyloid plaques is the amyloid beta peptide (Aβ), a 40-42 amino-acid protein that is produced through cleavage of the amyloid precursor protein (APP). This deposition is assumed to cause the pathology of the disease by different ways and most approaches to prevent Alzheimer's disease is aimed at reducing, removing, or preventing the formation of amyloid plaques or to inhibiting the down-stream path ways activated by this amyloid plaques.

However there is still the need of further molecules for the treatment of Alzheimer's disease.

### SUMMARY OF THE INVENTION

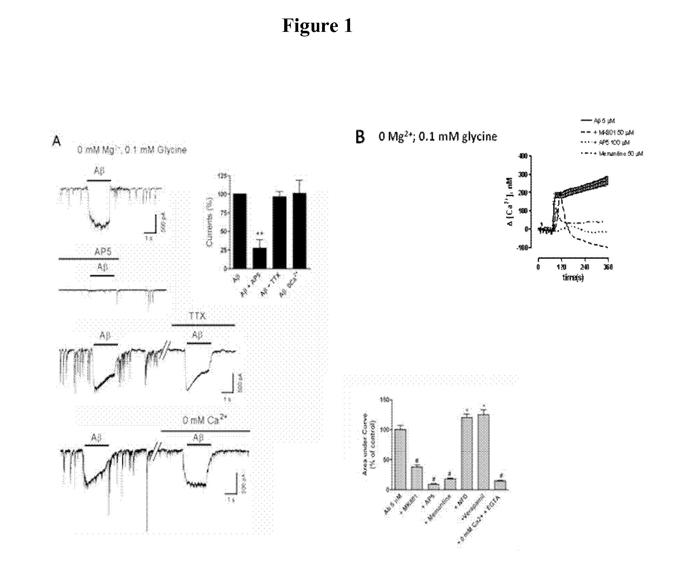

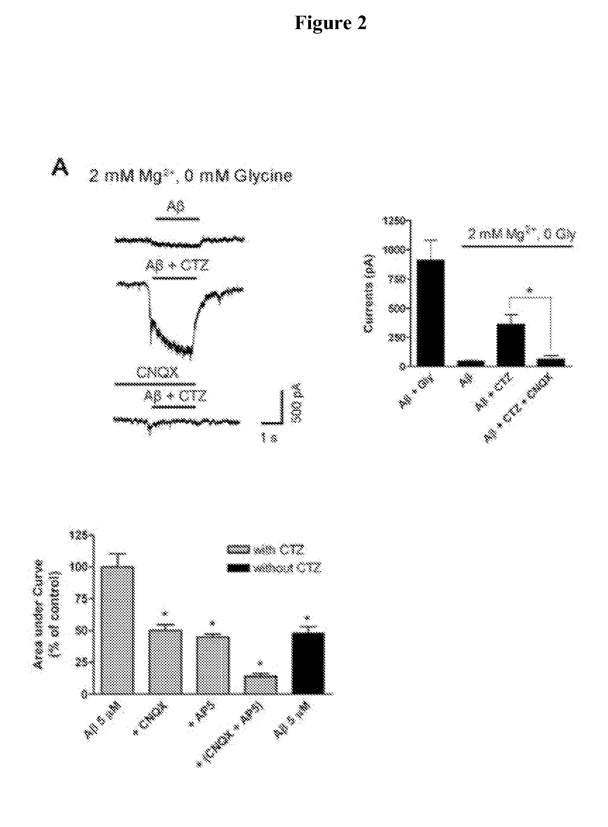

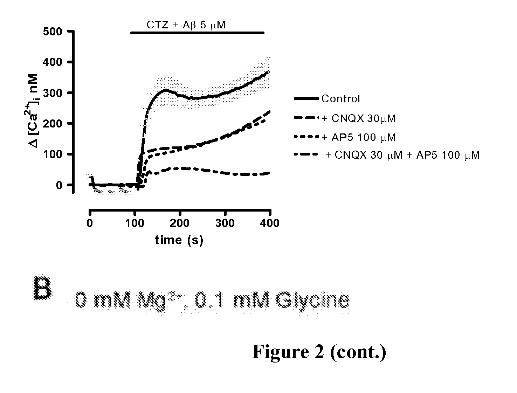

The inventors have shown that Aβ oligomers generate glutamate-independent inward currents, dysregulate Ca²⁺ homeostasis and induce cell death through both NMDA and AMPA receptors in cultured neurons or in enthorinal cortex-hippocampus organotypic slices (Figure 1). In this apoptotic neuronal death caspase-dependent and independent pathways are involved. The inventors have surprisingly seen that Aβ induced changes of [Ca²⁺]¡ and provoke mitochondrial Ca²⁺ overload, mitochondrial membrane depolarization and ROS generation. Moreover the inventors had surprisingly seen that antagonists of NMDA and AMPA receptor reduce deleterious events in mitochondria, indicating that AMPA receptors initiate the Aβ-induced Ca²⁺ dysregulation in neurons (Figures 2-5). These data indicate that Aβ induced Ca²⁺ dysregulation in neurons during amyloid protein deposition associated diseases and point to the AMPA receptors as a therapeutic target to limit tissue damage in said diseases.

Thus, in an aspect, the invention relates to an AMPA receptor inhibitor for use in the treatment of a disease associated with deposition of amyloid proteins.

In another aspect, the invention relates to a method of inhibiting Ca²⁺-triggered central nervous system cell death which comprises contacting central nervous system cells with an AMPA receptor inhibitor under conditions effective to inhibit Ca²⁺⁻triggered central nervous system cell death, wherein the inhibitor inhibits the Ca²⁺ flow through the AMPA receptor.

In another aspect, the invention relates to NMDA receptor inhibitor for use in the treatment of a disease associated with deposition of amyloid proteins.

In another aspect, the invention relates with a method for identifying a compound useful for inhibiting the cellular death induced by amyloid proteins deposition for the treatment of a disease associated with deposition of amyloid proteins comprising:
a) putting in contact a cell with an amyloid protein,;
b) putting the cell resulting from step a) with the test compound; and
c) determining the levels of Ca²⁺ and/or the levels of AMPA receptor activation in said cell ,
wherein if the levels of Ca²⁺ and /or the levels of AMPA receptor activation in said cell after the treatment of said cell with the test compound are lower than before the treatment, the test compound is able to inhibiting the cellular death induced by amyloid proteins deposition and useful for the treatment of a disease associated with deposition of amyloid proteins.

### BRIEF DESCRIPTION OF THE FIGURES

**Figure 1****.** Aβ oligomers activate NMDA receptors and induce Ca²⁺ influx into neurons. (A) Aβ (1 µM) evokes inward, non-desensitizing currents when applied together with glycine in Mg²⁺-free medium. Aβ-activated currents are blocked by AP5 (100 µM), a selective NMDA receptor antagonist. TTX (1 µM) and calcium-free extracellular bath solution do not attenuate Aβ-induced inward currents. (B) Aβ (5 µM) induces a rapid increase in [Ca²⁺]¡, an effect that is blocked by NMDA receptor antagonists, MK801, AP5 and memantine, or by removal of Ca²⁺ from the perfusate. Voltage-gated Ca²⁺ channel inhibitors nifedipine and verapamil do not diminish Aβ response.
**Figure 2****.** Aβ oligomers activate AMPA receptors and increase [Ca²⁺]¡ in neurons. (A) Aβ (1 µM) together with CTZ (100 µM) evokes inward, non-desensitizing currents in the presence of Mg²⁺. Currents are blocked by the AMPA/kainate receptor antagonist CNQX (30 µM). (B) Aβ in conjunction with CTZ induces [Ca²⁺]¡ increase, that is partially blocked by CNQX and AP5, and further reduced by both antagonists applied together.

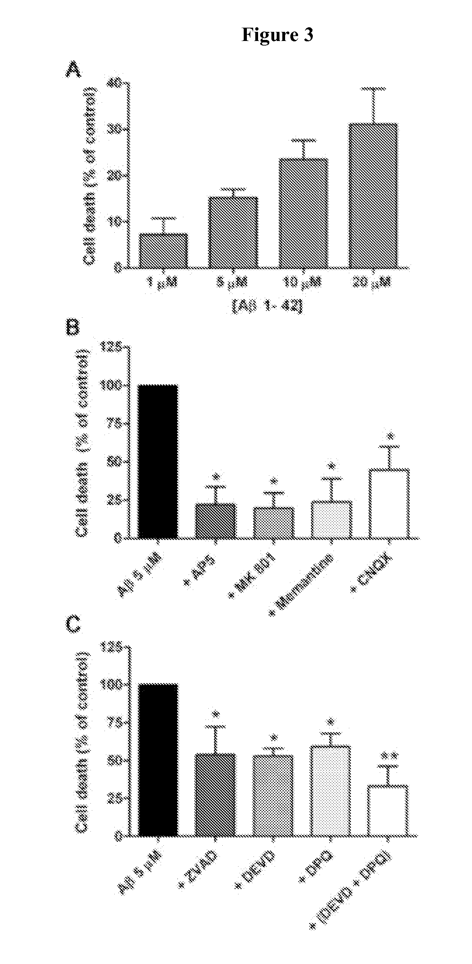

**Figure 3****.** Aβ oligomers trigger neuronal apoptotic death by activating NMDA and AMPA/kainate receptors. (A) Dose-response toxicity of Aβ oligomers in cortical neurons in culture as measured 24 h later with the LDH viability assay. (B) Toxicity is prevented by co-application of Aβ oligomers (5 µM) with NMDA receptor antagonists AP5 (100 µM), MK801 (50 µM) and memantine (50 µM), and with AMPA/kainate receptor antagonist CNQX (30 µM). (C) Aβ induced-toxicity was reduced by the broad-spectrum caspase-inhibitor ZVAD (50 µM), by caspase-3 inhibitor DEVD (100 µM) and by PARP-1 inhibitor DPQ (30 µM). (* p< 0.05, ** p < 0.01 for each comparison between Aβ alone and the co-applied with GluR antagonist or the corresponding inhibitor).

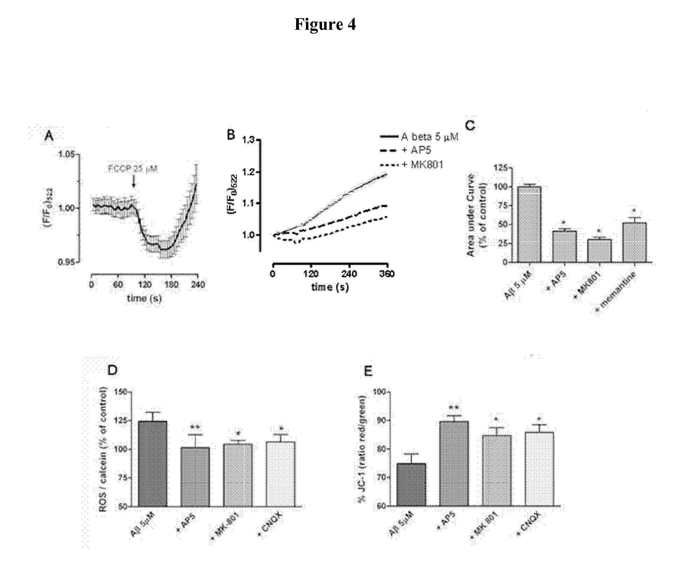

**Figure 4****.** Blockade of glutamate receptors attenuates Aβ-induced mitochondrial alterations. Neurons, loaded with Rhod-2 AM, were exposed to (A) FCCP (25 µM) used as an internal control to release Ca²⁺ from mitochondria, or to (B and C) Aβ alone (5 µM) or together with NMDA receptor antagonists. (B and C) [Ca²⁺]ₘᵢₜ induced by Aβ is reduced by NMDA receptor antagonists. Recordings in (B) illustrate average ± SEM responses of 74 cells from at least 5 experiments, and (C) histogram represents average ± SEM of area under Ca²⁺ curve for each condition. (D and E) Cultures were first exposed to Aβ alone or in conjunction with GluR antagonists for 2 hours, and cells were immediately loaded with the corresponding dyes to monitor ROS generation (D) and mitochondrial depolarization (E) by fluorimetry. NMDA and AMPA/kainate receptor antagonists prevent the increase in radical oxygen species and mitochondrial depolarization produced by Aβ. * p < 0.05, ** p < 0.01 for each antagonist versus Aβ.

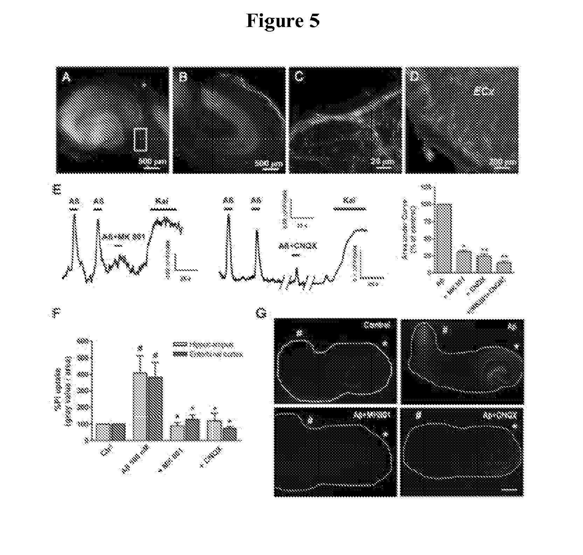

**Figure 5****.** MK801 and CNQX reduce Aβ-induced [Ca²⁺]_{¡} rises and abolish cell death in entorhinal cortex-hippocampus organotypic cultures. Cultures were fixed at 7 DIV and characterized by immunofluorescence with antibodies to neurofilaments (NFL) and cholinergic terminals (ChAT). (A, B) Hippocampus and entorhinal cortex showed profuse staining with NFL antibody. (C) Square shows the magnification in (A) of neuronal fibers from entorhinal cortex entering in CA1. (D) Cholinergic fibers revealed with ChAT in the entorhinal cortex (ECx) at high magnification. (E) Aβ (20 µM in the pipette) induces a rapid increase in [Ca²⁺]_{¡} in hippocampus, an effect that is blocked by NMDA receptor antagonists MK801 (50 µM; n=9) and by AMPA receptor antagonist CNQX (30 µM; n=43) and further reduced by both antagonists applied together (n=50). Kainate (Kai) 1 mM also increases [Ca²⁺]_{¡} in the same selected neurons. (F and G) Histogram and representative fields showing Aβ (100 nM, 4 days) toxicity in cultures treated after 7 DIV and protection when oligomers are applied in conjunction with MK801 (10 µM) or CNQX (30 µM). Scale bar in (G) represents 500 µm. (F) Bars (gray value/area) represent neuronal cell death occurs in Aβ-treated hippocampus and entorhinal cortex. MK801 and CNQX mainly blocked the cell damage in both areas. Bars in (F) represent the average ± SEM from 5 experiments. # < 0.05 for comparison Aβ vs control treatments. * p < 0.05 for comparison of Aβ vs antagonist treatment.

### DETAILED DESCRIPTION OF THE INVENTION

### THERAPEUTIC METHODS OF THE INVENTION

The authors of the present invention have surprisingly found that Aβ oligomers activates NMDA and AMPA receptor in neurons and that this activation causes an increase in basal [Ca²⁺]_{¡} levels. Moreover, the inventors have shown that Aβ induces mitochondrial Ca²⁺ overload and that this effect can be prevented by NMDA receptors antagonists.

This finding opens the possibility for improved therapies for diseases associated with deposition of amyloid proteins such Alzheimer by using AMPA receptor inhibitors.

Thus, in a first aspect, the invention relates to an AMPA receptor inhibitor for use in the treatment of a disease associated with deposition of amyloid proteins.

Although a variety of subjects can be treated, it is particularly preferred that the subject be a human being.

"AMPA receptors" or "AMPARs", as used herein, refers to proteins that are members of the ionotropic class of glutamate receptors. AMPA (α-amino-3-hydroxy-5-methyl-4-isoxazole proprionic acid). Th e α-amino-3-hydroxy-5-methyl-4-isoxazolepropionic acid receptor (also known as AMPA receptor, AMPAR, or quisqualate receptor) is a non-NMDA-type ionotropic transmembrane receptor for glutamate that mediates fast synaptic transmission in the central nervous system (CNS). Its name is derived from its ability to be activated by the artificial glutamate analog, AMPA.

AMPARs are composed of four types of subunits, designated as GluR1 (GRIA1), GluR2 (GRIA2), GluR3 (GRIA3), and GluR4, alternatively called GluRA-D2 (GRIA4), which combine to form tetramers (Shi S.H. et al. Science 284 (5421): 1811-6; Song I. et al. Trends Neurosci. 25 (11): 578-88). Most AMPARs are heterotetrameric, consisting of symmetric 'dimer of dimers' of GluR2 and either GluR1, GluR3 or GluR4. (Mayer, M. L. 2005. 15 (3), 282-288; Greger IH, et al. 2007. Trends Neurosci. 30 (8): 407-16).

AMPARs subunits differ most in their c-terminal sequence, which determines their interactions with scaffolding proteins. All AMPARs contain PDZ-binding domains, but which PDZ domain they bind to differs. For example, GluR1 binds to SAP97 through SAP97's class I PDZ domain (Leonard AS, et al. J. Biol. 1998. Chem. 273 (31): 19518-24), while GluR2 binds to PICK1 (Greger IH, et al. 2002. Neuron 34 (5): 759-72) and GRIP/ABP. Of note, AMPARs cannot directly bind to the common synaptic protein PSD-95 due to incompatible PDZ domains.

Phosphorylation of AMPARs can regulate channel localization, conductance, and open probability. GluRl has four known phosphorylation sites at serine 818 (S818), S831, threonine 840, and S845 (other subunits have similar phosphorylation sites, but GluR1 has been the most extensively studied). S818 is phosphorylated by PKC, and is necessary for long term potentiation (LTP; for GluR1's role in LTP, see below). (Boehm J. et al. 2006 Neuron 51 (2): 213-25). S831 is phosphorylated by CaMKII during LTP, which helps deliver GluR1-containing AMPAR to the synapse, (Hayashi Y. et al., 2000. Science 287 (5461): 2262-7) and increases their single channel conductance. The T840 site was more recently discovered, and has been implicated in LTD. Finally, S845 is phosphorylated by both PKA which regulates its open probability (Delgado JY, 2007. J. Neurosci. 27 (48): 13210-21).

In humans different genes coding for the different AMPAR subunits exist: GRIA1 (transcript variant 1: NM_000827.3 (SEQ ID NO:1), transcript variant 2: NM_001114183.1 (SEQ ID NO:2)) GRIA2 (transcript variant 1: NM_001083619.1 (SEQ ID NO:3), transcript variant 2: NM_000826.3 (SEQ ID NO:4), transcript variant 3: NM_001083620.1 (SEQ ID NO:5)) GRIA3 (transcript variant 1: NM_000828.4 (SEQ ID NO:6), transcript variant 2: NM_007325.4 (SEQ ID NO:7)) GRIA4 (transcript variant 1: NM_000829.3 (SEQ ID NO:8), transcript variant 2: NM_001077243.2 (SEQ ID NO:9), transcript variant 3: NM_001077244.1 (SEQ ID NO:10), transcript variant 4: NM_001112812.1 (SEQ ID NO:11)).

In mice: Gria1 (NP_032191.2 (SEQ ID NO:12)), Gria2 (NP_038568.2, (SEQ ID NO:13)), Gria 3 (NM_016886.2, (SEQ ID NO:14)), Gria4 (NM_019691.4, (SEQ ID NO:15)).

In Rat: Gria1 (NM_031608.1, (SEQ ID NO:16)), Gria2 (NM_017261, (SEQ ID NO:17)), Gria3 (NM_032990.2, (SEQ ID NO:18)), Gria4 (NM_017263.2, (SEQ ID NO:19)).

In chimpanzee: Gria1 (XP_527092.2, (SEQ ID NO:20)), Gria2 (XP_001141570.1, (SEQ ID NO:21)), Gria3 (XR_023006.1, (SEQ ID NO:22)), Gria4 (XP_522168.2, (SEQ ID NO:23)).

Each AMPAR has four sites to which an agonist (such as glutamate) can bind, one for each subunit (Mayer, M. L. 2005. Current Opinion in Neurobiology, 15 (3), 282-288). The binding site is believed to be formed by the n-tail, and the extracellular loop between transmembrane domains three and four (Armstrong N, 1998. Nature 395 (6705): 913-7). When an agonist binds, these two loops move towards each other, opening the pore. The channel opens when two sites are occupied, and increases its current as more binding sites are occupied. Once open, the channel may undergo rapid desensitization, stopping the current. The mechanism of desensitization is believed to be due to a small change in angle of one of the parts of the binding site, closing the pore. AMPARs open and close quickly, and are thus responsible for most of the fast excitatory synaptic transmission in the central nervous system.

In another aspect, the invention relates to a method for the treatment of a disease associated with deposition of amyloid proteins comprising the administration to a subject in need thereof of a composition comprising a therapeutical effective amount of an AMPA receptor inhibitor.

As used herein, the terms "treat", "treatment" and "treating" refer to the amelioration of one or more symptoms associated with a disease that results from the administration of a therapeutically effective amount of the composition of the invention or a pharmaceutical preparation comprising thereof, to a subject in need of said treatment. Thus, "treatment" as used herein covers any treatment of a disease, disorder or condition of a mammal, particularly a human, and includes: (a) preventing the disease or condition from occurring in a subject which may be predisposed to the disease or condition but has not yet been diagnosed as having it; (b) inhibiting the disease or condition, i.e., arresting its development; or (c) relieving the disease or condition, i.e., causing regression of the disease or condition or amelioration of one or more symptoms of the disease or condition. The population of subjects treated by the method includes a subject suffering from the undesirable condition or disease, as well as subjects at risk for development of the condition or disease. Thus, one of skill in the art realizes that a treatment may improve the patient's condition, but may not be a complete cure of the disease. As used herein, the terms "disorder" and "disease" are used interchangeably to refer to an abnormal or pathological condition in a subject that impairs bodily functions and can be deadly.

The phrase "therapeutically effective amount" is used herein to mean an amount sufficient to prevent, and preferably reduce by at least about 25 percent, more preferably by at least 50 percent, most preferably by at least 90 percent, a clinically significant change in a feature of pathology. As related to the present invention, the term may also mean an amount sufficient to ameliorate or reverse one or more symptoms associated with a disease associated with deposition of amyloid proteins, such as Alzheimer's disease. In particular, a "therapeutically effective amount" of the treatment may result in amelioration, reduction or elimination of at least one of the following symptoms: memory impairment, persistent sadness or anxiety, feelings of emptiness, hopelessness, pessimism, guilt, worthlessness, helplessness, a loss of interest or pleasure in hobbies and activities that were once enjoyed, decreased energy, or fatigue, difficulty concentrating, remembering, or making decisions, insomnia, early-morning awakening, or oversleeping, appetite and/or weight loss or overeating and weight gain, thoughts of death or suicide and suicide attempts, restlessness, irritability, and persistent physical symptoms that do not respond to treatment, such as headaches, digestive disorders, and chronic pain. When applied to an individual active ingredient, administered alone, the term refers to that ingredient alone. When applied to a combination, the term refers to combined amounts of the active ingredients that result in the therapeutic effect, whether administered in combination, serially or simultaneously.

The term "subject" refers to an animal, preferably a mammal including a non-primate (e.g. a cow, pig, horse, cat, dog, rat, or mouse) and a primate (e.g. a monkey or a human). In a preferred embodiment, the subject is a dog. In a more preferred embodiment, the subject is a primate which, in a still more preferred embodiment, said primate is a human.

In the context of the present invention, "a disease associated with deposition of amyloid proteins" includes diseases associated with the accumulation of amyloid protein which can either be restricted to one organ, i.e. "localized amyloidosis" or spread to several organs, which is denoted "systemic amyloidosis". Diseases associated with deposition of amyloid proteins that can be treated with the compositions according to the present invention include, without limitation, the disease shown below.

| Disease | Featured protein |
|---|---|
| Alzheimer's disease | Beta amyloid |
| Inclusion body myositis (IBM), | Beta amyloid |
| Type 2 diabetes mellitus | IAPP (Amylin) |

| | |
|---|---|
| Parkinson's disease | Alpha-synuclein |
| Transmissible spongiform encephalopathy (Mad Cow Disease) | Prion |
| Huntington's Disease | Huntingtin |
| Medullary carcinoma of the thyroid | Calcitonin |
| Cardiac arrhythmias | Atrial natriuretic factor |
| Atherosclerosis | Apolipoprotein AI |
| Rheumatoid arthritis | Serum amyloid A |
| Aortic medial amyloid | Medin |
| Prolactinomas | Prolactin |
| Familial amyloid polyneuropathy | Transthyretin |
| Hereditary non-neuropathic systemic amyloidosis | Lysozyme |
| Dialysis related amyloidosis | β2-Microglobulin |
| Finnish amyloidosis | Gelsolin |
| Lattice corneal dystrophy | Keratoepithelin |
| Cerebral amyloid angiopathy | Beta amyloid |
| Cerebral amyloid angiopathy (Icelandic type) | Cystatin |
| Systemic AL amyloidosis | Immunoglobulin light chain AL |

In a preferred embodiment, the disease is a disease associated with deposition of beta amyloid such as Alzheimer, Inclusion body myositis (IBM), cerebral amyloid angiopathy, loss of memory, hereditary cerebral haemorrhage Dutch-type, diabetes II, Parkinson disease, Down's Syndrome, Pick disease, multiple systemic atrophy (ASM), progressive supracraneal paralysis, multiple sclerosis, ischemia, cerebral lesion, schizophrenia, etc.

The term "amyloid beta peptide" is used herein interchangeably with Abeta, amyloid beta protein, "A beta", "beta AP", "A beta peptide" or "Aβ peptide" or "Aβ" and refers to a family of peptides that are the principal chemical constituent of the senile plaques and vascular amyloid deposits (amyloid angiopathy) found in the brain in patients of Alzheimer's disease (AD), Down's Syndrome, and Hereditary Cerebral Hemorrhage with Amyloidosis of the Dutch-Type (HCHWA-D), etc. In whatever form, amyloid beta peptide is a fragment of beta-amyloid precursor protein (APP) which comprises a variable number of amino acids, typically 39-43 amino acids. "Aβ(1-42)", as used herein, relates to a 42 amino acids peptide corresponding to amino acids 672 to 713 of APP and which is produced by the sequential proteolytic cleavage of the amyloid precursor protein by the β- and γ-secretases.

### AMPA receptor inhibitors

"AMPA receptor inhibitors", as used herein, relates to any compound capable of causing a decrease in the AMPA hemichannel activity, including those compounds which prevent expression of the AMPA hemichannel gene, leading to reduced AMPA mRNA or protein levels as well as compounds that inhibit AMPA causing a decrease in the cations (Ca^{2+,} Na⁺, K⁺, etc) flow through the channel. In a particular embodiment, the AMPA receptor inhibitor is an AMPA blocker or antagonist. An "AMPA blocker or antagonist" as used herein relates to any compound capable of binding to the AMPA molecule, either inside of the pore or at other part of the receptor important for the receptor function such as the phosphorylation sites described previously. The binding of the compound would then block the flow of the cations through the AMPA receptor. If the blocker acts as an antagonist, the compound would blocks or dampen an agonist-mediated response. The antagonists binding will disrupt the interaction and inhibit the function of an agonist or inverse agonist at receptors. Antagonists mediate their effects by binding to the active site or to allosteric sites on receptors, or they may interact at unique binding sites not normally involved in the biological regulation of the receptor's activity. Antagonist activity may be reversible or irreversible depending on the longevity of the antagonist-receptor complex, which, in turn, depends on the nature of antagonist receptor binding.

Compounds leading to the inhibition of an AMPA receptor can be identified using standard assays such as methods for measuring the cation (e.g. Ca²⁺) flow through the channel, using standard electrophysiology methods and receptor-binding assays as described in Matzen et al. (Matzen L. et al. 1997. J. Med. Chem., 40 (4), pp 520-527) and Hajnóczky et al. (G. Hajnóczky, et al. 1995. Cell 82 415-424), or in this specification (see Example, "Methods", "Electrophysiology"). The mitochondrial and cytosolic concentration of the cations can be also measured using, for example, fluorescent indicators for said cations, such as the Ca²⁺ dyes described by Grynkiewicz et al. (G. Grynkiewicz, et al. 1985. J. Biol. Chem. 260, 3440-3450) or in this specification (see methods, "Ca²⁺ imaging in the cytosol" and "Measurement of mitochondrial Ca^{2+,,}).

The determination of the inhibiting or blocking capacities of the compound on the biological activity of AMPA receptor can be detected using the above described standard assays.

The blocking capacities can be expressed as the affinity of the inhibitor, the potency of the inhibitor or the specificity of said inhibitor.

The affinity of a drug is calculated using the equilibrium dissociation constant of the receptor-drug complex, KD. KD is the ratio of rate constants for the reverse (k2) and forward (k1) reaction between the drug and receptor and the drug-receptor complex. KD is also the drug concentration at which receptor occupancy is half of maximum. Drugs with a high KD (low affinity) dissociate rapidly from receptors; conversely, drugs with a low KD (high affinity) dissociate slowly from receptors. The affinity of a drug for a receptor describes how avidly the drug binds to the receptor (ie, the KD). The affinity can be measured using equilibrium binding techniques such as the ones described by in Kessler et al. (Kessler M et al., Molecular Pharmacology January 1996 vol. 49 no. 1:123-131).

In a preferred embodiment, the AMPA receptor inhibitor has a KD between of between 0.001 nM and 10 µM, preferably between 0.01 µM and 1 µM, preferably between 0.01 nM and 100 nM, preferably between 0.1 nM and 10 nM.

Potency refers to the concentration (EC50) or dose (ED50) of a drug required to produce 50% of the drug's maximal effect. (http://www.ncgc.nih.gov/guidance/section3.html). EC50 equals KD when there is a linear relationship between occupancy and response. Often, signal amplification occurs between receptor occupancy and response, which results in the EC50 for response being much less (i.e., positioned to the left on the abscissa of the log dose-response curve) than KD for receptor occupancy. Potency depends on both the affinity of a drug for its receptor, and the efficiency with which drug-receptor interaction is coupled to response. The dose of drug required to produce an effect is inversely related to potency. In general, low potency is important only if it results in a need to administer the drug in large doses that are impractical. The ED50 for a quantal dose-response relationship is the dose at which 50% of individuals exhibit the specified quantal effect. In a preferred embodiment, the AMPA receptor inhibitor has a IC50 of between 0.001 nM and 10 µM, preferably between 0.01 nM and 1 µM, preferably between 0.1nM and 100 nM, preferably between 0.1 nM and 10 nM.

Efficacy (also referred to as intrinsic activity) of a drug is the ability of the drug to elicit a response when it binds to the receptor. In some tissues, agonists demonstrating high efficacy can result in a maximal effect, even when only a small fraction of the receptors are occupied. Efficacy is not linked to potency of a drug.

The median inhibitory concentration or IC50 is the concentration of an antagonist that reduces a specified response to 50% of the maximal possible effect.

The higher specificity of a compound for a define molecule refers to the ability of the compound for inhibiting the define molecule in a substantially higher manner in respect to the ability of the compound for inhibiting other molecule.

In a preferred embodiment, the inhibitor is a selective AMPA receptor inhibitor.

The language "selective AMPA receptor inhibitor" refers to a compound capable of inhibiting the activity of AMPA receptor while not substantially inhibiting the activity of at least one receptor form the same family (such as NMDA receptor) or a molecule from a different family at the same compound concentration For example, a selective AMPA receptor inhibitor may have an AMPA receptor inhibitory activity 2, 5, 10, 20, 50, 100, 500, or 1000-fold more potent than its NMDA inhibitory activity. In certain embodiments, the AMPA receptor inhibitory activity of a selective AMPA receptor inhibitor is at least 5-fold, 10-fold, or 20-fold more potent than the inhibitory activity for other molecule such as NMDA of the compound.

Exemplary, non-limitative, AMPA receptor inhibitors that can be used in the treatment of a disease associated with deposition of amyloid proteins, according to the instant invention are described under 1 to 21 in Table I or in the patent US2004049039A.

**Table I**

| | |
|---|---|
| **AMPA receptor inhibitors** | |

| | Compound |
|---|---|
| 1 | CFM-2 |
| | 1-(4'-Aminophenyl)-3,5-dihydro-7,8-dimethoxy-4H-2,3-ben zodiazepin-4-one |
| | 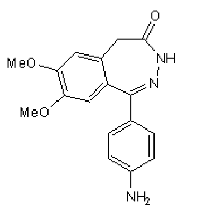 |
| 2 | CNQX |
| | 6-Cyano-7-nitroquinoxaline-2,3-dione |
| | 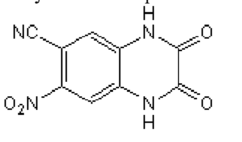 |
| 3 | CNQX disodium salt |
| | 6-Cyano-7-nitroquinoxaline-2,3-dione disodium |
| | 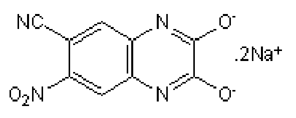 |
| 4 | CP 465022 hydrochloride |
| | 3-(2-Chlorophenyl)-2-[2-[6-[(diethylamino)methyl]-2-pyridinyl]ethenyl]-6-fluoro-4(3H)-quinazolinone hydrochloride |
| | 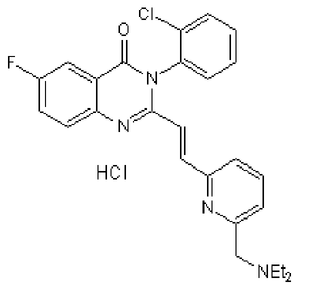 |
| 5 | DNQX |
| | 6,7-Dinitroquinoxaline-2,3-dione |
| | 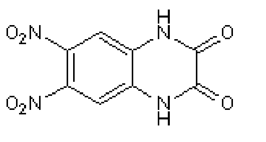 |
| 6 | DNQX disodium salt |
| | 6,7-Dinitroquinoxaline-2,3-dione disodium salt |
| | 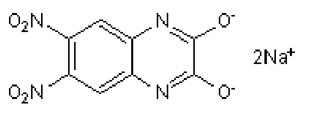 |
| 7 | Evans Blue tetrasodium salt |
| | 6,6-[(3,3'-Dimethyl[1,1'-biphenyl]-4,4'-diyl)bis(azo)bi s[4-amino- 5-hydroxy-1,3-naphthalenedisulphonic acid] tetrasodium salt. |
| | 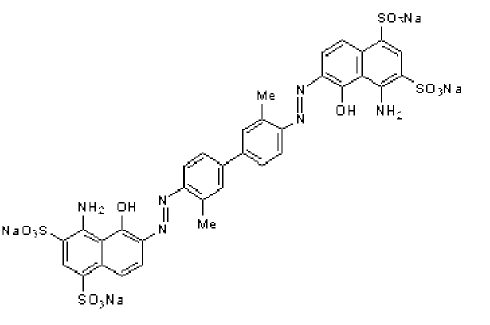 |
| 8 | GYKI 47261 dihydrochloride |
| | 4-(8-Chloro-2-methyl-11H-imidazo[1,2-c][2,3]benzodiazep in-6-benzeneamine dihydrochloride |
| | 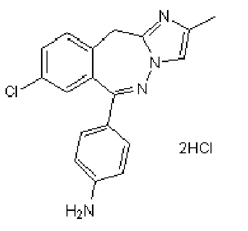 |
| 9 | NBQX |
| | 2,3-Dioxo-6-nitro-1,2,3,4-tetrahydrobenzo[f]quinoxaline -7-sulfonamide and 6,7-Dinitroquinoxaline-2,3-dione disodium salt |
| | 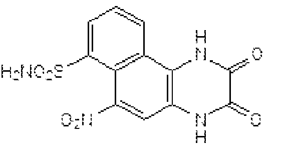 |
| | 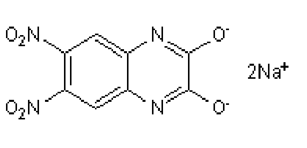 |
| 10 | Philanthotoxin 74 or PhTx-74 |
| | (S)-N-[7-[(4-Aminobutyl)amino]heptyl]-4-hydroxy-a-[(1-o xobutyl)amino]benzenepropanamide dihydrochloride |
| | 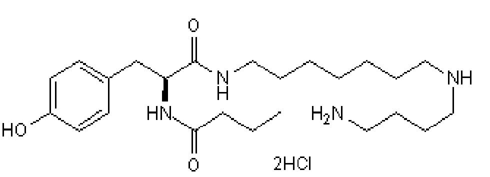 |
| 11 | SYM 2206 |
| | (±)-4-(4-Aminophenyl)-1,2-dihydro-1-methyl-2-propylcarb amoyl-6,7-methylenedioxyphthalazine |
| | 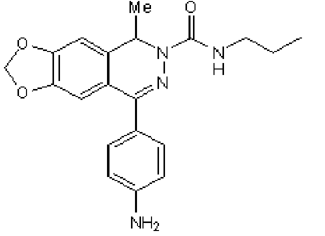 |
| 12 | UBP 282 or 3-CBW |
| | (aS)-a-Amino-3-[(4-carboxyphenyl)methyl]-3,4-dihydro-2, 4-dioxo-1(2H)-pyrimidinepropanoic acid |
| | 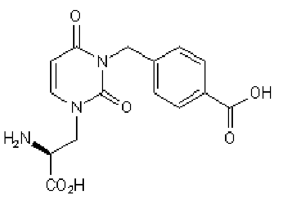 |
| 13 | YM 90K hydrochloride |
| | 1,4-Dihydro-6-(1H-imidazol-1-yl)-7-nitro-2,3-quinoxalin edione hydrochloride |
| | 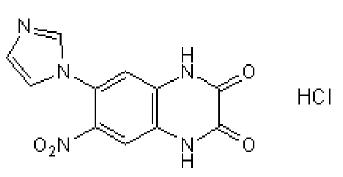 |
| 14 | ZK 200775 (MPQX) |
| | [[3,4-Dihydro-7-(4-morphofinyl)-2,3-dioxo-6-(trifluorom ethyl)-1(2H)-quinoxafinyl]methyl]phosphonic acid |
| | 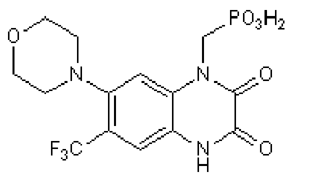 |
| 15 | LY 303070 |
| | 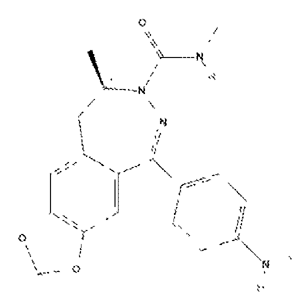 |
| 16 | LY300164 |
| | 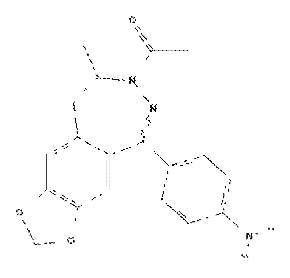 |
| 17 | An inhibitory antibody capable of specifically binding and inhibiting the activity of an AMPA receptor. |
| 18 | A ribozyme or a DNA enzyme specific for the sequence of an AMPAR. |
| 19 | An interfering RNA specific for the sequence of an AMPA receptor. |
| 20 | An inhibiting peptide specific for an AMPA receptor. |
| 21 | An AMPA receptor - specific antisense nucleic acid |

### AMPA receptor inhibitory antibodies

Antibodies against an epitope located in either the pore or an external part of the AMPA molecule may effectively inhibit the function of this protein and, therefore, can be used as blocker or inhibitor in the compositions of the present invention. Thus, "Inhibitory antibody", as used herein, refers to antibodies which are capable of inhibiting at least partially the biological activities of the AMPA receptor.

The determination of the inhibiting capacity on the biological activity of the AMPA receptor is detected using standard assays to measure the cation released through the AMPA receptor using methods described before.

Inhibiting antibodies or fragments specific for an AMPA receptor may be readily available, or may be readily produced using conventional molecular biology techniques. For example, using immunogens derived from, for example, AMPA receptor molecule it is possible to obtain anti-protein/anti-peptide antisera or monoclonal antibodies by using standard protocols (See, for example, Antibodies: A Laboratory Manual ed. by Harlow and Lane (Cold Spring Harbor Press: 1988)). A mammal, such as a mouse, a hamster or rabbit can be immunized with an immunogenic form of the peptide (e.g., AMPA receptor or an antigenic fragment thereof, which is capable of eliciting an antibody response). Techniques for conferring immunogenicity on a protein or peptide, include conjugation to carriers or other techniques, are well known in the art. An immunogenic portion of a polypeptide can be administered in the presence of adjuvant. The progress of immunization can be monitored by detection of antibody titers in plasma or serum. Standard ELISA or other immunoassays can be used with the immunogen as antigen to assess the levels of antibodies. In a preferred embodiment, the antibodies forming part of the compositions of the invention are immuno-specific for antigenic determinants of AMPA receptor (or a variant at least 80%, 85%, 90%, 95%, or 98% identical thereto). In certain embodiment, the immunospecific subject antibodies do not substantially cross react with a non-vertebrate (such as yeast) AMPA receptor related protein. By "not substantially cross react", it is meant that the antibody has a binding affinity for a non-homologous protein which is at least one order of magnitude, more preferably at least 2 orders of magnitude, and even more preferably at least 3 orders of magnitude less than the binding affinity of the antibody for a AMPA receptor.

Thus, the antibody which can be used for the purposes of the instant invention as a AMPA receptor inhibitory antibody, hereinafter referred to as the "antibody of the invention", is capable of binding an epitope of the AMPA receptor; typically, at least 6, 8, 10, or 12, contiguous amino acids are required to form an epitope, however, epitopes which involve non-contiguous amino acids may require more, e.g., at least 15, 25, or 50 amino acid. The term "antibody of the invention" includes, for example, polyclonal antibodies, monoclonal antibodies, Fab and single chain Fv (scFv) fragments thereof, bispecific antibodies, heteroconjugates, human and humanized antibodies. Such antibodies may be produced in a variety of ways, including hybridoma cultures, recombinant expression in bacteria or mammalian cell cultures, and recombinant expression in transgenic animals. Also antibodies can be produced by selecting a sequence from a library of sequences expressed in display systems such as filamentous phage, bacterial, yeast or ribosome. There is abundant guidance in the literature for selecting a particular production methodology, e.g., Chadd and Chamow, Curr. Opin. Biotechnol., 12:188-194 (2001). The choice of manufacturing methodology depends on several factors including the antibody structure desired, the importance of carbohydrate moieties on the antibodies, ease of culturing and purification, and cost. Many different antibody structures may be generated using standard expression technology, including full-length antibodies, antibody fragments, such as Fab and Fv fragments, as well as chimeric antibodies comprising components from different species. Antibody fragments of small size, such as Fab and Fv fragments, having no effector functions and limited pharmokinetic activity may be generated in a bacterial expression system. Single chain Fv fragments show low immunogenicity and are cleared rapidly from the blood.

The antibodies of the invention may be polyclonal antibodies. Such polyclonal antibodies can be produced in a mammal, such as a non-human mammal, for example, following one or more injections of an immunizing agent, and preferably, an adjuvant. Typically, the immunizing agent and/or adjuvant will be injected into the mammal by a series of subcutaneous or intraperitoneal injections. The immunizing agent may include AMPA receptors or fragments thereof or a fusion protein thereof or a cell expressing AMPA receptors. Such proteins, fragments or preparations are introduced into the non-human mammal in the presence of an appropriate adjuvant. Other form of administration of an immunogen is as a trasmembrane protein in the surface of a cell (methods described in, e.g., Spiller et al. J. Immunol. Methods, 224: 51-60 (1999)). These cells can be either cells which naturally express the antigen or in which this expression can be obtained after transfecting the cell with a DNA construct that contains among other DNA sequences those coding the antigen, those necessary for its sufficient expression in the cell. This approach is possible not only when the cell membrane is the natural site in which the antigen is expressed even the antigen once synthesized in the cell is directed at these location by a signal peptide which is added at the antigen coding sequence. If the serum contains polyclonal antibodies to undesired epitopes, the polyclonal antibodies can be purified by immunoaffinity chromatography.

Alternatively, said antibodies may be monoclonal antibodies. Monoclonal antibodies may be produced by hybridomas, wherein a mouse, hamster, or other appropriate host animal, is immunized with an immunizing agent to elicit lymphocytes that produce or are capable of producing antibodies that will specifically bind to the immunizing agent, e.g. Kohler and Milstein, Nature 256:495 (1975). The immunizing agent will typically include a AMPA receptor or a fragment thereof or a fusion protein thereof and optionally a carrier or a crude protein preparation which has been enriched for a AMPA receptor or a cell expressing any of said proteins. Such proteins, fragments or preparations are introduced into the non-human mammal in the presence of an appropriate adjuvant. Other form of administration of an immunogen is as a trasmembrane protein in the surface of a cell (methods described in, e.g., Spiller et al. J. Immunol. Methods, 224: 51-60 (1999)). These cells can be everyone which naturally express the antigen in its cell membrane or in which this expression can be obtained after transfecting the cell with a DNA construct that contains among other DNA sequences those coding the antigen, those necessary for its sufficient expression in the cell. This approach is possible not only when the cell membrane is the natural site in which the antigen is expressed even the antigen once synthesized in the cell is directed at these location by a signal peptide which is added at the antigen coding sequence. Alternatively, lymphocytes may be immunized in vitro. Generally, spleen cells or lymph node cells are used if non-human mammalian sources are desired, or peripheral blood lymphocytes ("PBLs") are used if cells of human origin are desired. The lymphocytes are fused with an immortalized cell line using a suitable fusing agent, such as polyethylene glycol, to produce a hybridoma cell. In general, immortalized cell lines are myeloma cells of rat, mouse, bovine or human origin. The hybridoma cells are cultured in a suitable culture medium that preferably contains one or more substances that inhibit the growth or survival of unfused, immortalized cells. Clones are isolated using the limiting dilution method and the culture medium (supernatant) in which the hybridoma cells are cultured can be assayed for the presence of monoclonal antibodies directed against AMPA receptors by conventional techniques, such as by flow cytometry or by immunoprecipitation or by other *in vitro* binding assay, such as RIA or ELISA. Clones can also be cultured *in vivo* as ascites tumours in an animal.

Preferably, the binding specificity of monoclonal antibodies produced by a clone of hybridoma cells is determined by immunoprecipitation or by an in vitro binding assay, such as radioimmunoassay (RIA) or enzyme-linked immunoabsorbent assay (ELISA) or by immunofluorescent techniques such as fluorescence microscopy or flow cytometry. The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in US 4,816,567. DNA encoding the monoclonal antibodies of the invention can be isolated from a AMPA receptor-specific hybridoma cells and sequenced by using conventional procedures, e.g., by using oligonucleotide probes that are capable of binding specifically to genes encoding the heavy and light chains of murine antibodies. The hybridoma cells serve as a preferred source of such DNA. Once isolated, the DNA may be inserted into an expression vector, which is then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for the murine heavy and light chain constant domains for the homologous human sequences, or by covalently joining to the immunoglobulin coding sequence all or part of the coding sequence for a non-immunoglobulin polypeptide. The non-immunoglobulin polypeptide can be substituted for the constant domains of an antibody of the invention, or can be substituted for the variable domains of one antigen-combining site of an antibody of the invention to create a chimeric bivalent antibody.

Another method of generating specific antibodies, or antibody fragments, reactive against a target molecule is to screen expression libraries encoding immunoglobulin genes, or portions thereof, expressed in bacteria, yeast, filamentous phages, ribosomes or ribosomal subunits and other display systems. These methods normally use large libraries of antibody sequences or antibody fragment sequences obtained from diverse sources such healthy donors or patients or animals healthy or not. These sequences are cloned and expressed in an appropriate system and selected by its binding affinity for the antigen. Diverse approaches have been described to select antibodies or fragments with desired properties e.g. neutralizing, agonist, etc (Fernández, Curr. Op. Biotech., 15: 364-373 (2004); Schmidt, Eur. J. Biochem., 268: 1730-1738 (2001)). In an embodiment, antibodies and antibody fragments characteristic of hybridomas of the invention can also be produced by recombinant means by extracting messenger RNA, constructing a cDNA library, and selecting clones which encode segments of the antibody molecule.

The antibodies may also be engineered to alter its clinical uses. Numerous approaches make use of the molecular biology and genetic techniques such as the good knowledge of the genetics ad structure of the immunoglobulins to construct different modifications of immunoglobulin molecule with the aim of improve its properties for clinical or other uses. Some of them tend to reduce the immunogenicity of the molecule in the species in which should be used and the resultant molecule has a sequence more homologous with this species. Various methods have been used to obtain mAbs of human origin avoiding the non ethically admissible proceedings in healthy humans. In other approaches the molecular weigh and size are reduced e.g. in order of improving the distribution of the molecule into solid tumours. Other possibilities are conjugation in a molecule of binding domains for more than one target molecule (bispecific antibody or also triespecific, etc) or the conjugation of an antibody or a fragment with another molecule with the desired function e.g. a toxic agent, a hormone, growth factor, a immunomodulating agent (immunosuppressor or immunostimulator), an inhibitor of cell growth, etc. In general all the resultant molecules retain almost one variable domain of an antibody which gives the high specificity and affinity characteristic of the antigen-antibody binding. Some examples of these constructions are:
- Chimeric antibodies: These refers to antibodies constructed with variable regions from an antibody of some species (normally a mammal in which the mAb was generated) and constant regions of other species (the one in which the chimeric antibody is to be used);
- Humanized antibodies: By "humanized antibody" is meant an antibody derived from a non-human antibody, typically a murine antibody, that retains the antigen-binding properties of the parent antibody, but which is less immunogenic in humans. This may be achieved by various methods, including (a) grafting the entire non-human variable domains onto human constant regions to generate chimeric antibodies; (b) grafting only the non-human complementarity determining regions (CDRs) into human framework and constant regions with or without retention of critical framework residues; and (c) transplanting the entire non-human variable domains, but "cloaking" them with a human-like section by replacement of surface residues. Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Reichmann et al., Nature, 332:323-327 (1988); Verhoeyen et al., Science, 239:1534-1536 (1988)), by substituting hypervariable region sequences for the corresponding sequences of a human antibody. (Carter et al., Proc. Natl. Acad. Sci. USA, 89:4285 (1992); Presta et al., J. Immunol., 151:2623 (1993)). It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences.
- Primatized antibodies: A next step in this approach of making an antibody more similar at humans' are the so called primatized antibodies, i.e. a recombinant antibody which has been engineered to contain the variable heavy and light domains of a monkey (or other primate) antibody, in particular, a cynomolgus monkey antibody, and which contains human constant domain sequences, preferably the human immunoglobulin gamma 1 or gamma 4 constant domain (or PE variant). The preparation of such antibodies is described in Newman et al., Biotechnology, 10: 1458-1460 (1992); US 5,658,570 and US 6,113,898. These antibodies have been reported to exhibit a high degree of homology to human antibodies, i.e., 85-98%, display human effector functions, have reduced immunogenicity, and may exhibit high affinity to human antigens. Another highly efficient means for generating recombinant antibodies is disclosed by Newman, Biotechnology, 10: 1455-1460 (1992).
- Human antibodies: By "human antibody" is meant an antibody containing entirely human light and heavy chain as well as constant regions, produced by any of the known standard methods. As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region PH) gene in chimeric and germ-line mutant mice results in complete inhibition of endogenous antibody production. Transfer of the human germ-line immunoglobulin gene array in such germ line mutant mice will result in the production of human antibodies after immunization. See, e.g., Jakobovits et al., Proc. Mad. Acad. Sci. USA, 90:255 1 (1993); Jakobovits et al., Nature, 362:255-258 (1993). Alternatively, phage display technology (McCafferty et al., Nature 348:552-553 (1990)) can be used to produce human antibodies and antibody fragments in vitro, from immunoglobulin variable (V) domain gene repertoires from donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for their review see, e.g., Johnson, Kevin S. and Chiswell, David J., Current Opinion in Structural Biology 3:564-57 1 (1993). Human antibodies may also be generated by in vitro activated B cells or SCID mice with its immune system reconstituted with human cells. Once obtained a human antibody, its coding DNA sequences can be isolated, cloned and introduced in an appropriate expression system i.e. a cell line preferably from a mammal which subsequently express and liberate into culture media from which the antibody can be isolated.
- Antibody fragments: An antibody fragment is a fragment of an antibody such as, for example, Fab, F(ab')2, Fab' and scFv. Various techniques have been developed for the production of antibody fragments. Traditionally, these fragments were derived via proteolytic digestion of intact antibodies but more recently these fragments can be produced directly by recombinant host cells. In other embodiments, the antibody of choice is a single chain Fv (scFv) fragment which additionally may be monospecific or bispecific. Papain digestion of antibodies produces two identical antigen-binding fragments, called "Fab" fragments, each with a single antigen-binding site, and a residual "Fc" fragment, which name reflects its ability to crystallize readily. Pepsin treatment yields an F(ab')2 fragment that has two antigen-binding sites and is still capable of cross-linking antigen. "Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the VH-VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site. The Fab fragment also contains the constant domain of the light chain and the first constant domain (CHI) of the heavy chain. Fab' fragments differ from Fab fragments by the addition of a few residues at the carboxy terminus of the heavy chain CHI domain including one or more cysteines from the antibody hinge region. Fab'-SH is the designation herein for Fab' in which the cysteine residue(s) of the constant domains bear at least one free thiol group. F(ab')Z antibody fragments originally were produced as pairs of Fab' fragments which have hinge cysteines between them. Other chemical couplings of antibody fragments are also known. "Single-chain Fv" or "scFv" antibody fragments comprise the VH and VL domains of an antibody, wherein these domains are present in a single polypeptide chain. Preferably, the Fv polypeptide further comprises a polypeptide linker between the VH and VL domains which enables the scFv to form the desired structure for antigen binding. For a review of scFv see Pluckthun in The Pharmacology of Monoclonal Antibodies, vol. 113, Rosenburg and Moore eds., Springer-Verlag, N.Y., pp. 269-315 (1994). The term "diabodies" refers to small antibody fragments with two antigen-binding sites, which fragments comprise a heavy-chain variable domain (VH) connected to a light chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).
- Bispecific antibodies: Bispecific antibodies are antibodies that have binding specificities for at least two different epitopes. Methods for making bispecific antibodies are known in the art. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J, 10:3655-3659 (1991).

### AMPA receptor-specific ribozymes

Ribozyme molecules designed to catalytically cleave a target mRNA transcripts can also be used to prevent translation of AMPA receptor mRNAs. Accordingly, in another embodiment, the compositions of the invention comprise ribozymes specifically directed to the AMPA receptor mRNA. Ribozymes are enzymatic RNA molecules capable of catalyzing the specific cleavage of RNA. (For a review, see Rossi, Current Biology 4:469-471, 1994). The mechanism of ribozyme action involves sequence specific hybridization of the ribozyme molecule to complementary target RNA, followed by an endonucleolytic cleavage event. The composition of ribozyme molecules preferably includes one or more sequences complementary to a target mRNA, and the well known catalytic sequence responsible for mRNA cleavage or a functionally equivalent sequence (see, e.g., U.S. Pat. No. 5,093,246, incorporated herein by reference in its entirety).

While ribozymes that cleave mRNA at site specific recognition sequences can be used to destroy target mRNAs, the use of hammerhead ribozymes is preferred. Hammerhead ribozymes cleave mRNAs at locations dictated by flanking regions that form complementary base pairs with the target mRNA. Preferably, the target mRNA has the following sequence of two bases: 5'-UG-3'. The construction and production of hammerhead ribozymes is well known in the art and is described more fully in Haseloff and Gerlach, Nature 334: 585-591, 1988; and see PCT Appln. No. WO89/05852, the contents of which are incorporated herein by reference. Hammerhead ribozyme sequences can be embedded in a stable RNA such as a transfer RNA (tRNA) to increase cleavage efficiency in vivo (Perriman et al., Proc. Natl. Acad. Sci. USA, 92: 6175-79, 1995; de Feyter, and Gaudron, Methods in Molecular Biology, Vol. 74, Chapter 43, "Expressing Ribozymes in Plants", edited by Turner, P. C, Humana Press Inc., Totowa, N.J.). In particular, RNA polymerase III-mediated expression of tRNA fusion ribozymes are well known in the art (see, Kawasaki et al., Nature 393: 284-9, 1998; Kuwabara et al., Nature Biotechnol. 16: 961-5, 1998; and Kuwabara et al., Mol. Cell. 2: 617-27, 1998; Koseki et al., J Virol 73: 1868-77, 1999; Kuwabara et al., Proc Natl Acad Sci USA 96: 1886-91, 1999; Tanabe et al., Nature 406: 473-4, 2000). There are typically a number of potential hammerhead ribozyme cleavage sites within a given target CDNA sequence. Preferably the ribozyme is engineered so that the cleavage recognition site is located near the 5' end of the target mRNA to increase efficiency and minimize the intracellular accumulation of non-functional mRNA transcripts. Furthermore, the use of any cleavage recognition site located in the target sequence encoding different portions of the C-terminal amino acid domains of, for example, long and short forms of target would allow the selective targeting of one or the other form of the target, and thus, have a selective effect on one form of the target gene product.

Gene targeting ribozymes necessarily contain a hybridizing region complementary to two regions, each of at least 5 and preferably each 6, 7, 8, 9, 10, 11, 12, 13, 14, 15, 16, 17, 18, 19 or 20 contiguous nucleotides in length of a target mRNA, such as an mRNA of a sequence represented in the AMPA receptor genes. In addition, ribozymes possess highly specific endoribonuclease activity, which autocatalytically cleaves the target sense mRNA. The present invention extends to ribozymes which hybridize to a sense mRNA encoding a target gene such as a therapeutic drug target candidate gene, thereby hybridizing to the sense mRNA and cleaving it, such that it is no longer capable of being translated to synthesize a functional polypeptide product.

The ribozymes used in the compositions of the present invention also include RNA endoribonucleases (hereinafter "Cech-type ribozymes") such as the one which occurs naturally in Tetrahymena thermophila (known as the IVS, or L-19 IVS RNA) and which has been extensively described by Thomas Cech and collaborators (Zaug et al., Science 224:574-578, 1984; Zaug et al., Science 231: 470-475, 1986; Zaug et al., Nature 324: 429-433, 1986; published International patent application No. WO88/04300 by University Patents Inc.; Been, et al., Cell 47: 207-216, 1986). The Cech-type ribozymes have an eight base pair active site which hybridizes to a target RNA sequence whereafter cleavage of the target RNA takes place. The invention encompasses those Cech-type ribozymes which target eight base-pair active site sequences that are present in a target gene or nucleic acid sequence.

Ribozymes can be composed of modified oligonucleotides (e.g., for improved stability, targeting, etc.) and should be delivered to cells which express the target gene in vivo. A preferred method of delivery involves using a DNA construct "encoding" the ribozyme under the control of a strong constitutive pol III or pol II promoter, so that transfected cells will produce sufficient quantities of the ribozyme to destroy endogenous target messages and inhibit translation. Because ribozymes, unlike antisense molecules, are catalytic, a lower intracellular concentration is required for efficiency.

In certain embodiments, a ribozyme may be designed by first identifying a sequence portion sufficient to cause effective knockdown by RNAi. The same sequence portion may then be incorporated into a ribozyme. In this aspect of the invention, the gene-targeting portions of the ribozyme or RNAi are substantially the same sequence of at least 5 and preferably 6, 7, 8, 9, 10, 11, 12, 13, 14, 15, 16, 17, 18, 19 or 20 or more contiguous nucleotides of a target nucleic acid, such as a nucleic acid of any of the human acid ceramidase or choline kinase sequences. In a long target RNA chain, significant numbers of target sites are not accessible to the ribozyme because they are hidden within secondary or tertiary structures (Birikh et al., Eur J Biochem 245: 1-16, 1997). To overcome the problem of target RNA accessibility, computer generated predictions of secondary structure are typically used to identify targets that are most likely to be single-stranded or have an "open" configuration (see Jaeger et al., Methods Enzymol 183: 281-306, 1989). Other approaches utilize a systematic approach to predicting secondary structure which involves assessing a huge number of candidate hybridizing oligonucleotides molecules (see Milner et al., Nat Biotechnol 15: 537-41, 1997; and Patzel and Sczakiel, Nat Biotechnol 16: 64-8, 1998). Additionally, U.S. Pat. No. 6,251,588, the contents of which are hereby incorporated herein, describes methods for evaluating oligonucleotide probe sequences so as to predict the potential for hybridization to a target nucleic acid sequence. The method of the invention provides for the use of such methods to select preferred segments of a target mRNA sequence that are predicted to be single-stranded and, further, for the opportunistic utilization of the same or substantially identical target mRNA sequence, preferably comprising about 10-20 consecutive nucleotides of the target mRNA, in the design of both the RNAi oligonucleotides and ribozymes of the invention.

### AMPA receptor-specific RNA interference (RNAi)

In another embodiment, the inhibitors of the AMPA receptor that form part of the compositions of the invention are RNAi which are capable of knocking down the expression of an AMPA receptor or any component gene necessary for an AMPA receptor function. RNAi is a process of sequence-specific post-transcriptional gene repression which can occur in eukaryotic cells. In general, this process involves degradation of an mRNA of a particular sequence induced by double-stranded RNA (dsRNA) that is homologous to that sequence. For example, the expression of a long dsRNA corresponding to the sequence of a particular single-stranded mRNA (ss mRNA) will labilize that message, thereby "interfering" with expression of the corresponding gene. Accordingly, any selected gene may be repressed by introducing a dsRNA which corresponds to all or a substantial part of the mRNA for that gene. It appears that when a long dsRNA is expressed, it is initially processed by a ribonuclease III into shorter dsRNA oligonucleotides of as few as 21 to 22 base pairs in length. Accordingly, RNAi may be effected by introduction or expression of relatively short homologous dsRNAs. Indeed, the use of relatively short homologous dsRNAs may have certain advantages as discussed below.

The double stranded oligonucleotides used to effect RNAi are preferably less than 30 base pairs in length and, more preferably, comprise about 25, 24, 23, 22, 21, 20, 19, 18 or 17 base pairs of ribonucleic acid. Optionally the dsRNA oligonucleotides of the invention may include 3' overhang ends. Exemplary 2-nucleotide 3' overhangs may be composed of ribonucleotide residues of any type and may even be composed of 2'-deoxythymidine residues, which lowers the cost of RNA synthesis and may enhance nuclease resistance of siRNAs in the cell culture medium and within transfected cells (see Elbashir et al., Nature 411: 494-8, 2001). Longer dsRNAs of 50, 75, 100 or even 500 base pairs or more may also be utilized in certain embodiments of the invention. Exemplary concentrations of dsRNAs for effecting RNAi are about 0.05 nM, 0.1 nM, 0.5 nM, 1.0 nM, 1.5 nM, 25 nM or 100 nM, although other concentrations may be utilized depending upon the nature of the cells treated, the gene target and other factors readily discernable to the skilled artisan. Exemplary dsRNAs may be synthesized chemically or produced in vitro or in vivo using appropriate expression vectors. Exemplary synthetic RNAs include 21 nucleotide RNAs chemically synthesized using methods known in the art (e.g., Expedite RNA phosphoramidites and thymidine phosphoramidite (Proligo, Germany). Synthetic oligonucleotides are preferably deprotected and gel-purified using methods known in the art (see, e.g., Elbashir et al., Genes Dev. 15: 188-200, 2001). Longer RNAs may be transcribed from promoters, such as T7 RNA polymerase promoters, known in the art. A single RNA target, placed in both possible orientations downstream of an in vitro promoter, will transcribe both strands of the target to create a dsRNA oligonucleotide of the desired target sequence. Any of the above RNA species will be designed to include a portion of nucleic acid sequence represented in a target nucleic acid, such as, for example, a nucleic acid that hybridizes, under stringent and/or physiological conditions, to the polynucleotide encoding human a AMPA receptor.

The specific sequence utilized in design of the oligonucleotides may be any contiguous sequence of nucleotides contained within the expressed gene message of the target. Programs and algorithms, known in the art, may be used to select appropriate target sequences. In addition, optimal sequences may be selected utilizing programs designed to predict the secondary structure of a specified single stranded nucleic acid sequence and allowing selection of those sequences likely to occur in exposed single stranded regions of a folded mRNA. Methods and compositions for designing appropriate oligonucleotides may be found, for example, in U.S. Pat. No. 6,251,588, the contents of which are incorporated herein by reference. Messenger RNA (mRNA) is generally thought of as a linear molecule which contains the information for directing protein synthesis within the sequence of ribonucleotides, however studies have revealed a number of secondary and tertiary structures that exist in most mRNAs. Secondary structure elements in RNA are formed largely by Watson-Crick type interactions between different regions of the same RNA molecule. Important secondary structural elements include intramolecular double stranded regions, hairpin loops, bulges in duplex RNA and internal loops. Tertiary structural elements are formed when secondary structural elements come in contact with each other or with single stranded regions to produce a more complex three dimensional structure. A number of researchers have measured the binding energies of a large number of RNA duplex structures and have derived a set of rules which can be used to predict the secondary structure of RNA (see, e.g., Jaeger et al., Proc. Natl. Acad. Sci. USA 86: 7706, 1989; and Turner et al., Annu. Rev. Biophys. Biophys. Chem. 17:167, 1988). The rules are useful in identification of RNA structural elements and, in particular, for identifying single stranded RNA regions which may represent preferred segments of the mRNA to target for silencing RNAi, ribozyme or antisense technologies. Accordingly, preferred segments of the mRNA target can be identified for design of the RNAi mediating dsRNA oligonucleotides as well as for design of appropriate ribozyme and hammerhead ribozyme compositions of the invention.

Several different types of molecules have been used effectively in the RNAi technology. Small interfering RNA (siRNA), sometimes known as short interfering RNA or silencing RNA, are a class of 20-25 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g., as an antiviral mechanism or in shaping the chromatin structure of a genome. Synthetic siRNAs have been shown to be able to induce RNAi in mammalian cells. This discovery led to a surge in the use of siRNA/RNAi for biomedical research and drug development.

MicroRNA (miRNA) are a related class of gene regulatory small RNAs, typically 21-23 nt in length. They typically differ from siRNA because they are processed from single stranded RNA precursors and show only partially complementary to mRNA targets. Initial studies have indicated that miRNAs regulate gene expression post-transcriptionally at the level of translational inhibition at P-Bodies in the cytoplasm. However, miRNAs may also guide mRNA cleavage similar to siRNAs. This is often the case in plants where the target sites are typically highly complementary to the miRNA. While target sites in plant mRNAs can be found in the 5'UTR, open-reading frames and 3'UTR, in animals, it is the 3' UTR that is the main target. miRNAs are first transcribed as part of a primary microRNA (pri-miRNA). This is then processed by the Drosha with the help of Pasha/DGCR8 (=Microprocessor complex) into pre-miRNAs. The ca. 75 nt pre-miRNA is then exported to the cytoplasm by exportin-5, where it is then diced into 21-23 nt siRNA-like molecules by Dicer. In some cases, multiple miRNAs can be found on the pri-miRNA.

Short hairpin RNA (shRNA) is yet another type of RNA that may be used to effect RNAi. It is a sequence of RNA that makes a tight hairpin turn that can be used to silence gene expression. shRNA is transcribed by RNA polymerase III.

Currently, short-interfering RNAs (siRNAs) and short-hairpin RNAs (shRNAs) are being extensively used to silence various genes to silence functions carried out by the genes. It is becoming easier to harness RNAi to silence specific genes, owing to the development of libraries of readymade shRNA and siRNA gene-silencing constructs by using a variety of sources. For example, RNAi Codex, which consists of a database of shRNA related information and an associated website, has been developed as a portal for publicly available shRNA resources and is accessible at http://codex.cshl.org. RNAi Codex currently holds data from the Hannon-Elledge shRNA library and allows the use of biologist-friendly gene names to access information on shRNA constructs that can silence the gene of interest. It is designed to hold user-contributed annotations and publications for each construct, as and when such data become available. Olson et al. (Nucleic Acids Res. 34(Database issue): D153-D157, 2006, incorporated by reference) have provided detailed descriptions about features of RNAi Codex, and have explained the use of the tool. All these information may be used to help design the various siRNA or shRNA targeting AMPA receptor or other proteins of interest.
Illustrative, non-limitative, examples of interfering RNA specific for the sequence of an AMPA receptor include those described by Passafaro M, et al. Nature. 2003 424(6949):677-81.

### GluR2 siRNA1:

GATCCCCGGAGCACTCCTTAGCTTGATTCAAGAGATCAAGCTAAGGAGTGC
TCCTTTTTGGAAA (SEQ ID NO: 24 )

### GluR2 siRNA2:

AGCTTTTCCAAAAAGGAGCACTCCTAGCTTGATCTCTTGAATCAAGCTAAGG
AGTGCTCCGGG (SEQ ID NO: 25)

### AMPA receptor specific-inhibiting peptides

An "AMPA receptor specific-inhibiting peptide" as used herein, relates to a peptide that biologically mimics regions of the protein sequence of the AMPA receptor that are involve in the AMPA receptor activity and thus they would inhibit the physiological activity of the receptor.

The screening or design of an AMPA receptor specific-inhibiting peptide can be done as described in Olson, G.L et al (J. Med. Chem., 36, 3039-3049, 1993) or in Gillespie, P.et al. (Biopolymers, 43:191-217 1997).

Illustrative, non-limitative, examples of inhibiting peptides specific for an AMPA receptor include:
GluR1(Ser⁸³¹) rat: LIPQQ(pS)INEAIK(SEQ ID NO:26)
GluR1(Ser⁸³¹) rat2 : LIPQQSINEAIK(SEQ ID NO: 27)
GluR4 rat: KHTGTAIRQSSGLAVIASDLP (SEQ ID NO: 28)

### AMPA receptor-specific antisense nucleic acids

In a further embodiment, the invention relates to the use of the isolated "antisense" nucleic acids to inhibit expression, e.g., by inhibiting transcription and/or translation of AMPA receptors nucleic acids. The antisense nucleic acids may bind to the potential drug target by conventional base pair complementarity, or, for example, in the case of binding to DNA duplexes, through specific interactions in the major groove of the double helix. In general, these methods refer to the range of techniques generally employed in the art, and include any methods that rely on specific binding to oligonucleotide sequences.

An antisense construct of the present invention can be delivered, for example, as an expression plasmid which, when transcribed in the cell, produces RNA which is complementary to at least a unique portion of the cellular mRNA which encodes a AMPA receptor polypeptide. Alternatively, the antisense construct is an oligonucleotide probe, which is generated *ex vivo* and which, when introduced into the cell causes inhibition of expression by hybridizing with the mRNA and/or genomic sequences of a target nucleic acid. Such oligonucleotide probes are preferably modified oligonucleotides, which are resistant to endogenous nucleases, e.g., exonucleases and/or endonucleases, and are therefore stable in vivo. Exemplary nucleic acid molecules for use as antisense oligonucleotides are phosphoramidate, phosphothioate and methylphosphonate analogs of DNA (see also U.S. Pat. Nos. 5,176,996; 5,264,564; and 5,256,775). Additionally, general approaches to constructing oligomers useful in antisense therapy have been reviewed, for example, by Van der Krol et al., BioTechniques 6: 958-976, 1988; and Stein et al., Cancer Res 48: 2659-2668, 1988.

With respect to antisense DNA, oligodeoxyribonucleotides derived from the translation initiation site, e.g., between the -10 and +10 regions of the target gene, are preferred. Antisense approaches involve the design of oligonucleotides (either DNA or RNA) that are complementary to mRNA encoding the target polypeptide. The antisense oligonucleotides will bind to the mRNA transcripts and prevent translation. Absolute complementarity, although preferred, is not required. In the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

Oligonucleotides that are complementary to the 5' end of the mRNA, e.g., the 5' untranslated sequence up to and including the AUG initiation codon, should work most efficiently at inhibiting translation. However, sequences complementary to the 3' untranslated sequences of mRNAs have recently been shown to be effective at inhibiting translation of mRNAs as well (Wagner, Nature 372: 333, 1994). Therefore, oligonucleotides complementary to either the 5' or 3' untranslated, non-coding regions of a gene could be used in an antisense approach to inhibit translation of that mRNA. Oligonucleotides complementary to the 5' untranslated region of the mRNA should include the complement of the AUG start codon. Antisense oligonucleotides complementary to mRNA coding regions are less efficient inhibitors of translation but could also be used in accordance with the invention. Whether designed to hybridize to the 5', 3' or coding region of mRNA, antisense nucleic acids should be at least six nucleotides in length, and are preferably less that about 100 and more preferably less than about 50, 25, 17 or 10 nucleotides in length.

It is preferred that *in vitro* studies are first performed to quantitate the ability of the antisense oligonucleotide to inhibit gene expression. It is preferred that these studies utilize controls that distinguish between antisense gene inhibition and nonspecific biological effects of oligonucleotides. It is also preferred that these studies compare levels of the target RNA or protein with that of an internal control RNA or protein. Results obtained using the antisense oligonucleotide may be compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

The antisense oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (e.g., for targeting host cell receptors), or agents facilitating transport across the cell membrane (see, e.g., Letsinger et al., Proc. Natl. Acad. Sci. U.S.A. 86: 6553-6556, 1989; Lemaitre et al., Proc. Natl. Acad. Sci. 84: 648-652, 1987; PCT Publication No. WO88/09810) or the blood-brain barrier (see, e.g., WO89/10134), hybridization-triggered cleavage agents (see, e.g., Krol et al., BioTechniques 6: 958-976, 1988) or intercalating agents (see, e.g., Zon, Pharm. Res. 5: 539-549, 1988). To this end, the oligonucleotide may be conjugated to another molecule, e.g., a peptide, hybridization triggered cross-linking agent, transport agent, hybridization-triggered cleavage agent, etc.

The antisense oligonucleotide may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxytiethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including but not limited to arabinose, 2-fluoroarabinose, xylulose, and hexose. The antisense oligonucleotide can also contain a neutral peptide-like backbone. Such molecules are termned peptide nucleic acid (PNA)-oligomers and are described, e.g., in Perry-O'Keefe et al., Proc. Natl. Acad. Sci. U.S.A. 93: 14670, 1996, and in Eglom et al., Nature 365: 566, 1993. One advantage of PNA oligomers is their capability to bind to complementary DNA essentially independently from the ionic strength of the medium due to the neutral backbone of the DNA. In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet a further embodiment, the antisense oligonucleotide is an alpha-anomeric oligonucleotide. An alpha-anomeric oligonucleotide forms specific double-stranded hybrids with complementary RNA in which, contrary to the usual antiparallel orientation, the strands run parallel to each other (Gautier et al., Nucl. Acids Res. 15: 6625-6641, 1987). The oligonucleotide is a 2'-0-methylribonucleotide (Inoue et al., Nucl. Acids Res. 15: 6131-6148, 1987), or a chimeric RNA-DNA analogue (Inoue et al., FEBS Lett. 215: 327-330, 1987).

While antisense nucleotides complementary to the coding region of a target mRNA sequence can be used, those complementary to the transcribed untranslated region may also be used.

In certain instances, it may be difficult to achieve intracellular concentrations of the antisense sufficient to suppress translation on endogenous mRNAs. Therefore a preferred approach utilizes a recombinant DNA construct in which the antisense oligonucleotide is placed under the control of a strong pol III or pol II promoter. The use of such a construct to transfect target cells will result in the transcription of sufficient amounts of single stranded RNAs that will form complementary base pairs with the endogenous potential drug target transcripts and thereby prevent translation. For example, a vector can be introduced such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in mammalian cells. Expression of the sequence encoding the antisense RNA can be by any promoter known in the art to act in mammalian, preferably human cells. Such promoters can be inducible or constitutive. Such promoters include but are not limited to: the SV40 early promoter region (Bernoist and Chambon, Nature 290: 304-310, 1981), the promoter contained in the 3' long terminal repeat of Rous sarcoma virus (Yamamoto et al., Cell 22: 787-797, 1980), the herpes thymidine kinase promoter (Wagner et al., Proc. Natl. Acad. Sci. U.S.A. 78: 1441-1445, 1981), the regulatory sequences of the metallothionein gene (Brinster et al, Nature 296: 39-42, 1982), etc. Any type of plasmid, cosmid, YAC or viral vector can be used to prepare the recombinant DNA construct, which can be introduced directly into the tissue site.

Alternatively, target gene expression can be reduced by targeting deoxyribonucleotide sequences complementary to the regulatory region of the gene (i.e., the promoter and/or enhancers) to form triple helical structures that prevent transcription of the gene in target cells in the body (see generally, Helene, Anticancer Drug Des. 6(6): 569-84, 1991; Helene et al., Ann. N.Y. Acad. Sci., 660: 27-36, 1992; and Maher, Bioassays 14(12): 807-15, 1992).

Nucleic acid molecules to be used in triple helix formation for the inhibition of transcription are preferably single stranded and composed of deoxyribonucleotides. The base composition of these oligonucleotides should promote triple helix formation via Hoogsteen base pairing rules, which generally require sizable stretches of either purines or pyrimidines to be present on one strand of a duplex. Nucleotide sequences may be pyrimidine-based, which will result in TAT and CGC triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, containing a stretch of G residues. These molecules will form a triple helix with a DNA duplex that is rich in GC pairs, in which the majority of the purine residues are located on a single strand of the targeted duplex, resulting in CGC triplets across the three strands in the triplex.

Alternatively, the potential target sequences that can be targeted for triple helix formation may be increased by creating a so called "switchback" nucleic acid molecule. Switchback molecules are synthesized in an alternating 5'-3',3'-5' manner, such that they base pair with first one strand of a duplex and then the other, eliminating the necessity for a sizable stretch of either purines or pyrimidines to be present on one strand of a duplex.

In certain embodiments, the antisense oligonucleotides are morpholino antisenses. Morpholinos are synthetic molecules which are the product of a redesign of natural nucleic acid structure. Usually 25 bases in length, they bind to complementary sequences of RNA by standard nucleic acid base-pairing. Structurally, the difference between morpholinos and DNA is that while morpholinos have standard nucleic acid bases, those bases are bound to morpholine rings instead of deoxyribose rings, and linked through phosphorodiamidate groups instead of phosphates. Replacement of anionic phosphates with the uncharged phosphorodiamidate groups eliminates ionization in the usual physiological pH range, so morpholinos in organisms or cells are uncharged molecules. Morpholinos are not chimeric oligos; the entire backbone of a morpholino is made from these modified subunits. Morpholinos are most commonly used as single-stranded oligos, though heteroduplexes of a morpholino strand and a complementary DNA strand may be used in combination with cationic cytosolic delivery reagents.

Unlike many antisense structural types (e.g., phosphorothioates), morpholinos do not degrade their target RNA molecules. Instead, morpholinos act by "steric blocking," binding to a target sequence within an RNA and simply getting in the way of molecules which might otherwise interact with the RNA. Morpholino oligos are often used to investigate the role of a specific mRNA transcript in an embryo, such as eggs or embryos of zebrafish, African clawed frog (Xenopus), chick, and sea urchin, producing morphant embryos. With appropriate cytosolic delivery systems, morpholinos are effective in cell culture.

Bound to the 5'-untranslated region of messenger RNA (mRNA), morpholinos can interfere with progression of the ribosomal initiation complex from the 5' cap to the start codon. This prevents translation of the coding region of the targeted transcript (called "knocking down" gene expression). Morpholinos provide a convenient means of knocking down expression of the protein and learning how that knockdown changes the cells or organism. Some morpholinos knock down expression so effectively that after degradation of preexisting proteins the targeted proteins become undetectable by Western blot.

Morpholinos can also interfere with pre-mRNA processing steps, usually by preventing the splice-directing snRNP complexes from binding to their targets at the borders of introns on a strand of pre-RNA. Preventing U1 (at the donor site) or U2/U5 (at the polypyrimidine moiety and acceptor site) from binding can cause modified splicing, commonly leading to exclusions of exons from the mature mRNA. Targeting some splice targets results in intron inclusions, while activation of cryptic splice sites can lead to partial inclusions or exclusions. Targets of U11/U12 snRNPs can also be blocked. Splice modification can be conveniently assayed by reverse-transcriptase polymerase chain reaction (RT-PCR) and is seen as a band shift after gel electrophoresis of RT-PCR products.

Morpholinos have also been used to block miRNA activity, ribozyme activity, intronic splice silencers, and splice enhancers. U2 and U12 snRNP functions have been inhibited by Morpholinos. Morpholinos targeted to "slippery" mRNA sequences within protein coding regions can induce translational frameshifts. Activities of Morpholinos against this variety of targets suggest that Morpholinos can be used as a general-purpose tool for blocking interactions of proteins or nucleic acids with mRNA.

A person skilled in the art would understand that the AMPA receptor can be formed by homomultimeric assemblies by using the same subunits (e.g. only GluR1 (GRIA1), GluR2 (GRIA2), GluR3 (GRIA3),or GluR4 subunits in one receptor ), or in the form of heteromultimeric assemblies (e.g. with both GluR1 (GRIA1) or GluR2 (GRIA2)subunits in one receptor). Thus the inhibition of the AMPA receptor by the AMPA receptor inhibitor is going to depend on the multimeric assembly of the hemichannel.

Thus, the AMPA receptor inhibitors can be combinations of specific inhibitors for the different subunits.

In an embodiment the AMPA receptor is selected from a homomultimeric hemichannel form by GluR1 (GRIA1), GluR2 (GRIA2), GluR3 (GRIA3), or GluR4 subunits. In another embodiment, the AMPA receptor is selected from heteromultimeric assemblies of GluR1 (GRIA1), GluR2 (GRIA2), GluR3 (GRIA3), or GluR4. In a preferred embodiment, the AMPA receptor is a heterotetrameric, consisting of symmetric 'dimer of dimers' of GluR2 and either GluR1, GluR3 or GluR4.

In a particular embodiment the AMPA receptor inhibitor is selected from the compounds of Table I, including combinations thereof. In a preferred embodiment the AMPA receptor is selected from the group consisting of CFM-2, CNQX, CNQX disodium salt, CP 465022 hydrochloride, DNQX, DNQX disodium salt, Evans Blue tetrasodium salt, GYKI 47261 dihydrochloride, NBQX, Philanthotoxin 74 or PhTx-74, SYM 2206, UBP 282 or 3-CBW, YM 90K hydrochloride, ZK 200775 (MPQX).

Effective amounts of the AMPA receptor inhibitor will depend upon the mode of administration, frequency of administration, nature of the treatment, age and condition of the individual to be treated, and the type of pharmaceutical composition used to deliver the compound into a living system. Effective levels of AMPA receptor inhibitor may range from 50 nM to 5 µM (given to experimental animals as 20-30 mg/kg twice daily for ten days), depending upon the compound, system, experimental and clinical endpoints, and toxicity thresholds. While individual doses vary, optimal ranges of effective amounts may be determined by one of ordinary skill in the art. For AMPA receptor inhibitors that are involved in clinical trials for other indications, the safe and effective dosages identified in such trials can be considered when selecting dosages for treatments according to the present invention.

The AMPA receptor inhibitor used according to the methods of the present invention can be administered alone or as a pharmaceutical composition, which includes the compound(s) and a pharmaceutically-acceptable carrier. The AMPA receptor blockers are typically provided as a pharmaceutical composition. The pharmaceutical composition can also include suitable excipients, or stabilizers, and can be in solid or liquid form such as, tablets, capsules, powders, solutions, suspensions, or emulsions. Typically, the composition will contain from about 0.01 to 99 percent, preferably from about 5 to 95 percent of active compound(s), together with the carrier.

The AMPA receptor inhibitor, when combined with pharmaceutically or physiologically acceptable carriers, excipients, or stabilizers, whether in solid or liquid form such as, tablets, capsules, powders, solutions, suspensions, or emulsions, can be administered orally, parenterally, subcutaneously, intravenously, intramuscularly, intraperitoneally, by intranasal instillation, by implantation, by intracavitary or intravesical instillation, intraocularly, intraarterially, intralesionally, transdermally, or by application to mucous membranes, such as, that of the nose, throat, and bronchial tubes (i.e., inhalation).

For most therapeutic purposes, the AMPA receptor inhibitor can be administered orally as a solid or as a solution or suspension in liquid form, via injection as a solution or suspension in liquid form, or via inhalation of a nebulized solution or suspension. The solid unit dosage forms can be of the conventional type. The solid form can be a capsule, such as an ordinary gelatin type containing the compounds of the present invention and a carrier, for example, lubricants and inert fillers such as, lactose, sucrose, or cornstarch. In another embodiment, these compounds are tableted with conventional tablet bases such as lactose, sucrose, or cornstarch in combination with binders like acacia, cornstarch, or gelatin, disintegrating agents, such as cornstarch, potato starch, or alginic acid, and a lubricant, like stearic acid or magnesium stearate.

For injectable dosages, solutions or suspensions of these materials can be prepared in a physiologically acceptable diluent with a pharmaceutical carrier. Such carriers include sterile liquids, such as water and oils, with or without the addition of a surfactant and other pharmaceutically and physiologically acceptable carrier, including adjuvants, excipients or stabilizers. Illustrative oils are those of petroleum, animal, vegetable, or synthetic origin, for example, peanut oil, soybean oil, or mineral oil. In general, water, saline, aqueous dextrose, and related sugar solution, and glycols, such as propylene glycol or polyethylene glycol, are preferred liquid carriers, particularly for injectable solutions.

For use as aerosols, the compound in solution or suspension may be packaged in a pressurized aerosol container together with suitable propellants, for example, hydrocarbon propellants like propane, butane, or isobutane with conventional adjuvants. The materials of the present invention also may be administered in a non-pressurized form such as in a nebulizer or atomizer.

For transdermal routes, the compound is present in a carrier which forms a composition in the form of a cream, lotion, solution, and/or emulsion. The composition can be included in a transdermal patch of the matrix or reservoir type as are conventional in the art for this purpose.

It is contemplated the use of combinations of the AMPA receptor inhibitors described in Table I.

It is also contemplated that administration of the AMPA receptor inhibitor can be carried out in combination with other suitable therapeutic treatments which are useful for treating a disease associated with deposition of amyloid proteins, thus, in other embodiment, the AMPA receptor inhibitor can be combined with other drugs for the treatment of a disease associated with deposition of amyloid proteins such as acetylcholinesterase inhibitors, memantine, donepezil, rivastigmine, galantamine, etc.

In a preferred embodiment the drug for the treatment of a disease associated with deposition of amyloid proteins is a NMDA receptor inhibitor.

Table II include illustrative, non-limitative, NMDA receptor inhibitors.

**Table II**

| | |
|---|---|
| **NMDA receptor inhibitors** | |

| | **NMDA receptor inhibitor** |
|---|---|
| 1 | 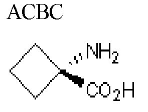 |
| | 1-Aminocyclobutane-1-carboxylic acid |
| 2 | D-AP5 and DL-AP5 (2-APV, D-APV) |
| | 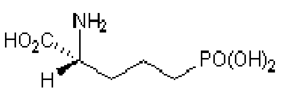 |
| | 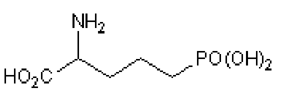 |
| | D-(-)-2-Amino-5-phosphonopentanoic acid |
| | DL-2-Amino-5-phosphonopentanoic acid |
| 3 | L-AP5 |
| | 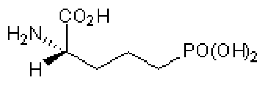 |
| | L-(+)-2-Amino-5-phosphonopentanoic acid |
| 4 | D-AP7 and D1-AP7 |
| | 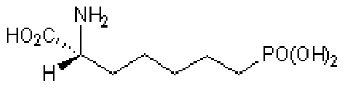 |
| | 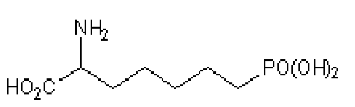 |
| | D-(-)-2-Amino-7-phosphonoheptanoic acid |
| | DL-2-Amino-7-phosphonoheptanoic acid |
| 5 | Arcaine sulfate |
| | 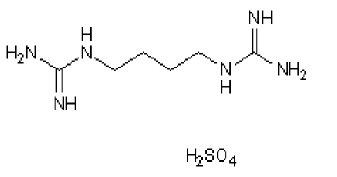 |
| | N,N'-1,4-Butanediylbisguanidine sulfate |
| 6 | *(R*)-4-Carboxyphenylglycine |
| | 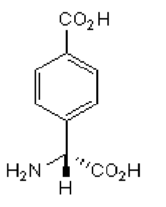 |
| | (R)-4CPG |
| 7 | CGP 37849 |
| | 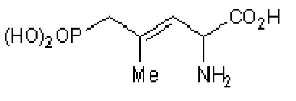 |
| | (E)-(±)-2-Ammo-4-methyl-5-phosphono-3-pentenoic acid |
| 8 | CGP 39551 |
| | 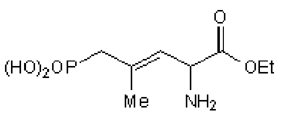 |
| | (E)-(±)-2-Ammo-4-methyl-5-phosphono-3-pentenoic acid ethyl ester |
| 9 | CGP 78608 hydrochloride |
| | 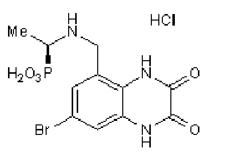 |
| | [(1S)-1-[[(7-Bromo-1,2,3,4-tetrahydro-2,3-dioxo-5-quino xafinyl)methyl]amino]ethyl]phosphonic acid hydrochloride |
| 10 | CGS 19755 |
| | 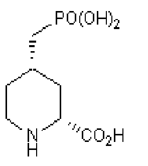 |
| | cis-4-[Phosphomethyl]-piperidine-2-carboxylic acid |
| 11 | 7-Chlorokynurenic acid |
| | 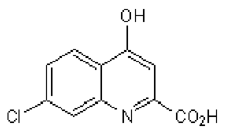 |
| | 7-Chloro-4-hydroxyquinoline-2-carboxylic acid |
| 12 | (2R,3S)-Chlorpheg |
| | 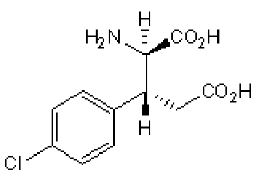 |
| | (2R,3 S)-b-p-Chlorophenylglutamic acid |
| 13 | CNQX |
| | 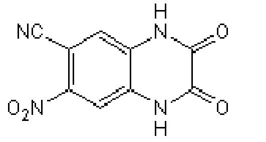 |
| | 6-Cyano-7-nitroquinoxafine-2,3-dione |
| 14 | Co 101244 hydrochloride |
| | 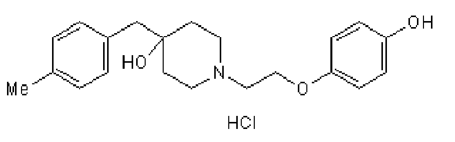 |
| | 1-[2-(4-Hydroxyphenoxy)ethyl]-4-[(4-methylphenyl)methyl]-4-piperidinol hydrochloride |
| 15 | (*R*)-CPP and (RS)-CPP |
| | 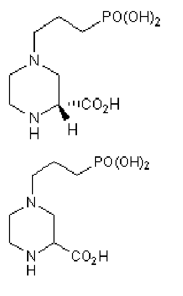 |
| | 3-((R)-2-Carboxypiperazin-4-yl)-propyl-1-phosphonic acid |
| | (RS)-3-(2-Carboxypiperazin-4-yl)-propyl-1-phosphonic acid |
| 16 | D-CPP-ene |
| | 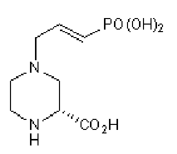 |
| | D-4-[(2E)-3-Phosphono-2-propenyl]-2-piperazinecarboxyli c acid |
| 17 | Dextromethorphan hydrobromide |
| | 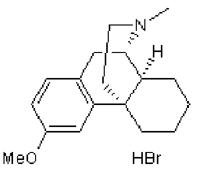 |
| | (9a, 13a, 14a)-3 -Methoxy-17-methylmorphinan hydrobromide |
| 18 | 5,7-Dichlorokynurenic acid |
| | 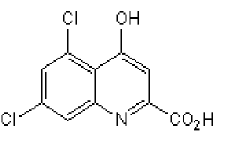 |
| | 5,7-Dichloro-4-hydroxyquinoline-2-carboxylic acid |
| 19 | (±)-1-(1,2-Diphenylethyl)piperidine maleate |
| | 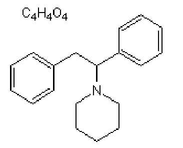 |
| 20 | Eliprodil |
| | 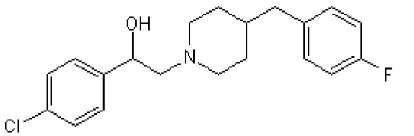 |
| | a-(4-Chlorophenyl)-4-[(4-fluorophenyl)methyl]-1-piperid ineethanol |
| 21 | Felbamate |
| | 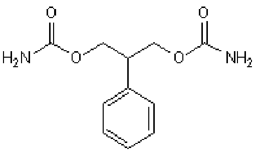 |
| | 2-Phenyl-1,3-propanedioldicarbamate |
| 22 | Flupirtine maleate |
| | 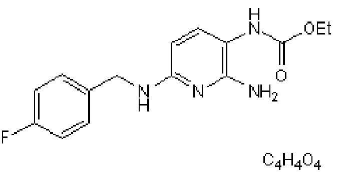 |
| | N-[2-Amino-6-[[4-fluorophenyl)methyl]amino]-3-pyridinyl]carbamic acid ethyl ester maleate |
| 23 | Gavestinel |
| | 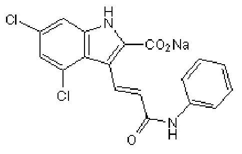 |
| | 4,6-Dichloro-3-[(1E)-3-oxo-3-(phenylamino)-1-propenyl]-1H-indole-2-carboxylic acid sodium salt |
| 24 | (S)-(-)-HA-966 |
| | 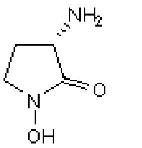 |
| | S)-(-)-3-Amino-1-hydroxypyrrolidin-2-one |
| 25 | HU 211 |
| | 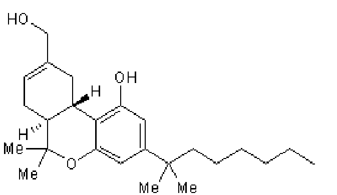 |
| | (6aS,10aS)-3-(1,1 -Dimethylheptyl)-6a,77,10, 10a-tetrahyd ro-1-hydroxy-6,6-dimethyl-6H-dibenzo[b,d]pyran-9-methan ol |
| 26 | *N*-(4-Hydroxyphenylacetyl)spermine |
| | 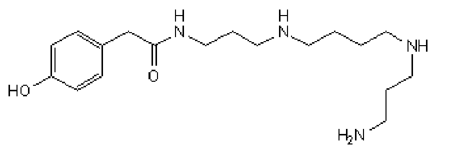 |
| | N-(N-(4-Hydroxyphenylacetyl)-3-aminopropyl)-(N'-3-aminopropyl)-1,4-butanediamine |
| 27 | Ifenprodil hemitartrate and threo Ifenprodil hemitartrate |
| | 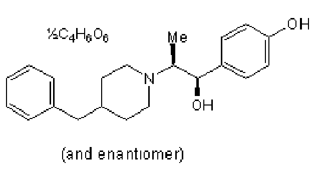 |
| | 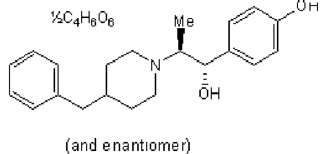 |
| | (1R*,2S*)-erythro-2-(4-Benzylpiperidino)-1-(4-hydroxyphenyl)-1-propanol hemitartrate |
| | (1S*,2S*)-threo-2-(4-Benzylpiperidino)-1-(4-hydroxyphen yl)-1-propanol hemitartrate |
| 28 | Ketamine hydrochloride |
| | 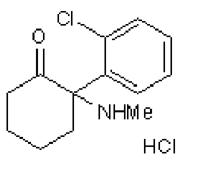 |
| | 2-(2-Chlorophenyl)-2-(methylamino)cyclohexanone hydrochloride |
| 29 | L-689,560 |
| | 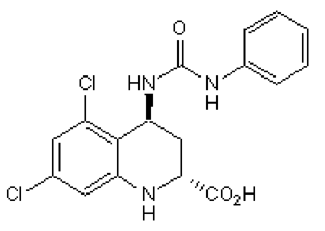 |
| | trans-2-Carboxy-5,7-dichloro-4-phenylaminocarbonylamino-1,2,3,4-tetrahydroquinoline |
| 30 | L-701,252 |
| | 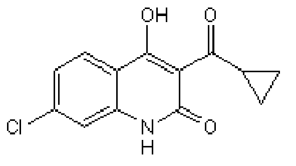 |
| | 7-Chloro-3-(cyclopropylcarbonyl)-4-hydroxy-2(1H)-quinolinone |
| 31 | L-701,324 |
| | 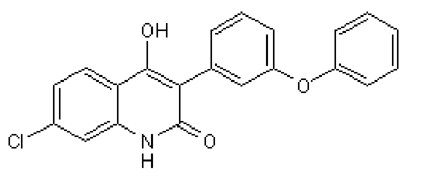 |
| | 7-Chloro-4-hydroxy-3-(3-phenoxy)phenyl-2(1H)-quinolinone |
| 31 | Loperamide hydrochloride |
| | 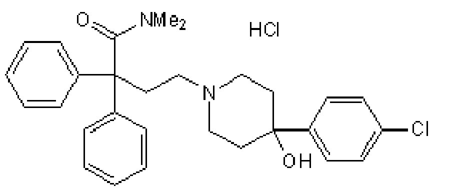 |
| | 4-(4-Chlorophenyl)-4-hydroxy-N,N-dimethyl-a,a-diphenyl- 1-piperidinebutanamide hydrochloride |
| 33 | LY 233053 |
| | 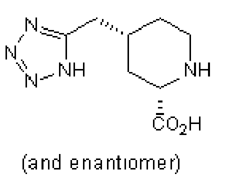 |
| | (2R*,4S*)-4-(1H-Tetrazol-5-ylmethyl)-2-piperidinecarbox ylic acid |
| 34 | LY 235959 |
| | 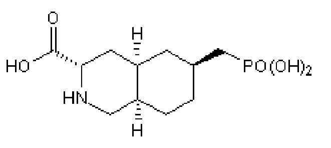 |
| | [3S-(3a,4aa,6b,8aa)]-Decahydro-6-(phosphonomethyl)-3-is oquinolinecarboxylic acid) |
| 35 | Norketamine hydrochloride |
| | 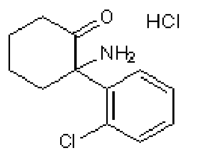 |
| | 2-Amino-2-(2-chlorophenyl)cyclohexanonehydrochloride |
| 36 | Pentamidine isethionate |
| | 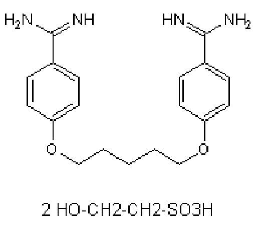 |
| | 4,4'-(Pentamethylenedioxy)dibenzamidine bis-2-hydroxyethanesulfonate salt |
| 37 | Phencyclidine hydrochloride |
| | 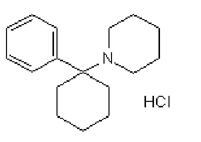 |
| | 1-(1-Phenylcyclohexyl)piperidine hydrochloride |
| 38 | PMPA (NMDA antagonist) |
| | 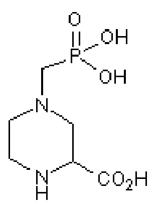 |
| | 4-(Phosphonomethyl)-2-piperazinecarboxylic acid |
| 39 | PPDA |
| | 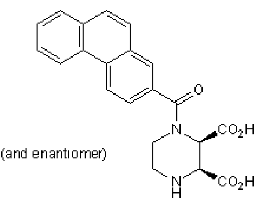 |
| | 2S*,3R*)-1-(Phenanthren-2-carbonyl)piperazine-2,3-dicarboxylic acid |
| 40 | PPPA |
| | 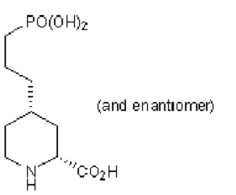 |
| | (2R*,4S*)-4-(3-Phosphonopropyl)-2-piperidinecarboxylic acid |
| 41 | Remacemide hydrochloride |
| | 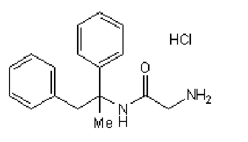 |
| | 2-Amino-N-(1-methyl-1,2-diphenylethyl)acetamidehydrochloride |
| 42 | Ro 04-5595 hydrochloride |
| | 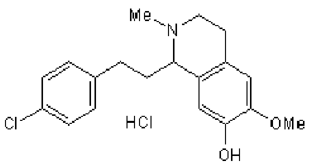 |
| | 1-[2-(4-Chlorophenyl)ethyl]-1,2,3,4-tetrahydro-6-methox y-2-methyl-7-isoquinolinol hydrochloride |
| 43 | Ro 25-6981 maleate |
| | 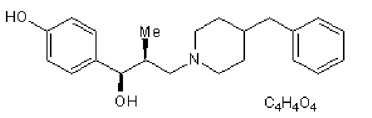 |
| | (aR,bS)-a-(4-Hydroxyphenyl)-b-methyl-4-(phenylmethyl)-1 -piperidinepropanol maleate |
| 44 | Ro 61-8048 |
| | 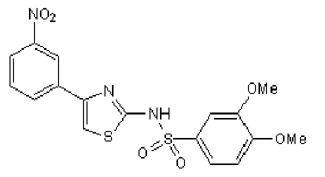 |
| | 3,4-Dimethoxy-N-[4-(3-nitrophenyl)-2-thiazolyl]benzenes ulfonamide |
| 45 | Ro 8-4304 hydrochloride |
| | 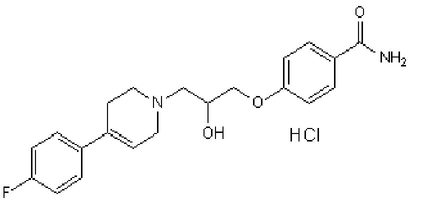 |
| | 4-[3-[4-(4-Fluorophenyl)-1,2,3,6-tetrahydro-1(2H)-pyrid inyl]-2-hydroxypropoxy]benzamide hydrochloride |
| 46 | SDZ 220-040 |
| | 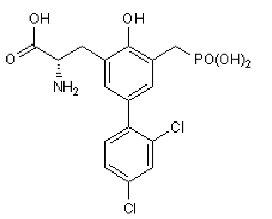 |
| | (S)-a-Amino-2',4'-dichloro-4-hydroxy-5-(phosphonomethyl)-[1,1'-biphenyl]-3-propanoic acid |
| 47 | SDZ 220-581 |
| | 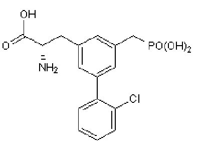 |
| | S)-a-Amino-2'-chloro-5-(phosphonomethyl)[1,1'-biphenyl]-3-propanoic acid |
| 48 | Synthalin sulfate |
| | 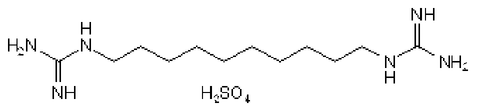 |
| | N,N'-1,10-Decanediylbisguanidine sulfate |
| 49 | TCS 46b |
| | 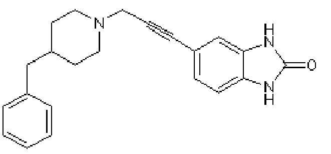 |
| | 1,3-Dihydro-5-[3-[4-(phenylmethyl)-1-2H-benzimidazol-2- one |
| 50 | Memantine |
| | 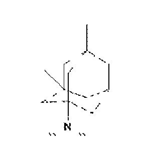 |
| | Dimethyladamantan |
| 51 | An inhibitory antibody capable of specifically inhibiting the activity of a NMDA receptor. |
| 52 | A ribozyme or a DNA enzyme specific for the sequence of a NMDA receptor. |
| 53 | An interfering RNA specific for the sequence of a NMDA receptor. |
| 54 | An inhibiting peptide specific for a NMDA receptor. Conantokin-R |
| | |
| | 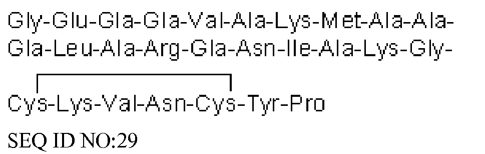 Gly-Glu-Gla-Gla-Val-Ala-Lys-Met-Ala-Ala- Gla-Leu-Ala-Arg-Gla-Asn-Ile-Ala-Lys-Gly- Cys-Lys-Val-Asn-Cys-Tyr-Pro SEQ ID NO:29 Conantokin-T Gly-Glu-Gla-Gla-Tyr-Gln-Lys-Met-Leu-Gla- Asn-Leu-Arg-Gla-Ala-Glu-Val-Lys-Lys-Asn- Ala-NH2 SEQ ID NO:30 |
| 55 | A NMDA receptor specific antisense nucleic acid. |

In a particular embodiment, the NMDA inhibitor is an inhibitory antibody, a ribozyme, an interfering RNA, an inhibiting peptide specific for a NMDA or a NMDA receptor specific antisense nucleic acid.

The definition, design strategy, synthesis, etc., of inhibitory antibodies, ribozyme, interfering RNA, inhibiting peptides specific for a NMDA specific antisense nucleic acid is essentially the same as it was described previously for AMPA receptor.

Suitable NMDA receptor inhibitors include, between others, the compounds described in Table II. In a particular embodiment, the NMDA inhibitor is the antagonist memantine.

Another aspect of the invention refers to a pharmaceutical composition which comprises of at least one NMDA receptor inhibitor, either wide spectrum or selective of a receptor subgroup, along with at least one pharmaceutically accepted excipient. Accepted excipients and ways of administration have been previously described.

Another aspect of the invention refers to a pharmaceutical composition which comprises of at least one NMDA receptor inhibitor, either wide spectrum or selective of a receptor subgroup, along with at least one pharmaceutically accepted excipient for the treatment of Alzheimer.

A further aspect of the present invention relates to a method of treating a subject with a disease associated with deposition of amyloid proteins. This method involves administering an AMPA receptor inhibitor to the subject under conditions effective to treat a disease associated with deposition of amyloid proteins.

This aspect of the present invention is carried out in substantially the same manner as described above.

Another aspect of the present invention relates to a method of inhibiting Ca²⁺-triggered central nervous system cell death which comprises contacting central nervous system cells with an AMPA receptor inhibitor under conditions effective to inhibit Ca²⁺-triggered central nervous system cell death, wherein the inhibitor inhibits the Ca²⁺ flow through the AMPA receptor. Causes of such central nervous system cell death include deposition of amyloid proteins. In a particular embodiment, this method can be used with brain or spinal cord cells which are neural cells (e.g., neurons, oligodendrocytes, or astrocytes) as well as with endothelial cells. Preferably, all of these cell types are from humans. In this case, the AMPA receptor inhibitor inhibits the release of cations through the AMPA receptor. In a preferred embodiment, the cells are neurons.

In a particular embodiment, this method additionally comprises the contacting of a CNS cell with a NMDA receptor inhibitor under conditions effective to inhibit Ca²⁺-triggered brain or spinal cord cell death. This method can be also used with brain or spinal cord cells which are neural cells (e.g., neurons, oligodendrocytes, or astrocytes) as well as with endothelial cells. Preferably, all of these cell types are from humans. In this case, the NMDA receptor inhibitor inhibits the ion flow through the NMDA receptor.

AMPA receptor inhibitors and NMDA receptor inhibitors have been described previously.

In another embodiment the invention relates with a NMDA receptor inhibitor for use in the treatment of Alzheimer.

### SCREENING METHODS OF THE INVENTION

In another aspect the invention relates with a method for identifying a compound useful for inhibiting the cellular death induced by amyloid proteins deposition for the treatment of a disease associated with deposition of amyloid proteins comprising:
a) putting in contact a cell with an amyloid protein,;
b) putting the cells resulting from step a) with the test compound; and
c) determining the levels of Ca²⁺ and/or the levels of AMPA receptor activation in said cell,
wherein if the levels of Ca²⁺ and /or the levels of AMPA receptor activation in said cell after the treatment of said cell with the test compound are lower tahn before the treatment, the test compound is able to inhibiting the cellular death induced by amyloid proteins deposition and useful for the treatment of a disease associated with deposition of amyloid proteins.

Disease associated with deposition of amyloid proteins and AMPA receptors have been described previously.

In a first step [step a)], the method of the invention for identifying a therapeutic agent or screening method of the invention comprises putting in contact a cell with an amyloid protein. The different amyloid proteins have been described previously. In a preferred embodiment, the amyloid protein used in step a) is the Aβ peptide.

In the present invention, as "cell" it is understand any single cell in culture alone or a plurality of cells in culture or forming part of a 3D (three-dimensional) structure such an organotypic culture. Thus, in the present invention the wording "culture" includes any of the above mention possibilities.

Examples of cells that can be use in the method of the invention for identifying a therapeutic agent or screening method of the invention are, without limitation , primary cultures obtained from divers tissues as skin, blood, brain etc; immortalized cells obtained from primary cultures such as osteoblast, mioblast, fibroblast, stem cells, etc. In a preferred embodiment, the cells used are from neuronal origin (primary cultures of neurons or immortalized cells cultures like SN4741, IMR32, N-TERA, PC12, GN11, C1300).

In another embodiment, the cells form a 3D structure denominated organotypic culture. In the present invention "organotypic culture" is any tridimensional culture form tissue that maintains in a high degree the structure, cellular connections and physiological characteristics from the tissue/organ where it has been extracted or dissected. (Gahwiler, 1981 J. Neurosci. Meth. 4, 329-42; Gahwiler 1984 Neuroscience. 11:751-60; Gahwiler 1988 Trends Neurosc. 11:484-9; Stoppini et al. 1991 J. Neuroscience Methods 37:173-82).

Organotypic cultures can be extracted from any organ wherein the influence of amyloid protein deposition associated cellular death, such as the 1-42 Aβ depossition associated celular death, wants to be studied.

In a particular embodiment, the tissue is brain tissue. As a person skilled in the art would understand, the organotypic culture used in the method of the invention, may contain many different regions of the brain and it will be made attending to the particular needs of each assay.

Methods for making an organotypic culture are known by the person skilled in the art (Gahwiler, 1981 J. Neurosci. Meth. 4, 329-42; Gahwiler 1984 Neuroscience. 11:751-60; Gahwiler 1988 Trends Neurosc. 11:484-9; Stoppini et al. 1991 J. Neuroscience Methods 37:173-82). The brain can be from any non-human animal, preferible a vertebrate, such as mammal like a mice, a rat, etc. Said animal can a genetically modify animal or a non-genetically modify animal. Said genetically modify animals can be transgenic animals, namely animals that present inserted in their genome the sequence of a gene of interest (e.g. YFP as described in Winter SM et al., 2007 Respir Physiol Neurobiol. 159:108-14). Also animals with a blocked expression of a gene (i.e. knock out mice) can be used. Methods for the generation of said animals are well known by the person skilled in the art.

One example of a brain organotypic culture is shown in the present application (see methods "Preparation of organotypic cultures").

The cellular (or organotypic) culture is maintained in conditions that permit the survival of the cells of said cultures. Said conditions included, between others, adequate humidity, temperature, gases concentration, nutrients (culture medium) etc. Special incubators, well known by the person skilled in the art, can be used for reaching the previously mention conditions. The culture conditions would be defined for each particular cultured.

Different cell culture mediums are known by the person skilled in the art (Vergni et al., 2009 PLoS ONE4(4):e5278, Chechneva et al., 2006 Neurobiol of Dis. 23(2):247-59).

The expression "putting in contact a cell with an amyloid protein" refers to any process where the cell are put in contact with a amyloid protein such as the Aβ peptide, and includes any possible "in vitro" way of putting extracellular in contact said amyloid protein or any way of introducing said proteins to the cells.

The amyloid proteins can be purchased or produced using chemical or biological synthesis as it is described in the examples of the present invention.

The concentrations of the amyloid protein such as the Aβ peptide used in the screening method of the invention, when using cell cultures can be from between 0.001 µM and 40 µM, 0.05 µM and 30 µM, preferably between 0.15 µM y 10 µM, 0.3 and 5 µM, 0.1 µM y 2 µM. In a preferred embodiment, the cell culture is a neuronal cell culture and the concentration of Aβ peptide is from between 0.5 µM and 3 µM and more preferably de 1.25 µM. In another particular embodiment, the culture is an brain organotypic culture and the Aβ peptide concentration is from 10 nM and 1 µM, preferably 100 nM.

The step (b) of the screening method of the invention comprises the step of contacting the cell (i.e. single cell, a cell population or a organotypic culture) obtained in step a) with a compound whose capacity to inhibit the cellular death induced by amyloid proteins deposition through the AMPA receptor wants to be assessed. According to the present invention, "putting in contact" a cell with the candidate compound includes any possible way of taking the candidate compound extracellular or inside the cell (or cell population, or organotypic culture). Thus, in the event that the candidate compound is a molecule with low molecular weight, it is enough to add said molecule to the culture medium. In the event that the candidate compound is a molecule with a high molecular weight (for example, biological polymers such as a nucleic acid or a protein), it is necessary to provide the means so that this molecule can access the cell interior. In the event that the candidate molecule is a nucleic acid, conventional transfection means can be used, as described previously for the introduction of the DNA construct. In the event that the candidate compound is a protein, the cell can be put in contact with the protein directly or with the nucleic acid encoding it coupled to elements allowing its transcription / translation once they are in the cell interior. To that end, any of the aforementioned methods can be used to allow its entrance in the cell interior. Alternatively, it is possible to put the cell in contact with a variant of the protein to be studied which has been modified with a peptide which can promote the translocation of the protein to the cell interior, such as the Tat peptide derived from the HIV-1 TAT protein, the third helix of the *Antennapedia homeodomain* protein from D. *melanogaster,* the VP22 protein of the herpes simplex virus and arginine oligomers (Lindgren, A. et al., 2000, Trends Pharmacol. Sci, 21:99-103, Schwarze, S.R. et al., 2000, Trends Pharmacol. Sci., 21:45-48, Lundberg, M et al., 2003, Mol. Therapy 8:143-150 and Snyder, E.L. and Dowdy, S.F., 2004, Pharm. Res. 21:389-393).

The compound to be assayed is preferably not isolated but forms part of a more or less complex mixture derived from a natural source or forming part of a library of compounds. Examples of libraries of compounds which can be assayed according to the method of the present invention include, but are not limited to, libraries of peptides including both peptides and peptide analogs comprising D-amino acids or peptides comprising non-peptide bonds, libraries of nucleic acids including nucleic acids with phosphothioate type non-phosphodiester bonds or peptide nucleic acids, libraries of antibodies, of carbohydrates, of compounds with a low molecular weight, preferably organic molecules, of peptide mimetics and the like. In the event that a library of organic compounds with a low molecular weight is used, the library can have been preselected so that it contains compounds which can access the cell interior more easily. The compounds can thus be selected based on certain parameters such as size, lipophilicity, hydrophilicity, capacity to form hydrogen bonds.

The compounds to be assayed can alternatively form part of an extract obtained from a natural source. The natural source can be an animal, plant source obtained from any environment, including but not limited to extracts of land, air, marine organisms and the like.

The incubation of the test compound is made a t different concentrations and times of incubation. The use of negative and positive controls is highly recommendable.

The third step [step c)] comprises the determination of the levels of Ca²⁺ and/or the levels of AMPA receptor activation in said cell treated with the candidate compound.

The Ca²⁺ measured can be cytosolic or mitochondrial. Methods that can be used for the determination of the levels of the cytosolic or mitochondrial Ca²⁺ have been described previously. The methods for determining the AMPA receptor activation in said cell treated with the candidate compound are well known by the person skilled in the art and included without limiting, electrophysiology methods or receptor binding assays. Alternately, FRET-biosensors can be used.

In a particular embodiment, the third step of the screening method of the invention comprises additionally the measurements of the cell death levels or the viability of the culture. Methods for measuring the cell death levels or the viability of the culture are well known by the person skilled in the art. Non limitative examples are described in the present applications (see methods, "Cell viability and toxicity assays")

In general it is consider that the compound would be potentially useful for the treatment of a disease associated with deposition of amyloid proteins, like disease associated with deposition of the Aβpeptide such as Alzheimer, if the levels of Ca²⁺ and /or the levels of AMPA receptor activation in said cell after the treatment of said cell with the test compound are lower than before the treatment.

It is consider that the levels of Ca²⁺ and /or the levels of AMPA receptor activation in said cell after the treatment of said cell with the test compound are lower than before the treatment, when the Ca²⁺ and /or AMPA receptor activation levels are 2, 5, 10, 15, 20, 30, 40, 50, or 100 times lower than before the treatment of the cells with the compound.

A person skilled in the art understands that if the lysis of the cell it is required for the measurements of the Ca²⁺ and/or AMPA receptor activation levels, then it would be necessary the use of a second culture that has not been treated with the test compound for comparing the results.

### EXAMPLE 1

### 1. Materials and methods

### 1.1 Drugs and culture medium

Neurobasal medium, B27 supplement, foetal bovine serum, horse serum and other culture reagents were from Gibco (Invitrogen, Barcelona, Spain). Receptor antagonists MK801, DAP5 (AP5), and memantine were all obtained from Tocris (Cookson, Bristol, UK). CNQX and other chemicals were from Sigma (St Louis, MO, USA). The general caspase inhibitor ZVAD and the caspase-3 specific inhibitor DEVD were purchased in Peptides International (Louisville, KY, USA). DPQ, the PARP-1 inhibitor, was obtained from Calbiochem (La Jolla, CA, USA).

### 1.2 Preparation oƒ Aβ oligomers

Aβ1-42 oligomers were prepared as reported previously (W.L. Klein, et al. 2002. Neurochem. Int. 41 345-352). Briefly, Aβ1-42 (ABX, Radeberg, Germany) was initially dissolved to 1 mM in hexafluoroisopropanol (Sigma; St Louis, MO, USA) and separated into aliquots in sterile microcentrifuge tubes. Hexafluoroisopropanol was totally removed under vacuum in a speed vac system and the peptide film was stored desiccated at -80 °C. For the aggregation protocol, the peptide was first resuspended in dry DMSO (Sigma; St Louis, MO, USA) to a concentration of 5 mM and Hams F-12 (PromoCell, Labclinics, Barcelona, Spain) was added to bring the peptide to a final concentration of 100 µM and incubated at 4°C for 24 h. The preparation was then centrifuged at 14,000 x g for 10 min at 4°C to remove insoluble aggregates and the supernatants containing soluble Aβ1-42 was transferred to clean tubes and stored at 4°C.

### 1.3 Cortical cell culture

Primary neuron cultures were obtained from the cortical lobes of E18 Sprague-Dawley rat embryos according to previously described procedures (Gottlieb M. et al. 2006. Neurobiol. Dis. 23 374-386.) Cells were resuspended in B27 Neurobasal medium plus 10% FBS (Sigma; St Louis, MO, USA) and then seeded onto poly-1-ornithine-coated glass coverslips at 1x10⁵ cells per coverslip (12 mm in diameter) and 48-well plates at 1.5 x 10⁵ per well. One day later, the medium was replaced by serum-free-, B27-supplemented Neurobasal medium. The cultures were essentially free of astrocytes and microglia; they were maintained at 37°C and 5% CO₂. Cultures were used 8-10 days after plating.

### 1.4 Preparation oƒ organotypic cultures

Brain was removed and two hemispheres were separated in HBSS. Thalamus and midbrain was removed and each hemisphere sliced with a tissue chopper (McIlwan Tissue Chopper, Campden Instruments Ltd, Lafayette, IN, USA) in order to obtain coronal slices of 400 µm of thickness. Entorhinal cortex, in connection with hippocampus, were isolated under a dissection microscope, 2 slices plated on each Millicell CM culture inserts (Millipore Ibérica, Madrid, Spain) and maintained in Neurobasal medium supplemented with 0.5% B27, 25% horse serum, 25% HBSS and 25 mg/ml gentamycin at 37°C. Experiments were performed at 7-10 days in vitro. Cultures were immunostained with antibodies to neurofilament-L (NFL 1:200; Cell Signaling Technology, Boston; MA) and choline acetyltransferase (ChAT 40 µg/ml; Chemicon, Millipore Ibérica, Spain) and labelling was revealed with fluorescent goat anti-rabbit and donkey anti-goat secondary antibodies respectively (Alexa Fluor 488, Invitrogen, Barcelona, Spain)

### 1. 5 Electrophysiology

Whole-cell recordings were performed at room temperature using the EPC-7 patch-clamp amplifier (HEKA Elektronik, Lambrecht, Germany). Currents were recorded at a holding membrane potential of -70 mV. Extracellular bath solution with a pH of 7.3 contained the following (in mM): 140 NaCl, 5 KCl, 2.5 CaCl₂, 1 MgCl₂, 10 HEPES and 10 mM glucose. Patch-clamp pipettes (3-5 MΩ) were filled with internal solution at a pH of 7.3 containing the following (in mM): 140 K-gluconate, 1 CaCl₂, 2 MgCl₂, 10 HEPES, 10 EGTA, 2 Mg-ATP, 0.2 Na-GTP.

### 1.6 Ca²⁺ imaging in the cytosol

[Ca²⁺]¡ was determined according to the method described previously (G. Grynkiewicz, et al. 1985. J. Biol. Chem. 260:3440-3450). Neurons were loaded with fura-2 AM (5 µM; Molecular Probes, Invitrogen, Barcelona, Spain) in culture medium for 30 min at 37°C. Cells were washed in HBSS containing 20 mM HEPES, pH 7.4, 10 mM glucose, and 2 mM CaCl₂ (incubation buffer) for 10 min at room temperature. Experiments were performed in a coverslip chamber continuously perfused with incubation buffer at 4 ml/min. The perfusion chamber was mounted on the stage of a inverted epifluorescence microscope (Zeiss Axiovert 35; Oberkochen, Germany) equipped with a 150 W xenon lamp Polychrome IV (T.I.L.L. Photonics, Martinsried, Germany) and a Plan Neofluar 40 x oil immersion objective (Zeiss). Cells were visualized with a high-resolution digital black/white CCD camera (ORCA C4742-80-12 AG; Hamamatsu Photonics Iberica). [Ca²⁺]¡ was estimated by the 340/380 ratio method, using a Kd value of 224 nM. At the end of the assay, in situ calibration was performed with the successive addition of 10 mM ionomycin and 2 M Tris/50 mM EGTA, pH 8.5. Data were analyzed with Excel (Microsoft, Seattle, WA, USA) and Prism (GraphPad Software, San Diego, CA, USA) software.

### 1. 7 Measurement of mitochondrial Ca²⁺

Rhod-2 AM (Molecular Probes, Invitrogen, Barcelona, Spain) was used to measure mitochondrial Ca²⁺ according to the previous procedure with modifications (G. Hajnóczky, L.D. et al. 1995. Cell 82 415-424) Rhod2-AM has a net positive charge, which facilitates its sequestration into mitochondria due to membrane potential-driven uptake. Cells were loaded with 2 µM rhod2-AM for 6 hours. The residual cytosolic fraction of the dye was eliminated when the cells were kept in culture for an additional 18h after loading, whereas the mitochondrial dye fluorescence was maintained. Fluorescence images of rhod-2 were acquired using 550 nm excitation and 590 nm emission. The fluorescence of rhod-2 was not calibrated in terms of [Ca²⁺]mit since it is not a ratiometric dye.

### 1.8 Measurement oƒ Ca²⁺ in organotypic slices

Ca²⁺ levels in hippocampus were monitored by fluorescence microscopy using the Ca²⁺ indicator fluo-4 (Molecular Probes, Invitrogen, Barcelona, Spain). Slices were incubated with fluo-4-AM (5 µM and 0.01 % of pluronic acid) for 45 min at 37 °C. Experiments were performed in a chamber continuously perfused with incubation buffer. Drugs were perfused by a micromanifold with a quartz/polyimide tube of 200 µm ID, attached to a micromanipulator in order to perfuse the drugs directly to the cells. The perfusion chamber was mounted on the stage of a Leica DMLFSA up-right microscope equipped with a 150 W xenon lamp Polychrome V (T.I.L.L. Photonics, Martinsried, Germany) and a HCX Apo 40 x water immersion objective (Leica). Cells were visualized with a CCD camera (EM CCD 9100; Hamamatsu Photonics Iberica, Barcelona, Spain).

### 1.9. Cell viability and toxicity assays

Cortical neurons at 8-10 days in culture were exposed for 24 hours to Aβ oligomers. Antagonists and inhibitors were added to the cultures 30 min before the Aβ. Twenty four hours after drug application, cellular damage was estimated by measuring the level of lactate dehydrogenase released (LDH; Cytotox 96^{®}, Promega, Madison, WI) from damaged cells into the culture media. Data were normalized to the activity of LDH released from vehicle-treated cells (100%) and calculated as a percentage of the control. Results were expressed as the means ± SEM of at least three independent experiments performed in triplicates.

Hippocampus-entorhinal cultures were exposed to Aβ oligomers at 100 nM for 4 days. Antagonists were added to cultures 30 min before the Aβ. Cell death in organotypic cultures was evaluated by cellular uptake of propidium iodide (PI). Slices were stained by adding 10 µM PI into the culture for 2 h at 37°C and washed with PBS by two times for 10 min. Slices were fixed with 4% PFA in PBS for 40 min at room temperature [24]. Afterwards, the slices were excited with 510―560 nm light and the emitted fluorescence acquired at 610 nm using a rhodamine filter on an inverted fluorescence microscope (Cell Observer Z1, Zeiss). PI fluorescence images were captured with a Plan NeoFluar 2.5 x objective (Zeiss), using an EM CCD camera (Hamamatsu, C9100-13), controlled by Axio Vision program (Zeiss). Images were analyzed with the ImageJ analysis program (NIH, MD, USA) and neuronal cell death was expressed as the percentage of the mean gray value of each treatment vs. control.

### 1.10 Measurement of intracellular reactive oxygen species

Neurons were exposed to Aβ oligomers alone or with GluR antagonists as described. Cells were loaded with CM-H2DCFDA at 30 µM to assay the ROS levels. Calcein-AM (1 µM; Molecular Probes, Invitrogen, Barcelona, Spain) was used to quantify the number of cells within the reading field. Fluorescence was measured using a Synergy-HT fluorimeter (Bio-Tek Instruments Incl, Beverly, MA, USA; excitation at 485 nm, emission at 527 nm). All experiments (n≥3) were performed at least in quadruplicate and plotted as means ± SEM.

### 1.11Analysis oƒ mitochondrial membrane potential

Neurons were exposed to Aβ oligomers alone or in presence of different drugs and changes in mitochondrial membrane potential were monitored by reduction of JC-1 (Molecular Probes, Invitrogen, Barcelona, Spain), according to the manufacturer protocol. Briefly, after drug treatment, cells were loaded with 3 µM JC-1 for 15 min at 37°C and were washed with HBSS without phenol red by two times to eliminate the excess of dye. In the cytosol the monomeric form of this dye fluoresces green (excitation at 485 nm, emission at 527 nm), whereas within the mitochondrial matrix highly concentrated JC-1 forms aggregates that fluoresce red (excitation at 485 nm, emission at 590 nm). Both JC-1 monomers and aggregates were detectable using a Synergy-HT fluorimeter (Bio-Tek Instruments Incl, Beverly, MA, USA) and the changes in mitochondrial potential were calculated as the red/green ratio in each condition. All experiments (*n*≥3) were performed at least in triplicate and plotted as meant SEM.

### 1.12 Statistical Analysis

Data are presented as means ± SEM. Statistical analysis was carried out with the Student *t* test and, in all instances, a value ofp < 0.05 was considered significant.

### 2. Results

### 2.1 Aβ oligomers activate NMDA and AMPA receptors in neurons

Dysregulation of intracellular Ca²⁺ homeostasis may underlie Aβ peptide toxicity in AD but the mechanisms are unknown. Here, we tested whether the activation of ionotropic GluRs is involved in neuronal response to Aβ 1-42 oligomeric peptide. Previous studies have shown that Aβ oligomers produce reversible synapse loss (G.M. Shankar, et al. 2007. J. Neurosci. 27 2866-2875), neuronal oxidative stress and dynamin-1 degradation through an NMDA receptor dependent mechanism. First, using electrophysiological recording and Ca²⁺ imaging methods, it was studied whether soluble Aβ oligomers interact with NMDA receptor in neurons. Whole-cell patch-clamp recording was used to examine the effects of Aβ oligomers on cortical neurons in culture. In conditions which favour NMDA receptor activation (glycine 100 µM and no Mg²⁺), Aβ oligomers (1 µM) induced an inward current in the majority of neurons examined (816 ± 101 pA; n=30). Preincubation of APS (100 µM), a competitive NMDA antagonist, reduced the peak of the currents (n=7). Silencing neural activity with TTX (1µM) or using a calcium-free extracellular bath solution did not attenuate the response to Aβ oligomers suggesting a direct interaction of the peptide with NMDA receptors (n= 11 and 13 respectively; Fig 1A).

To characterize further the properties of Aβ responses, we next monitored the concentration of intracellular Ca²⁺ [Ca²⁺]¡ in cultured cortical neurons. We found that oligomeric Aβ (5 µM) caused a robust and sustained increase in [Ca²⁺]¡ (270 nM, n=42), which is nearly abolished by coapplication of NMDA receptor antagonists AP5, MK801 and memantine (n=70). Similarly, Aβ applied in Ca²⁺-free extracellular buffer containing EGTA 50 µM greatly reduced [Ca²⁺]¡ rise (n=11). In contrast, the voltage-gated Ca²⁺ channel inhibitors nifedipine and verapamil were ineffective (n=66; Fig 1B).

Together these results provide evidence indicating that Aβ oligomers activate NMDA receptors in cortical cultured neurons.

Previous results have shown that Aβ(1-42) but not Aβ(1-40) closely interacts with synaptic AMPA receptors. Because of that, we next examined whether Aβ oligomers also activate AMPA receptors in cultured neurons. In the presence of Mg²⁺ and no glycine, Aβ (1µM) alone elicited a small current (43 ± 14 pA, n= 6) and co-applied with cyclothiazide (CTZ), an inhibitor of AMPA receptor desensitization, activated a sustained current (n=18; Fig 2A) that was blocked by the AMPA/kainate antagonist CNQX (n= 6; Fig 2A). We then measured the effects of antagonists of NMDA or AMPA receptor on [Ca²⁺]¡ in neurons exposed to Aβ plus CTZ (5 µM and 100 µM, respectively; Fig 2B). In these experimental conditions, Aβ oligomers increased the [Ca²⁺]¡ in 308 ± 44.7 nM (100 % of Ca²⁺ increase, control value, n=23) a level that was substantially reduced by CNQX 30 µM (50 ± 4 % of control, n=17) and by AP5 100 µM (45 ± 2 % of control, n=17; Fig. 2B). Co-incubation with CNQX and AP5 further reduced [Ca²⁺]¡ to 14 ± 2 % of control (n=18; Fig. 2B). Any facilitatory role of AMPA receptors on Aβ-induced NMDA receptor response was excluded since in physiological conditions (2 mM Mg²⁺, 0.1 mM Gly) CNQX did not modify the Aβ-produced inward current (98.3 ± 6.8 % Aβ + CNQX vs 100 % Aβ alone, n=6). These results indicate that Aβ oligomers in presence of CTZ activate AMPA receptors and, in these conditions, the contribution of both receptors to Ca²⁺ influx is substantial.

### 2.2 Aβ oligomers trigger apoptotic neuronal death through NMDA and AMPA receptors.

As illustrated above, NMDA and AMPA receptor activation by Aβ oligomers in neurons causes an increase in basal [Ca²⁺]¡ levels. Because GluRs mediate Ca²⁺-dependent―neuronal cell death, we tested whether activation of these receptors by Aβ is toxic to neurons. Incubation of Aβ oligomers for 24h caused neuronal death in a dose-dependent manner (Fig 3A). In turn, Aβ toxicity (5 µM; 15.2 ±1.8 % cell death vs. non-treated cells) was greatly attenuated by NMDA receptor antagonists AP5 100 µM, MK801 50 µM and memantine 50 µM and by AMPA/kainate receptor antagonist CNQX 30 µM (Fig. 3B).

Oligomeric Aβ peptides induce mitochondrial dependent-apoptotic cell death in cultured neurons. Damage to mitochondria can result in both caspase-dependent and - independent cell death. To characterize whether these two types of apoptosis are involved in GluR-mediated Aβ toxicity, we used the pan-caspase inhibitor ZVAD-F, a caspase-3 inhibitor Ac-DEVD-F and the nuclear enzyme poly (ADP-ribose) polymerase-1 (PARP-1) inhibitor DPQ. We observed that ZVAD-F (50 µM), DEVD (100 µM) and DPQ (30 µM) reduced Aβ-induced cell death (Fig. 3C). Aβ toxicity decreased further by blocking simultaneously caspase-3 and PARP-1 with DEVD + DPQ (Fig. 3C). These results indicate that Aβ oligomers induce caspase-3- and PARP-1-dependent neuronal death via activation of NMDA and AMPA receptors.

### 2.3 Aβ oligomers induce mitochondrial damage by activating glutamate receptors

To characterize further the signalling cascades triggered by Aβ oligomers, we next studied mitochondrial Ca²⁺ uptake in neurons with rhod-2 AM, a cationic indicator which loads selectively into respiring mitochondria. To assess that feature, we used FCCP 25 µM, a protonophore that collapses the mitochondria membrane potential and releases Ca²⁺ from mitochondria. As expected, FCCP induced a rapid and transitory reduction of rhod-2 fluorescence (Fig 4A). In these loading conditions, we assayed the effects of Aβ oligomers on [Ca²⁺]ₘᵢₜ. Aβ 5 µM increased [Ca²⁺]ₘᵢₜ levels with respect to the control assay (incubation buffer). Ca²⁺ rise was reduced by NMDA antagonist MK801, AP5 and memantine (Fig 4B and C). These results indicate that Aβ induces mitochondrial Ca²⁺ overload and that this effect can be prevented by NMDA receptors antagonists.

The results shown that NMDA antagonists reduce Aβ-generated oxidative stress (Fig. 4D) and mitochondrial depolarization (Fig. 4E) in cortical neurons. Incubation of neurons with Aβ (5 µM; 2 hours) increased ROS levels to 124 ± 7.8 % and reduced the mitochondrial potential to 75 ± 3% with respect to control (100%, non-treated cells). Both parameters were partially restored by NMDA receptor antagonists AP5 (102 ± 11 % and 90 ± 2 %, respectively) and MK801 (104 ± 3.4 % and 85 ± 3 %, respectively) and by AMPA receptor antagonist CNQX (106 ± 6.3 % and 86 ± 3 %, respectively; Fig.4D and E).

Taken together, these results demonstrate that both NMDA and AMPA receptors mediate Aβ-induced mitochondrial dysfunction.

### 2.4 Blockade of NMDA and AMPA receptors protect from Aβ-induced toxicity in entorhinal cortex-hippocampus organotypic slices.

Next it was examined if Aβoligomers induce Ca²⁺ and cell death in a more integral preparation. To that end, rganotypic cultures from entorhinal cortex and hippocampus, two regions that are profoundly affected early in Alzheimer's disease and cause cardinal symptoms of short-term memory loss were used (De Lacoste, M.C. et al. 1993. Neurobiol. Aging 14 1-16).

Slices for organotypic cultures were prepared as to preserve major connections between entorhinal cortex and hippocampus. Thus, neurofilament immunofluorescence with antibodies to NFL revealed axons entering the hippocampus from the entorhinal cortex and layer preservation of these structures (Fig. 5A, B and C). In addition, antibodies to choline acetyltransferase show cholinergic fibers and terminals in entorhinal cortex (Fig. 5 D).

To monitor the effects of Aβ in these cultures, we measured Ca²⁺ levels after application of Aβ to neurons from CA1 in organotypic slices. We found that oligomeric Aβ (20 µM in the pipette) increased [Ca²⁺]¡ in those cells, and that this response was greatly reduced by MK801 (50 µM, n=9) and CNQX (30 µM, n=43) treatment (Fig 5E). Aβ-induced [Ca²⁺]¡ decreased further by blocking simultaneously NMDA and AMPA receptors with MK801+CNQX (n=50; Fig 5E). In turn, Ca²⁺ responses elicited by kainate (1 mM) served as a positive control to evaluate the integrity of the culture (Fig. 5E).

Finally, to test Aβ toxicity cultures were chronically incubated with low concentrations of Aβ oligomers (100 nM) for 4 days, and used PI fluorescence to measure cell death. PI uptake in slices exposed to Aβ showed 4.1 ± 1.0 and 3.8 ± 0.9 fold over the control slices (n=7) in hippocampus and entorhinal cortex respectively (Fig. 5F-G). Instead, co-incubation of GluR antagonists MK801 (10 µM) or CNQX (30 µM) with Aβ oligomers reduced PI uptake to 0.9 ± 0.2 and 1.3 ± 0.4 fold in hippocampus and to 1.2 ± 0.4 and 0.73 ± .01 fold in entorhinal cortex as compared to control slices treated with MK801 or CNQX alone (Fig 5F-G).

Overall, these results indicate that Aβ induces cytosolic Ca²⁺ overload and cell death in organotypic cultures, and that antagonists of NMDA and AMPA receptors prevent both effects.

## Claims

1. An AMPA receptor inhibitor for use in the treatment of a disease associated with deposition of amyloid proteins.

2. AMPA receptor inhibitor according to claim, wherein the AMPA receptor inhibitor is selected from the compounds of Table I.

3. AMPA receptor inhibitor according to any of the preceding claims, wherein the AMPA receptor inhibitor is selected from the group consisting of the compounds described in Table I and combinations thereof.

4. AMPA receptor inhibitor according to any of the preceding claims, wherein the inhibitor is combined with an additional drug for the treatment of disease associated with deposition of amyloid proteins.

5. AMPA receptor inhibitor according to claim 5, wherein said additional drug is a NMDA receptor inhibitor.

6. AMPA receptor inhibitor according to claim 6, wherein the NMDA receptor inhibitor is selected from the compounds described in Table II or combinations thereof.

7. AMPA receptor inhibitor according to any of the preceding claims, wherein the AMPA receptor inhibitor is administrated with a pharmaceutically acceptable carrier or excipient.

8. AMPA receptor inhibitor according to any of the preceding claims, wherein the disease associated with deposition of amyloid proteins is a disease associated with deposition of Beta amyloid peptide.

9. AMPA receptor inhibitor according to claim 9, wherein the disease is Alzheimer.

10. A method of inhibiting Ca²⁺-triggered central nervous system cell death which comprises contacting central nervous system cells with an AMPA receptor inhibitor under conditions effective to inhibit Ca²⁺-triggered central nervous system cell death, wherein the inhibitor inhibits the Ca²⁺ flow through the AMPA receptor.

11. The method according to claim 10, wherein the central nervous system cells are selected from the group consisting of astrocytes, neurons, oligodendrocytes, and combinations thereof.

12. NMDA receptor inhibitor for use in the treatment of Alzheimer.

13. Method for identifying a compound useful for inhibiting the cellular death induced by amyloid proteins deposition for the treatment of a disease associated with deposition of amyloid proteins comprising:
a) putting in contact a cell with an amyloid protein,;
b) putting the cell resulting from step a) with the test compound; and
c) determining the levels of Ca²⁺ and/or the levels of AMPA receptor activation in said cell ,
wherein if the levels of Ca²⁺ and/or the levels of AMPA receptor activation in said cell after the treatment of said cell with the test compound are lower than before the treatment, the test compound is able to inhibit the cellular death induced by amyloid proteins deposition and useful for the treatment of a disease associated with deposition of amyloid proteins.

14. Method according to claim 13, wherein the amyloid protein is the Beta amyloid peptide.
